(19) Europäisches Patentamt
European Patent Office
Office européen des brevets

(11) EP 3 046 573 B1

(12) EUROPEAN PATENT SPECIFICATION

(45) Date of publication and mention of the grant of the patent:
**12.12.2018 Bulletin 2018/50**

(21) Application number: **14776835.2**

(22) Date of filing: **19.09.2014**

(51) Int Cl.:
*A61K 38/22* (2006.01) *C07K 14/575* (2006.01)
*A61K 38/26* (2006.01) *A61K 38/28* (2006.01)
*A61K 47/54* (2017.01)

(86) International application number:
**PCT/EP2014/070037**

(87) International publication number:
**WO 2015/040182 (26.03.2015 Gazette 2015/12)**

(54) **AMYLIN ANALOGUES**

AMYLIN-ANALOGA

ANALOGUES D'AMYLINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.09.2013 DK 201300536**
**09.05.2014 DK 201400253**

(43) Date of publication of application:
**27.07.2016 Bulletin 2016/30**

(73) Proprietor: **Zealand Pharma A/S**
**2600 Glostrup (DK)**

(72) Inventors:
- **DEMMER, Oliver**
**DK-2400 København NV (DK)**
- **JUST, Rasmus**
**2200 København N (DK)**
- **GIEHM, Lise**
**2000 Frederikberg (DK)**
- **SKARBALIENE, Jolanta**
**2690 Karlsunde (DK)**
- **MATHIESEN, Jesper Mosolff**
**3520 Farum (DK)**

(74) Representative: **Forrest, Graham Robert et al**
**Mewburn Ellis LLP**
**City Tower**
**40 Basinghall Street**
**London EC2V 5DE (GB)**

(56) References cited:
**WO-A1-93/10146** **WO-A1-2006/083254**
**WO-A1-2009/034118** **WO-A1-2010/046357**
**WO-A1-2012/168432** **WO-A1-2013/156594**
**WO-A2-2005/000222** **WO-A2-2006/042745**

- **L.-M. YAN ET AL: "Design of a mimic of nonamyloidogenic and bioactive human islet amyloid polypeptide (IAPP) as nanomolar affinity inhibitor of IAPP cytotoxic fibrillogenesis", PROCEEDINGS OF THE NATIONAL ACADEMY OF SCIENCES, vol. 103, no. 7, 7 February 2006 (2006-02-07), pages 2046-2051, XP055159709, ISSN: 0027-8424, DOI: 10.1073/pnas.0507471103 cited in the application**
- **MUTHUSAMY K ET AL: "Design and study of peptide-based inhibitors of amylin cytotoxicity", BIOORGANIC & MEDICINAL CHEMISTRY LETTERS, PERGAMON, AMSTERDAM, NL, vol. 20, no. 4, 15 February 2010 (2010-02-15), pages 1360-1362, XP026887070, ISSN: 0960-894X, DOI: 10.1016/J.BMCL.2010.01.004 [retrieved on 2010-02-03]**
- **DATABASE Geneseq [Online] 14 June 2007 (2007-06-14), "Hybrid polypeptide amylin analog parent molecule SEQ ID NO 78.", XP002739549, retrieved from EBI accession no. GSP:AFC32081 Database accession no. AFC32081**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **DIRK T. S. RIJKERS ET AL: "Inhibition of Amyloid Fibril Formation of Human Amylin by N-Alkylated Amino Acid and -Hydroxy Acid Residue Containing Peptides", CHEMISTRY - A EUROPEAN JOURNAL, vol. 8, no. 18, 16 September 2002 (2002-09-16), pages 4285-4291, XP055007079, ISSN: 0947-6539, DOI: 10.1002/1521-3765(20020916)8:18<4285::AID-CHEM4285>3.0.CO;2-0**

## Description

The present invention relates to amylin analogues that are amylin receptor agonists, and to their medical use in the treatment and/or prevention of a variety of diseases, conditions or disorders, including treatment and/or prevention of excess food intake, obesity and excess body weight, metabolic diseases, and other conditions and disorders described herein. In particular, the present invention relates to stable amylin analogues that have a long duration of action and are well suited for use in the form of a liquid formulation.

## BACKGROUND OF THE INVENTION

Amylin is one of a family of peptide hormones that includes amylin, calcitonin, calcitonin gene-related peptide, adrenomedullin and intermedin (intermedin also being known as AFP-6), and has been implicated in various metabolic diseases and disorders. Human amylin was first isolated, purified and characterized as the major component of amyloid deposits in the islets of pancreases from type 2 diabetes patients.

Native human amylin is a 37-amino acid peptide having the formula

Hy-KC()NTATC()ATQRLANFLVHSSNNFGAILSSTNVGSNTY-$NH_2$ (SEQ ID NO: 1)

wherein Hy- at the N-terminus designates a hydrogen atom, corresponding to the presence of a free amino group on the N-terminal amino acid residue [i.e. the lysine (K) residue at sequence position number 1 in SEQ ID NO: 1]; wherein -$NH_2$ at the C-terminus indicates that the C-terminal carboxyl group is in the amide form; and wherein the parentheses () associated with the two cysteine (C, Cys) residues at sequence positions 2 and 7 indicate the presence of an intramolecular disulfide bridge between the two Cys residues in question.

Amylin is believed to regulate gastric emptying, and to suppress glucagon secretion and food intake, thereby regulating the rate of glucose release to the circulation. Amylin appears to complement the actions of insulin. Compared to healthy adults, type 1 diabetes patients have no circulating amylin, and type 2 diabetes patients exhibit reduced postprandial amylin concentrations. In human trials an amylin analogue known as pramlintide, described in WO 93/10146 and having the sequence Lys-Cys-Asn-Thr-Ala-Thr-Cys-Ala-Thr-Gln-Arg-Leu-Ala-Asn-Phe-Leu-Val-His-Ser-Ser-Asn-Asn-Phe-Gly-Pro-Ile-Leu-Pro-Pro-Thr-Asn-Val-Gly-Ser-Asn-Thr-Tyr) (SEQ ID NO: 2), has been shown to reduce body weight or reduce weight gain. Amylin may be beneficial in treating metabolic disorders such as diabetes and/or obesity. An alternative amylin analogue incorporating N-methylated residues and having a reduced tendency to fibrillation, designated IAPP-GI, has been described by Yan et al. (PNAS, 103(7), 2046-2051, 2006). Further analogues of amylin or pramlintide are described in WO2012/168430, WO2012/168431 and WO2012/168432, as well as WO2006/042745.

Obesity is believed to be a major causal factor in development of type 2 diabetes, which constitutes a growing and worldwide major health problem. Diseases or disorders that may develop as a consequence of untreated diabetes include cardiovascular and peripheral artery disease, micro- and macrovascular complications, stroke, and certain forms of cancer, particularly hematopoietic cancers.

## SUMMARY OF THE INVENTION

The invention provides a compound having the formula I:

$$R^1\text{-}Z\text{-}R^2 \qquad (I)$$

wherein

$R^1$ is hydrogen, $C_{1-4}$ acyl, benzoyl or $C_{1-4}$ alkyl, or a half-life extending moiety M, wherein M is optionally linked to Z via a linker moiety L;

$R^2$ is OH or $NHR^3$, wherein $R^3$ is hydrogen or $C_{1-3}$-alkyl; and

Z is an amino acid sequence of formula Id:

X1-Cys()-X3-Thr-Ala-Thr-Cys()-Ala-Thr-X10-Arg-Leu-Ala-X14-Phe-X16-X17-X18-X19-X20-X21-X22-X23-Gly(Me)-X25-Ile(Me)-X27-X28-X29-X30-X31-Val-Gly-Ser-X35-Thr-X37 (Id);

wherein

X1 is selected from the group consisting of Trp, Lys and Arg;

X3 is selected from the group consisting of Gly, Asn, Glu and Pro;

X10 is selected from the group consisting of Gin, Asp and Glu;

X14 is selected from the group consisting of Asp and Glu;

X16 is Leu or is absent;

X17 is His or is absent;

X18 is selected from the group consisting of His and Arg, or is absent;

X19 is selected from the group consisting of Ser and D-Ser, or is absent;

X20 is Ser or is absent;

X21 is selected from the group consisting of Asn and Ser, or is absent;

X22 is selected from the group consisting of Asn and Ser, or is absent;

X23 is selected from the group consisting of Phe and D-Phe, or is absent;

X25 is Ala or is absent;

X27 is Leu or is absent;

X28 is selected from the group consisting of Ser, Ser(Me), D-Ser and D-Ser(Me), or is absent;

X29 is selected from the group consisting of Ser and Ser(Me) or is absent;

X30 is Thr or is absent;

X31 is selected from the group consisting of Asp, Glu and Asn;

X35 is selected from the group consisting of Asp, Glu and Asn; and

X37 is selected from the group consisting of Tyr, Tyr(Me), Pro, Hyp, Apr, Aze, Pip, Php and Bep;

wherein the parentheses () associated with the two cysteine (C, Cys) residues at sequence positions 2 and 7 indicate the presence of an intramolecular disulfide bridge between the two Cys residues in question;

wherein no more than three positions may be absent, with the proviso that:

(i) if two positions are absent, they are adjacent to one another or one of the absent positions is X25; and
(ii) if three positions are absent, two are adjacent to one another and the other is X25;

and wherein the compound has improved potency compared to human amylin at one or more of the receptors hCTR2, hAMRY1, hAMRY2 or hAMRY3;

or a pharmaceutically acceptable salt or solvate thereof.

In certain embodiments, Z is an amino acid sequence of formula Ie:

X1-Cys()-X3-Thr-Ala-Thr-Cys()-Ala-Thr-X10-Arg-Leu-Ala-Glu-Phe-Leu-His-X18-Ser-Ser-X21-X22-X23-Gly(Me)-Ala-Ile(Me)-Leu-X28-Ser-Thr-X31-Val-Gly-Ser-X35-Thr-X37 (Ie);

wherein

X1 is selected from the group consisting of Trp, Lys and Arg;
X3 is selected from the group consisting of Gly, Asn and Pro;
X10 is selected from the group consisting of Gin, Asp and Glu;
X18 is selected from the group consisting of His and Arg;
X21 is selected from the group consisting of Asn and Ser, or is absent;
X22 is selected from the group consisting of Asn and Ser, or is absent;
X23 is selected from the group consisting of Phe and D-Phe;
X28 is selected from the group consisting of Ser and D-Ser;
X31 is selected from the group consisting of Asp, Glu and Asn;
X35 is selected from the group consisting of Asp, Glu and Asn; and
X37 is selected from the group consisting of Pro and Hyp.

In any of the above formulae (except where stated otherwise), the following residues or combinations of residues may be employed.

X3 is selected from Asn, Gly and Pro, e.g. X3 is Asn.

X10 is Gin.

X14 is Glu.

X23 is D-Phe and/or X28 is D-Ser. For example, X23 is D-Phe and X28 is D-Ser.

X21 and/or X22 are absent. For example, X21 and X22 are absent.

X21 and/or X22 are Asn. For example, X21 and X22 are Asn.

X21 and/or X22 are Ser. For example, X21 and X22 are Ser.

X19 and/or X20 are absent. For example. X19 and X20 are absent. (Not applicable for formula Ie.)

X19 and/or X20 are Ser. For example, X19 and X20 are Ser.

X25 is absent. (Not applicable for formula Ie.)

X31 and/or X35 are Glu. For example, X31 and X35 are Glu.

X31 and/or X35 are Asp. For example, X31 and X35 are Asp.

X31 and/or X35 are Asn. For example, X31 and X35 are Asn.

In further embodiments, the present invention provides a compound or pharmaceutically acceptable salt or solvate thereof of the invention for use in a method of medical treatment of a variety of conditions, diseases, or disorders in a subject, including, but not limited to, obesity and various obesity-related conditions, diseases, or disorders, such as diabetes (e.g. type 2 diabetes).

In further embodiments, peptides of the present invention may also be useful as pharmaceutical agents for treating or preventing weight gain, promoting weight loss, reducing excess body weight and/or treating obesity (e.g. by control of appetite, feeding, food intake, calorie intake, and/or energy expenditure), including morbid obesity, as well as associated diseases, disorders and health conditions, including, but not limited to, obesity-linked inflammation, obesity-linked gallbladder disease and obesity-induced sleep apnea and respiratory problems, degeneration of cartilage, osteoarthritis, and reproductive health complications such as infertility. They may also be useful in the treatment of insulin resistance, glucose intolerance, pre-diabetes, elevated fasting glucose levels, type 1 and/or type 2 diabetes, hypertension and/or dyslipidemia (or a combination of these and other metabolic risk factors), metabolic syndrome and/or metabolic disease, atherosclerosis, arteriosclerosis, coronary heart disease, peripheral artery disease and stroke. Effects of peptides of the invention on these conditions may be mediated in whole or in part via an effect on body weight, or may be independent thereof.

Further aspects and embodiments of the present invention will become apparent from the disclosure below.

## BRIEF DESCRIPTION OF THE DRAWINGS

**Fig. 1:** Dose-normalized PK profiles in male Sprague Dawley rats. Panel A shows data for Compounds 18, 72, 73, 76, 82 and 118 and for a comparison pramlintide derivative denoted NN96. Panel B shows data for compounds 162, 166 and 172. See Example 7 herein for further details.

**Fig. 2** : Effect of 4 weeks treatment with vehicle, Compound 18 (1, 5 and 30 nmol/kg, QW), Compound 18 (5 nmol/kg, every 4th day), NN96 (1 and 30 nmol/kg, QW) or liraglutide (50 nmol/kg, bid) on food intake, water intake and body weight in male Sprague Dawley rats. A) Accumulated food intake (g/rat), B) Accumulated water intake (g/rat), and C) Relative body weight changes (%, vehicle corrected). The legend shown for panel A applies also to panels B and C. Data are mean values ±SEM (n = 6-10/group). Data were compared by 2-way ANOVA followed by Bonferroni post tests. The lines below the graphs represent significant difference vs. vehicle. Differences were considered statistically significant at $p < 0.05$. See Example 9 for further information.

**Fig. 3**: Effect of 4 weeks treatment with vehicle, Compound 18 (1, 5 and 30 nmol/kg, QW), Compound 18 (5 nmol/kg, every 4th day), NN96 (1 and 30 nmol/kg, QW) or liraglutide (50 nmol/kg, bid) on terminal liver fat levels (g). Data are mean values ±SEM (n = 6-10/group). Data were compared by 1-way ANOVA followed by Bonferroni post tests or Kruskal-Wallis test followed by Dunnett's multiple comparison test; *$p < 0.05$, **$p < 0.01$ vs. vehicle. See Example 9 for further information.

**Fig. 4**: Effect of 4 weeks treatment with vehicle, Compound 18 (1, 5 and 30 nmol/kg, QW), Compound 18 (5 nmol/kg, every 4th day), NN96 (1 and 30 nmol/kg, QW) or liraglutide (50 nmol/kg, bid) on terminal mass of fat depots. A) Inguinal fat depot (g), B) Retroperitoneal fat depot (g), C) Epididymal fat depot (g), and D) Mesenteric fat depot (g). Data are mean values ±SEM (n = 6-10/group). Data were compared by 1-way ANOVA followed by Bonferroni post tests or Kruskal-Wallis test followed by Dunnett's multiple comparison test; *$p < 0.05$, **$p < 0.01$, ***$p < 0.001$ vs. vehicle. See Example 9 for further information.

**Fig. 5**: Effect of 4 weeks treatment with vehicle, Compound 18 (1, 5 and 30 nmol/kg, QW), Compound 18 (5 nmol/kg, every 4th day), NN96 (1 and 30 nmol/kg, QW) or liraglutide (50 nmol/kg, bid) on fasted blood glucose and plasma insulin levels. A) Baseline fasted blood glucose levels (mmol/liter), B) Terminal fasted blood glucose levels (mmol/liter), C) Baseline fasted plasma insulin levels (ng/ml), and D) Terminal fasted plasma insulin levels (ng/ml). Data are mean values ± SEM (n = 6-10/group). Data were compared by 1-way ANOVA followed by Bonferroni post tests or Kruskal-Wallis test followed by Dunnett's multiple comparison test; *$p < 0.05$, **$p < 0.01$, ***$p < 0.001$ vs. vehicle. See Example 9 for further information.

**Fig. 6:** Effect of 4 weeks treatment with vehicle or Compound 18 (30 nmol/kg, QW) on food and water intake, and food preference (FP). A) Accumulated high-fat diet (HFD) and low-fat diet (LFD) food intake (g), B) Accumulated water intake (g), and C) Accumulated calorie intake from LFD (kcal). The legend shown for panel A applies also to panels B and C. Data are mean values ± SEM (n = 10/group). Data were compared by 2-way ANOVA followed by Bonferroni post tests. The lines below the graphs represent significant difference vs. vehicle. Differences were considered statistically significant at $p < 0.05$. See Example 10 for further information.

**Fig. 7:** Effect of 4 weeks treatment with vehicle or Compound 18 (30 nmol/kg, QW) on body weight [relative body weight changes (%, vehicle corrected)]. Data are mean values ± SEM (n = 10/group). Data were compared by 2-way ANOVA followed by Bonferroni post tests. The lines below the graphs represent significant difference vs. vehicle. Differences were considered statistically significant at $p < 0.05$. See Example 10 for further information.

**Fig. 8:** Effect of 4 weeks treatment with either vehicle or Compound 18 (30 nmol/kg, QW) on terminal liver fat levels (g). Data are mean values ±SEM (n = 10/group). Data were compared by unpaired t-test or Mann Whitney test; $^{###}p < 0.001$ vs.vehicle. See Example 10 for further information.

**Fig. 9:** Effect of 4 weeks treatment with either vehicle or Compound 18 (30 nmol/kg, QW) on terminal mass of fat depots. A) Inguinal fat depot (g), B) Retroperitoneal fat depot (g), C) Epididymal fat depot (g), and D) Mesenteric fat depot (g). Data are mean values ± SEM (n = 10/group). Data were compared by unpaired t-test or Mann Whitney test; $^{###}p < 0.001$ vs. vehicle. See Example 10 for further information.

**Fig 10:** Effect of 4 weeks treatment with either vehicle or Compound 18 (30 nmol/kg, QW) on blood glucose and plasma insulin levels. A) Baseline fasted blood glucose levels (mmol/liter), B) Terminal fasted blood glucose levels (mmol/liter), C) Baseline fasted plasma insulin levels (ng/ml), and D) Terminal fasted plasma insulin levels (ng/ml). Data are mean values ± SEM (n = 10/group). Data were compared by unpaired t-test or Mann Whitney test; $^{#}p < 0.05$ vs. vehicle.

**Fig. 11:** Effect on non-fasting and fasting blood glucose levels of 4 weeks treatment with vehicle, Compound 18 (30 nmol/kg, every 5th day), Compound 82 (3, 30 and 100 nmol/kg, every 5th day) or liraglutide (40 nmol/kg, twice daily).

A) Non-fasting blood glucose levels (baseline, mmol/liter), B) Non-fasting blood glucose levels (day 13, mmol/liter), C) Non-fasting blood glucose levels (day 27, mmol/liter), D) Fasting blood glucose levels (baseline, mmol/liter), and E) Terminal fasted blood glucose levels (mmol/liter). Data are mean values $\pm$ SEM (n = 12/group). Data were analyzed by 1-way ANOVA followed by Dunnett's multiple comparison test, ***p < 0.001 vs. vehicle; and Student's two-tailed and unpaired t-test, $^{\#}p < 0.05$ vs. pair-fed group.

**Fig. 12:** Effect on HbA1c levels of 4 weeks treatment with vehicle, Compound 18 (30 nmol/kg, every 5$^{th}$ day), Compound 82 (3, 30 and 100 nmol/kg, every 5$^{th}$ day) or liraglutide (40 nmol/kg, twice daily). A) Terminal HbA1c levels (%), and B) Changes in Hb1Ac levels (baseline-to-termination, %). Data are mean values $\pm$ SEM (n = 12/group). Data were analyzed by 1-way ANOVA followed by Dunnett's multiple comparison tests, ***p < 0.001 vs. vehicle; and Student's two-tailed and unpaired t-test, $^{\#\#\#}p < 0.001$ vs. pair-fed group.

**Fig. 13:** Accumulated food intake (in grams) by C57BL/6J male mice over the 12 hour period following administration of 50 nmol/kg body weight of: vehicle and Compounds 175 and 176 (upper panel); and vehicle and Compounds 1, 2 and 18 (lower panel).

**Fig. 14:** Accumulated food intake (in grams) by C57BL/6J male mice over the 12 hour period following administration of 200 nmol/kg body weight of: vehicle and Compounds 175 and 176 (upper panel); and vehicle and Compounds 1, 2 and 18 (lower panel).

## DETAILED DESCRIPTION OF THE INVENTION

Unless otherwise defined herein, scientific and technical terms used herein shall have the meanings that are commonly understood by those of ordinary skill in the art. Generally, nomenclature employed herein in connection with techniques of chemistry, molecular biology, cell and cancer biology, immunology, microbiology, pharmacology, and protein and nucleic acid chemistry, described herein, is that well known and commonly used in the art.

Throughout this specification, the word "comprise" or variations such as "comprises" or "comprising" will be understood to imply the inclusion of a stated integer (or component) or group of integers (or components), but not the exclusion of any other integer (or component) or group of integers (or components).

The singular forms "a," "an," and "the" include the plurals unless the context clearly dictates otherwise.

The term "including" is used to mean "including but not limited to." "Including" and "including but not limited to" are used interchangeably.

The terms "patient," "subject," and "individual" may be used interchangeably and refer to either a human or a non-human animal. These terms include mammals such as humans, primates, livestock animals (e.g., bovines, porcines), companion animals (e.g., canines, felines) and rodents (e.g., mice and rats).

As used herein, the term "pharmaceutically acceptable salt" is intended to indicate a salt which is not harmful to a patient or subject to which the salt in question is administered. It may suitably be a salt chosen, e.g., among acid addition salts and basic salts. Examples of acid addition salts include chloride salts, citrate salts and acetate salts. Examples of basic salts include salts where the cation is selected among alkali metal cations, such as sodium or potassium ions, alkaline earth metal cations, such as calcium or magnesium ions, as well as substituted ammonium ions, such as ions of the type $N(R^1)(R^2)(R^3)(R^4)^+$, where $R^1$, $R^2$, $R^3$ and $R^4$ independently will typically designate hydrogen, optionally substituted $C_{1-6}$-alkyl or optionally substituted $C_{2-6}$-alkenyl. Examples of relevant $C_{1-6}$-alkyl groups include methyl, ethyl, 1-propyl and 2-propyl groups. Examples of $C_{2-6}$-alkenyl groups of possible relevance include ethenyl, 1-propenyl and 2-propenyl. Other examples of pharmaceutically acceptable salts are described in "Remington's Pharmaceutical Sciences", 17th edition, Alfonso R. Gennaro (Ed.), Mark Publishing Company, Easton, PA, USA, 1985 (and more recent editions thereof), in the "Encyclopaedia of Pharmaceutical Technology", 3rd edition, James Swarbrick (Ed.), Informa Healthcare USA (Inc.), NY, USA, 2007, and in J. Pharm. Sci. 66: 2 (1977).

The term "solvate" in the context of the present invention refers to a complex of defined stoichiometry formed between a solute (*in casu,* a peptide or pharmaceutically acceptable salt thereof according to the invention) and a solvent. The solvent in this connection may, for example, be water, ethanol or another pharmaceutically acceptable - typically small-molecular - organic species, such as, but not limited to, acetic acid or lactic acid. When the solvent in question is water, such a solvate is normally referred to as a hydrate.

The term "agonist" as employed in the context of the invention refers to a substance (ligand) that activates the receptor type in question.

Each embodiment of the invention described herein may be taken alone or in combination with one or more other embodiments of the invention.

Throughout the present specification, unless naturally occurring amino acids are referred to by their full name (e.g. alanine, arginine, etc.), they are designated by their conventional three-letter or single-letter abbreviations (e.g. Ala

or A for alanine, Arg or R for arginine, etc.). In the case of certain less common or non-naturally occurring amino acids, unless they are referred to by their full name (e.g. sarcosine, ornithine, etc.), frequently employed three- or four-character codes are employed for residues thereof, including Orn (ornithine, i.e. 2,5-diaminopentanoic acid), Aib (a-aminoisobutyric acid), Dab (2,4-diaminobutanoic acid), Dap (2,3-diaminopropanoic acid), Har (homoarginine), γ-Glu (γ-glutamic acid), Gaba (γ-aminobutanoic acid), β-Ala (i.e. 3-aminopropanoic acid) and 8Ado (8-amino-3,6-dioxaoctanoic acid).

The term "basic amino acid" as employed in the context of the present invention refers to an amino acid containing one or more additional basic groups. Examples thereof include Lys, DLys, Orn, DOrn, Dap, DDap, Dab, DDab, His, DHis, Arg and DArg. Further examples thereof include guanidino derivatives of such basic amino acids, e.g.

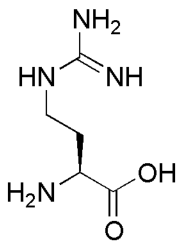

Guanidino analog
of L-Dab:
2-amino-4-guanidino-1-butyric acid

or

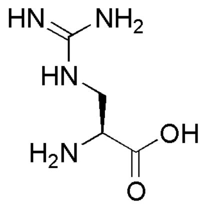

Guanidino analog
of L-Dap:
2-amino-3-guanidino-1-propionic acid

Unless otherwise indicated, reference is made to the L-isomeric forms of the amino acids in question. Where appropriate, the D-isomeric form of an amino acid is indicated in the conventional manner by the prefix "D" before the conventional three-letter code (e.g. DAsp, DPhe).

Additional abbreviations include the following:

| | |
|---|---|
| Ala(Me): | N-methylalanine |
| Gly(Me): | N-methylglycine [also known as sarcosine (Sar)] |
| Ile(Me): | N-methylisoleucine |
| Ser(Me): | N-methylserine |
| Tyr(Me): | N-methyltyrosine |
| Apr: | 4-aminoproline, e.g. (2S,4R)-4-aminoproline [also denoted (4R)-4-amino-L-proline] |
| Aze: | azetidine-2-carboxylic acid, e.g. (2S)-azetidine-2-carboxylic acid |
| Bep: | 4-benzylproline, e.g. (2S,4R)-4-benzylproline [also denoted (4R)-4-benzyl-L-proline] |
| Hyp: | 4-hydroxyproline, e.g. (2S,4R)-4-hydroxyproline [also denoted (4R)-4-hydroxy-L-proline] |
| Php: | 4-phenylproline, e.g. (2S,4S)-4-phenylproline [also denoted (4S)-4-phenyl-L-proline] |
| Pip: | (2S)-piperidine-2-carboxylic acid [also denoted (S)-pipecolic acid] |

Among more specific embodiments of compounds (peptides) of the invention are peptides having the following amino acid sequences:

| | |
|---|---|
| RC()NTATC()ATQRLAEFLHHSSNNF-Gly(Me)-A-Ile(Me)-LSSTNVGSNTP | SEQ ID NO: 3 |
| RC()NTATC()ATQRLAEFLHHSSNNF-Gly(Me)-Ile(Me)-LSSTNVGSNTP | SEQ ID NO: 4 |
| RC()NTATC()ATQRLAEFLHHSSNN-DPhe-Gly(Me)-A-Ile(Me)-LSSTNVGSNTP | SEQ ID NO: 15 |
| RC()NTATC()ATQRLAEFLHRSSNNF-Gly(Me)-A-Ile(Me)-LSSTNVGSNTP | SEQ ID NO: 20 |
| KC()NTATC()ATQRLAEFLHHSSNNF-Gly(Me)-A-Ile(Me)-LSSTNVGSNTP | SEQ ID NO: 21 |
| KC()NTATC()ATQRLAEFLHHSSNNF-Gly(Me)-Ile(Me)-LSSTNVGSNTP | SEQ ID NO: 22 |
| RC()NTATC()ATQRLAEFLHHSSNNF-Gly(Me)-A-Ile(Me)-L-DSer-STNVGSNTP | SEQ ID NO: 25 |
| RC()NTATC()ATQRLAEFLHHSSNNF-Gly(Me)-Ile(Me)-L-DSer-STNVGSNTP | SEQ ID NO: 26 |
| RC()NTATC()ATQRLAEFLHHSSNNF-Gly(Me)-A-Ile(Me)-LSTNVGSNTP | SEQ ID NO: 27 |
| RC()NTATC()ATQRLAEFLHHSSNNF-Gly(Me)-Ile(Me)-LSTNVGSNTP | SEQ ID NO: 28 |
| RC()NTATC()ATQRLAEFLHH-DSer-SNNF-Gly(Me)-A-Ile(Me)-LSSTNVGSNTP | SEQ ID NO: 31 |
| RC()NTATC()ATQRLAEFLHH-DSer-SNNF-Gly(Me)-Ile(Me)-LSSTNVGSNTP | SEQ ID NO: 32 |
| RC()NTATC()ATQRLAEFLHHSSNNF-Gly(Me)-A-Ile(Me)-LSSNVGSNTP | SEQ ID NO: 39 |
| RC()NTATC()ATQRLAEFLHHSSNF-Gly(Me)-Ile(Me)-LSSTNVGSNTP | SEQ ID NO: 40 |
| RC()NTATC()ATQRLAEFLHSSNNF-Gly(Me)-A-Ile(Me)-LSSTNVGSNTP | SEQ ID NO: 41 |
| RC()NTATC()ATQRLAEFLHSSNNF-Gly(Me)-Ile(Me)-LSSTNVGSNTP | SEQ ID NO: 42 |

RC()NTATC()ATQRLAEFLSSNNF-Gly(Me)-A-Ile(Me)-LSSTNVGSNTP SEQ ID NO: 43
RC()NTATC()ATQRLAEFLSSNNF-Gly(Me)-Ile(Me)-LSSTNVGSNTP SEQ ID NO: 44
RC()NTATC()ATQRLAEFLHHSSNF-Gly(Me)-A-Ile(Me)-LSSTNVGSNTP SEQ ID NO: 46
RC()NTATC()ATQRLAEFLHHSNNF-Gly(Me)-A-Ile(Me)-LSSTNVGSNTP SEQ ID NO: 47
RC()NTATC()ATQRLAEFLHHSNNF-Gly(Me)-Ile(Me)-LSSTNVGSNTP SEQ ID NO: 48
RC()NTATC()ATQRLAEFLHHNNF-Gly(Me)-A-Ile(Me)-LSSTNVGSNTP SEQ ID NO: 49
RC()NTATC()ATQRLAEFLHHNNF-Gly(Me)-Ile(Me)-LSSTNVGSNTP SEQ ID NO: 50
RC()NTATC()ATQRLAEFLHHSSF-Gly(Me)-A-Ile(Me)-LSSTNVGSNTP SEQ ID NO: 54
RC()NTATC()ATQRLAEFLHHSSF-Gly(Me)-Ile(Me)-LSSTNVGSNTP SEQ ID NO: 55
RC()NTATC()ATQRLAEFLHHSSNNF-Gly(Me)-A-Ile(Me)-SSTNVGSNTP SEQ ID NO: 56
RC()NTATC()ATQRLAEFLHHSSNNF-Gly(Me)-Ile(Me)-SSTNVGSNTP SEQ ID NO: 57
KC()NTATC()ATQRLAEFLHRSSNNF-Gly(Me)-A-Ile(Me)-LSSTNVGSNTP SEQ ID NO: 59
KC()NTATC()ATQRLAEFLHRSSNNF-Gly(Me)-Ile(Me)-LSSTNVGSNTP SEQ ID NO: 60
RC()NTATC()ATQRLAEFLHHSSNN-DPhe-Gly(Me)-A-Ile(Me)-L-DSer-STNVGSNTP SEQ ID NO: 61
RC()NTATC()ATQRLAEFLHHSSNN-DPhe-Gly(Me)-Ile(Me)-L-DSer-STNVGSNTP SEQ ID NO: 62
RC()NTATC()ATQRLAEFLHHSSNNF-Gly(Me)-A-Ile(Me)-LSSTNVGSNT-Tyr(Me) SEQ ID NO: 63
RC()NTATC()ATQRLAEFLHHSSNNF-Gly(Me)-Ile(Me)-LSSTNVGSNT-Tyr(Me) SEQ ID NO: 64
KC()NTATC()ATQRLAEFLHRSSNNF-Gly(Me)-A-Ile(Me)-L-DSer-STNVGSNTP SEQ ID NO: 70
KC()NTATC()ATQRLAEFLHRSSNNF-Gly(Me)-Ile(Me)-L-DSer-STNVGSNTP SEQ ID NO: 72
KC()NTATC()ATQRLAEFLHRSSNN-DPhe-Gly(Me)-A-Ile(Me)-LSSTNVGSNTP SEQ ID NO: 73
KC()NTATC()ATQRLAEFLHRSSNN-DPhe-Gly(Me)-A-Ile(Me)-L-DSer-STNVGSNTP SEQ ID NO: 74
RC()NTATC()ATQRLAEFLHRSSSSF-Gly(Me)-A-Ile(Me)-LSSTDVGSDTP SEQ ID NO: 75
RC()NTATC()ATQRLAEFLHRSSSSF-Gly(Me)-A-Ile(Me)-LSSTDVGSETP SEQ ID NO: 76
RC()NTATC()ATQRLAEFLHRSSSSF-Gly(Me)-Ile(Me)-LSSTDVGSETP SEQ ID NO: 77
RC()NTATC()ATQRLAEFLHRSSSSF-Gly(Me)-A-Ile(Me)-LSSTEVGSETP SEQ ID NO: 78
RC()NTATC()ATQRLAEFLHRSSSSF-Gly(Me)-Ile(Me)-LSSTEVGSETP SEQ ID NO: 79
RC()NTATC()ATQRLAEFLHRSSF-Gly(Me)-A-Ile(Me)-LSSTEVGSDTP SEQ ID NO: 80
RC()NTATC()ATQRLAEFLHRSSF-Gly(Me)-Ile(Me)-LSSTEVGSDTP EQ ID NO: 81
RC()NTATC()ATQRLAEFLHRSSF-Gly(Me)-A-Ile(Me)-LSSTDVGSETP SEQ ID NO: 82
RC()NTATC()ATQRLAEFLHRSSF-Gly(Me)-A-Ile(Me)-LSSTDVGSDTP SEQ ID NO: 83
RC()NTATC()ATQRLAEFLHRSSF-Gly(Me)-A-Ile(Me)-LSSTEVGSETP SEQ ID NO: 84
RC()NTATC()ATQRLAEFLHRSSF-Gly(Me)-Ile(Me)-LSSTEVGSETP SEQ ID NO: 91
RC()NTATC()ATQRLAEFLHRSSNNF-Gly(Me)-A-Ile(Me)-LSSTNVGSNT-DPro SEQ ID NO: 100
RC()NTATC()ATQRLAEFLHRSSNNF-Gly(Me)-A-Ile(Me)-LSSTNVGSNT-Hyp SEQ ID NO: 101
RC()NTATC()ATQRLAEFLHRSSNNF-Gly(Me)-Ile(Me)-LSSTNVGSNT-Hyp SEQ ID NO: 102
RC()NTATC()ATQRLAEFLHRSSNNF-Gly(Me)-A-Ile(Me)-LSSTNVGSNT-Aze SEQ ID NO: 103
RC()NTATC()ATQRLAEFLHRSSNNF-Gly(Me)-Ile(Me)-LSSTNVGSNT-Aze SEQ ID NO: 104
RC()NTATC()ATQRLAEFLHRSSNNF-Gly(Me)-A-Ile(Me)-LSSTNVGSNT-Pip SEQ ID NO: 105
RC()NTATC()ATQRLAEFLHRSSNNF-Gly(Me)-A-Ile(Me)-LSSTNVGSNT-Apr SEQ ID NO: 106
RC()NTATC()ATQRLAEFLHRSSNNF-Gly(Me)-Ile(Me)-LSSTNVGSNT-Apr SEQ ID NO: 107
RC()NTATC()ATQRLAEFLHRSSNNF-Gly(Me)-A-Ile(Me)-LSSTNVGSNT-Php SEQ ID NO: 108
RC()NTATC()ATQRLAEFLHRSSNNF-Gly(Me)-Ile(Me)-LSSTNVGSNT-Php SEQ ID NO: 109
RC()NTATC()ATQRLAEFLHRSSNNF-Gly(Me)-A-Ile(Me)-LSSTNVGSNT-Bep SEQ ID NO: 110
RC()NTATC()ATERLAEFLHRSSSSF-Gly(Me)-A-Ile(Me)-LSSTEVGSETP SEQ ID NO: 115
RC()NTATC()ATDRLAEFLHRSSSSF-Gly(Me)-A-Ile(Me)-LSSTEVGSETP SEQ ID NO: 116
WC()NTATC()ATQRLAEFLHRSSNNF-Gly(Me)-A-Ile(Me)-LSSTNVGSNTP SEQ ID NO: 121
RC()ETATC()ATQRLAEFLHRSSSSF-Gly(Me)-A-Ile(Me)-LSSTEVGSETP SEQ ID NO: 124
RC()PTATC()ATQRLAEFLHRSSSSF-Gly(Me)-A-Ile(Me)-LSSTEVGSETP SEQ ID NO: 125
RC()GTATC()ATQRLAEFLHRSSSSF-Gly(Me)-A-Ile(Me)-LSSTEVGSETP SEQ ID NO: 126
RC()ETATC()ATERLAEFLHRSSSSF-Gly(Me)-A-Ile(Me)-LSSTEVGSETP SEQ ID NO: 130
RC()PTATC()ATERLAEFLHRSSSSF-Gly(Me)-A-Ile(Me)-LSSTEVGSETP SEQ ID NO: 132
RC()ETATC()ATDRLAEFLHRSSSSF-Gly(Me)-A-Ile(Me)-LSSTEVGSETP SEQ ID NO: 146
RC()PTATC()ATDRLAEFLHRSSSSF-Gly(Me)-A-Ile(Me)-LSSTEVGSETP SEQ ID NO: 147
RC()NTATC()ATQRLAEfLHRSSNNf-Giy(Me)-lie(Me)-LSSTNVGSNTP SEQ ID NO: 148
RC()NTATC()ATQRLAEFLHRSSNNF-Gly(Me)-A-Ile(Me)-LSSTNVGSNTY SEQ ID NO: 149
RC()NTATC()ATQRLAEFLHRSSNNF-Gly(Me)-Ile(Me)-LSSTNVGSNTY SEQ ID NO: 150
WC()GTATC()ATDRLAEFLHRSSF-Gly(Me)-A-Ile(Me)-LSSTDVGSDTP SEQ ID NO: 151
RC()GTATC()ATDRLAEFLHRSSF-Gly(Me)-A-Ile(Me)-LSSTDVGSDTP SEQ ID NO: 152

RC()NTATC()ATERLAEFLHRSSSSF-Gly(Me)-A-Ile(Me)-LSSTDVGSDTP SEQ ID NO: 154
WC()GTATC()ATERLAEFLHRSSF-Gly(Me)-A-Ile(Me)-LSSTEVGSETP SEQ ID NO: 155
RC()GTATC()ATERLAEFLHRSSF-Gly(Me)-A-Ile(Me)-LSSTEVGSETP SEQ ID NO: 156
WC()GTATC()ATERLAEFLHRSSF-Gly(Me)-A-Ile(Me)-LSSTEVGSET-Hyp SEQ ID NO: 158
WC()GTATC()ATERLAEFLHRSSF-Gly(Me)-Ile(Me)-LSSTEVGSET-Hyp SEQ ID NO: 159
RC()GTATC()ATERLAEFLHRSSF-Gly(Me)-A-Ile(Me)-LSSTEVGSET-Hyp SEQ ID NO: 160
RC()GTATC()ATERLAEFLHRSSF-Gly(Me)-Ile(Me)-LSSTEVGSET-Hyp SEQ ID NO: 161
RC()ETATC()ATERLAEFLHRSSSSF-Gly(Me)-A-Ile(Me)-LSSTEVGSET-Hyp SEQ ID NO: 164
RC()ETATC()ATERLAEFLHRSSSSF-Gly(Me)-Ile(Me)-LSSTEVGSET-Hyp SEQ ID NO: 165
RC()PTATC()ATDRLAEFLHRSSF-Gly(Me)-A-Ile(Me)-LSSTDVGSDTP SEQ ID NO: 166
RC()PTATC()ATERLAEFLHRSSSSF-Gly(Me)-A-Ile(Me)-LSSTDVGSDTP SEQ ID NO: 167 and
RC()NTATC()ATQRLAEFLHHSSNNF-Gly(Me)-Ile(Me)-LPPTNVGSNTP (SEQ ID NO: 170)

Each of the specific amino acid sequences recited above, taken individually, may occur in an individual embodiment of a peptide, or pharmaceutically acceptable salt or solvate thereof, of the invention.

Specific compounds of the invention include:

| | |
|---|---|
| [19CD]-isoGlu-RC()NTATC()ATQRLAEFLHHSSNNF-Gly(Me)-A-Ile(Me)-LSSTNVGSNTP-NH2 | Compd. 1 |
| [19CD]-isoGlu-RC()NTATC()ATQRLAEFLHHSSNNF-Gly(Me)-Ile(Me)-LSSTNVGSNTP-NH2 | Compd. 2 |
| [19CD]-isoGlu-RC()NTATC()ATQRLAEFLHHSSNN-DPhe-Gly(Me)-A-Ile(Me)-LSSTNVGSNTP-NH2 | Compd. 13 |
| [19CD]-isoGlu-RC()NTATC()ATQRLAEFLHRSSNNF-Gly(Me)-A-Ile(Me)-LSSTNVGSNTP-NH2 | Compd. 18 |
| [19CD]-isoGlu-KC()NTATC()ATQRLAEFLHHSSNNF-Gly(Me)-A-Ile(Me)-LSSTNVGSNTP-NH2 | Compd. 19 |
| [19CD]-isoGlu-KC()NTATC()ATQRLAEFLHHSSNNF-Gly(Me)-Ile(Me)-LSSTNVGSNTP-NH2 | Compd. 20 |
| [19CD]-isoGlu-RC()NTATC()ATQRLAEFLHHSSNNF-Gly(Me)-A-Ile(Me)-L-DSer-STNVGSNTP-NH2 | Compd. 23 |
| [19CD]-isoGlu-RC()NTATC()ATQRLAEFLHHSSNNF-Gly(Me)-Ile(Me)-L-DSer-STNVGSNTP-NH2 | Compd. 24 |
| [19CD]-isoGlu-RC()NTATC()ATQRLAEFLHHSSNNF-Gly(Me)-A-Ile(Me)-LSTNVGSNTP-NH2 | Compd. 25 |
| [19CD]-isoGlu-RC()NTATC()ATQRLAEFLHHSSNNF-Gly(Me)-Ile(Me)-LSTNVGSNTP-NH2 | Compd. 26 |
| [19CD]-isoGlu-RC()NTATC()ATQRLAEFLHHSSNNF-Gly(Me)-ALSSTNVGSNTP-NH2 | Compd. 27 |
| [19CD]-isoGlu-RC()NTATC()ATQRLAEFLHH-DSer-SNNF-Gly(Me)-A-Ile(Me)-LSSTNVGSNTP-NH2 | Compd. 29 |
| [19CD]-isoGlu-RC()NTATC()ATQRLAEFLHH-DSer-SNNF-Gly(Me)-Ile(Me)-LSSTNVGSNTP-NH2 | Compd. 30 |
| [19CD]-isoGlu-RC()NTATC()ATQRLAEFLHHSSNNF-Gly(Me)-A-Ile(Me)-LSSNVGSNTP-NH2 | Compd. 37 |
| [19CD]-isoGlu-RC()NTATC()ATQRLAEFLHHSSNF-Gly(Me)-Ile(Me)-LSSTNVGSNTP-NH2 | Compd. 38 |
| [19CD]-isoGlu-RC()NTATC()ATQRLAEFLHSSNNF-Gly(Me)-A-Ile(Me)-LSSTNVGSNTP-NH2 | Compd. 39 |
| [19CD]-isoGlu-RC()NTATC()ATQRLAEFLHSSNNF-Gly(Me)-Ile(Me)-LSSTNVGSNTP-NH2 | Compd. 40 |
| [19CD]-isoGlu-RC()NTATC()ATQRLAEFLSSNNF-Gly(Me)-A-Ile(Me)-LSSTNVGSNTP-NH2 | Compd. 41 |
| [19CD]-isoGlu-RC()NTATC()ATQRLAEFLSSNNF-Gly(Me)-Ile(Me)-LSSTNVGSNTP-NH2 | Compd. 42 |
| [19CD]-isoGlu-RC()NTATC()ATQRLAEFLHHSSNF-Gly(Me)-A-Ile(Me)-LSSTNVGSNTP-NH2 | Compd. 44 |
| [19CD]-isoGlu-RC()NTATC()ATQRLAEFLHHSNNF-Gly(Me)-A-Ile(Me)-LSSTNVGSNTP-NH2 | Compd. 45 |
| [19CD]-isoGlu-RC()NTATC()ATQRLAEFLHHSNNF-Gly(Me)-Ile(Me)-LSSTNVGSNTP-NH2 | Compd. 46 |
| [19CD]-isoGlu-RC()NTATC()ATQRLAEFLHHNNF-Gly(Me)-A-Ile(Me)-LSSTNVGSNTP-NH2 | Compd. 47 |
| [19CD]-isoGlu-RC()NTATC()ATQRLAEFLHHNNF-Gly(Me)-Ile(Me)-LSSTNVGSNTP-NH2 | Compd. 48 |
| [19CD]-isoGlu-RC()NTATC()ATQRLAEFLHHSSNNFA-Ile(Me)-LSSTNVGSNTP-NH2 | Compd. 49 |
| [19CD]-isoGlu-RC()NTATC()ATQRLAEFLHHSSF-Gly(Me)-A-Ile(Me)-LSSTNVGSNTP-NH2 | Compd. 52 |
| [19CD]-isoGlu-RC()NTATC()ATQRLAEFLHHSSF-Gly(Me)-Ile(Me)-LSSTNVGSNTP-NH2 | Compd. 53 |
| [19CD]-isoGlu-RC()NTATC()ATQRLAEFLHHSSNNF-Gly(Me)-A-Ile(Me)-SSTNVGSNTP-NH2 | Compd. 54 |
| [19CD]-isoGlu-RC()NTATC()ATQRLAEFLHHSSNNF-Gly(Me)-Ile(Me)-SSTNVGSNTP-NH2 | Compd. 55 |
| [19CD]-isoGlu-KC()NTATC()ATQRLAEFLHRSSNNF-Gly(Me)-A-Ile(Me)-LSSTNVGSNTP-NH2 | Compd. 57 |
| [19CD]-isoGlu-KC()NTATC()ATQRLAEFLHRSSNNF-Gly(Me)-Ile(Me)-LSSTNVGSNTP-NH2 | Compd. 58 |
| [19CD]-isoGlu-RC()NTATC()ATQRLAEFLHHSSNN-DPhe-Gly(Me)-A-Ile(Me)-L-DSer-STNVGSNTP-NH2 | Compd. 59 |

(continued)

| | |
|---|---|
| [19CD]-isoGlu-RC()NTATC()ATQRLAEFLHHSSNN-DPhe-Gly(Me)-Ile(Me)-L-DSer-STNVGSNTP-NH2 | Compd. 60 |
| [19CD]-isoGlu-RC()NTATC()ATQRLAEFLHHSSNNF-Gly(Me)-A-Ile(Me)-LSSTNVGSNT-Tyr (Me)-NH2 | Compd. 61 |
| [19CD]-isoGlu-RC()NTATC()ATQRLAEFLHHSSNNF-Gly(Me)-Ile(Me)-LSSTNVGSNT-Tyr (Me)-NH2 | Compd. 62 |
| [19CD]-isoGlu-KC()NTATC()ATQRLAEFLHRSSNNF-Gly(Me)-A-Ile(Me)-L-DSer-STNVGSNTP-NH2 | Compd. 68 |
| [19CD]-isoGlu-KC()NTATC()ATQRLAEFLHRSSNNF-Gly(Me)-Ile(Me)-L-DSer-STNVGSNTP-NH2 | Compd. 70 |
| [19CD]-isoGlu-KC()NTATC()ATQRLAEFLHRSSNN-DPhe-Gly(Me)-A-Ile(Me)-LSSTNVGSNTP-NH2 | Compd. 71 |
| [19CD]-isoGlu-KC()NTATC()ATQRLAEFLHRSSNN-DPhe-Gly(Me)-A-Ile(Me)-L-DSer-STNVGSNTP-NH2 | Compd. 72 |
| [19CD]-isoGlu-RC()NTATC()ATQRLAEFLHRSSSSF-Gly(Me)-A-Ile(Me)-LSSTDVGSDTP-NH2 | Compd. 73 |
| [19CD]-isoGlu-RC()NTATC()ATQRLAEFLHRSSSSF-Gly(Me)-A-Ile(Me)-LSSTDVGSETP-NH2 | Compd. 74 |
| [19CD]-isoGlu-RC()NTATC()ATQRLAEFLHRSSSSF-Gly(Me)-Ile(Me)-LSSTDVGSETP-NH2 | Compd. 75 |
| [19CD]-isoGlu-RC()NTATC()ATQRLAEFLHRSSSSF-Gly(Me)-A-Ile(Me)-LSSTEVGSETP-NH2 | Compd. 76 |
| [19CD]-isoGlu-RC()NTATC()ATQRLAEFLHRSSSSF-Gly(Me)-Ile(Me)-LSSTEVGSETP-NH2 | Compd. 77 |
| [19CD]-isoGlu-RC()NTATC()ATQRLAEFLHRSSF-Gly(Me)-A-Ile(Me)-LSSTEVGSDTP-NH2 | Compd. 78 |
| [19CD]-isoGlu-RC()NTATC()ATQRLAEFLHRSSF-Gly(Me)-Ile(Me)-LSSTEVGSDTP-NH2 | Compd. 79 |
| [19CD]-isoGlu-RC()NTATC()ATQRLAEFLHRSSF-Gly(Me)-A-Ile(Me)-LSSTDVGSETP-NH2 | Compd. 80 |
| [19CD]-isoGlu-RC()NTATC()ATQRLAEFLHRSSF-Gly(Me)-A-Ile(Me)-LSSTDVGSDTP-NH2 | Compd. 81 |
| [19CD]-isoGlu-RC()NTATC()ATQRLAEFLHRSSF-Gly(Me)-A-Ile(Me)-LSSTEVGSETP-NH2 | Compd. 82 |
| [17CD]-isoGlu-RC()NTATC()ATQRLAEFLHRSSNNF-Gly(Me)-A-Ile(Me)-LSSTNVGSNTP-NH2 | Compd. 89 |
| [15CD]-isoGlu-RC()NTATC()ATQRLAEFLHRSSNNF-Gly(Me)-A-Ile(Me)-LSSTNVGSNTP-NH2 | Compd. 90 |
| [Tetradecanoyl]-isoGlu-RC()NTATC()ATQRLAEFLHRSSNNF-Gly(Me)-A-Ile(Me)-LSSTNVGSNTP-NH2 | Compd. 91 |
| [Hexadecanoyl]-isoGlu-RC()NTATC()ATQRLAEFLHRSSNNF-Gly(Me)-A-Ile(Me)-LSSTNVGSNTP-NH2 | Compd. 92 |
| [Octadecanoyl]-isoGlu-RC()NTATC()ATQRLAEFLHRSSNNF-Gly(Me)-A-Ile(Me)-LSSTNVGSNTP-NH2 | Compd. 93 |
| [Eicosanoyl]-isoGlu-RC()NTATC()ATQRLAEFLHRSSNNF-Gly(Me)-A-Ile(Me)-LSSTNVGSNTP-NH2 | Compd. 94 |
| [19CD]-isoGlu-RC()NTATC()ATQRLAEFLHRSSF-Gly(Me)-Ile(Me)-LSSTEVGSETP-NH2 | Compd. 95 |
| [19CD]-isoGlu-RC()NTATC()ATQRLAEFLHRSSNNF-Gly(Me)-A-Ile(Me)-LSSTNVGSNT-Hyp-NH2 | Compd. 105 |
| [19CD]-isoGlu-RC()NTATC()ATQRLAEFLHRSSNNF-Gly(Me)-Ile(Me)-LSSTNVGSNT-Hyp-NH2 | Compd. 106 |
| [19CD]-isoGlu-RC()NTATC()ATQRLAEFLHRSSNNF-Gly(Me)-A-Ile(Me)-LSSTNVGSNT-Aze-NH2 | Compd. 107 |
| [19CD]-isoGlu-RC()NTATC()ATQRLAEFLHRSSNNF-Gly(Me)-Ile(Me)-LSSTNVGSNT-Aze-NH2 | Compd. 108 |
| [19CD]-isoGlu-RC()NTATC()ATQRLAEFLHRSSNNF-Gly(Me)-A-Ile(Me)-LSSTNVGSNT-Pip-NH2 | Compd. 109 |
| [19CD]-isoGlu-RC()NTATC()ATQRLAEFLHRSSNNF-Gly(Me)-A-Ile(Me)-LSSTNVGSNT-Apr-NH2 | Compd. 110 |
| [19CD]-isoGlu-RC()NTATC()ATQRLAEFLHRSSNNF-Gly(Me)-Ile(Me)-LSSTNVGSNT-Apr-NH2 | Compd. 111 |
| [19CD]-isoGlu-RC()NTATC()ATQRLAEFLHRSSNNF-Gly(Me)-A-Ile(Me)-LSSTNVGSNT-Php-NH2 | Compd. 112 |
| [19CD]-isoGlu-RC()NTATC()ATQRLAEFLHRSSNNF-Gly(Me)-Ile(Me)-LSSTNVGSNT-Php-NH2 | Compd. 113 |
| [19CD]-isoGlu-RC()NTATC()ATQRLAEFLHRSSNNF-Gly(Me)-A-Ile(Me)-LSSTNVGSNT-Bep-NH2 | Compd. 114 |
| [19CD]-isoGlu-RC()NTATC()ATERLAEFLHRSSSSF-Gly(Me)-A-Ile(Me)-LSSTEVGSETP-NH2 | Compd. 119 |
| [19CD]-isoGlu-RC()NTATC()ATDRLAEFLHRSSSSF-Gly(Me)-A-Ile(Me)-LSSTEVGSETP-NH2 | Compd. 120 |
| [19CD]-isoGlu-WC()NTATC()ATQRLAEFLHRSSNNF-Gly(Me)-A-Ile(Me)-LSSTNVGSNTP-NH2 | Compd. 125 |
| [19CD]-isoGlu-RC()ETATC()ATQRLAEFLHRSSSSF-Gly(Me)-A-Ile(Me)-LSSTEVGSETP-NH2 | Compd. 128 |
| [19CD]-isoGlu-RC()PTATC()ATQRLAEFLHRSSSSF-Gly(Me)-A-Ile(Me)-LSSTEVGSETP-NH2 | Compd. 129 |
| [19CD]-isoGlu-RC()GTATC()ATQRLAEFLHRSSSSF-Gly(Me)-A-Ile(Me)-LSSTEVGSETP-NH2 | Compd. 130 |
| [19CD]-isoGlu-RC()ETATC()ATERLAEFLHRSSSSF-Gly(Me)-A-Ile(Me)-LSSTEVGSETP-NH2 | Compd. 134 |
| [19CD]-isoGlu-RC()PTATC()ATERLAEFLHRSSSSF-Gly(Me)-A-Ile(Me)-LSSTEVGSETP-NH2 | Compd. 136 |

(continued)

| | |
|---|---|
| [19CD]-isoGlu-RC()ETATC()ATDRLAEFLHRSSSSF-Gly(Me)-A-Ile(Me)-LSSTEVGSETP-NH2 | Compd. 150 |
| [19CD]-isoGlu-RC()PTATC()ATDRLAEFLHRSSSSF-Gly(Me)-A-Ile(Me)-LSSTEVGSETP-NH2 | Compd. 151 |
| [19CD]-isoGlu-RC()NTATC()ATQRLAEFLHRSSNNF-Gly(Me)-A-Ile(Me)-LSSTNVGSNTP-NHMe | Compd. 152 |
| [19CD]-isoGlu-RC()NTATC()ATQRLAEFLHRSSNNF-Gly(Me)-Ile(Me)-LSSTNVGSNTP-NHMe | Compd. 153 |
| [19CD]-isoGlu-RC()NTATC()ATQRLAEFLHRSSNNF-Gly(Me)-A-Ile(Me)-LSSTNVGSNTY-NHMe | Compd. 154 |
| [19CD]-isoGlu-RC()NTATC()ATQRLAEFLHRSSNNF-Gly(Me)-Ile(Me)-LSSTNVGSNTY-NHMe | Compd. 155 |
| [19CD]-isoGlu-RC()NTATC()ATQRLAEFLHRSSNNF-Gly(Me)-A-Ile(Me)-LSSTNVGSNTY-NH2 | Compd. 156 |
| [19CD]-isoGlu-WC()GTATC()ATDRLAEFLHRSSF-Gly(Me)-A-Ile(Me)-LSSTDVGSDTP-NH2 | Compd. 157 |
| [19CD]-isoGlu-RC()GTATC()ATDRLAEFLHRSSF-Gly(Me)-A-Ile(Me)-LSSTDVGSDTP-NH2 | Compd. 158 |
| [19CD]-isoGlu-RC()NTATC()ATERLAEFLHRSSSSF-Gly(Me)-A-Ile(Me)-LSSTDVGSDTP-NH2 | Compd. 160 |
| [19CD]-isoGlu-WC()GTATC()ATERLAEFLHRSSF-Gly(Me)-A-Ile(Me)-LSSTEVGSETP-NH2 | Compd. 161 |
| [19CD]-isoGlu-RC()GTATC()ATERLAEFLHRSSF-Gly(Me)-A-Ile(Me)-LSSTEVGSETP-NH2 | Compd. 162 |
| [19CD]-isoGlu-WC()GTATC()ATERLAEFLHRSSF-Gly(Me)-A-Ile(Me)-LSSTEVGSET-Hyp-NH2 | Compd. 164 |
| [19CD]-isoGlu-WC()GTATC()ATERLAEFLHRSSF-Gly(Me)-Ile(Me)-LSSTEVGSET-Hyp-NH2 | Compd. 165 |
| [19CD]-isoGlu-RC()GTATC()ATERLAEFLHRSSF-Gly(Me)-A-Ile(Me)-LSSTEVGSET-Hyp-NH2 | Compd. 166 |
| [19CD]-isoGlu-RC()GTATC()ATERLAEFLHRSSF-Gly(Me)-Ile(Me)-LSSTEVGSET-Hyp-NH2 | Compd. 167 |
| [19CD]-isoGlu-RC()ETATC()ATERLAEFLHRSSSSF-Gly(Me)-A-Ile(Me)-LSSTEVGSET-Hyp-NH2 | Compd. 170 |
| [19CD]-isoGlu-RC()ETATC()ATERLAEFLHRSSSSF-Gly(Me)-Ile(Me)-LSSTEVGSET-Hyp-NH2 | Compd. 171 |
| [19CD]-isoGlu-RC()PTATC()ATDRLAEFLHRSSF-Gly(Me)-A-Ile(Me)-LSSTDVGSDTP-NH2 | Compd. 172 |
| [19CD]-isoGlu-RC()PTATC()ATERLAEFLHRSSSSF-Gly(Me)-A-Ile(Me)-LSSTDVGSDTP-NH2 | Compd. 173 |
| [19CD]-isoGlu-RC()NTATC()ATQRLAEFLHHSSNNF-Gly(Me)-Ile(Me)-LPPTNVGSNTP-$NH_2$ | Compd. 176 |

wherein:

[19CD] represents [19-carboxynonadecanoyl],
[17CD] represents [17-carboxyheptadecanoyl, and
[15CD] represents [15-carboxypentadecanoyl];

and pharmaceutically acceptable salts and solvates thereof.

Each of the latter specific compounds (peptides), and pharmaceutically acceptable salts and solvates thereof, of the invention further constitutes an individual embodiment of the invention.

In some embodiments, the invention relates to pharmaceutical compositions comprising a peptide, or a pharmaceutically acceptable salt or solvate thereof, according to the invention, together with a pharmaceutically acceptable carrier, excipient or vehicle.

In some embodiments, the compounds of the present invention may be useful as pharmaceutical agents for the prevention of kidney failure, hypertension and/or dyslipidemia (or a combination of these metabolic and cardiovascular risk factors), atherosclerosis, arteriosclerosis, macrovascular disease, microvascular disease, coronary heart disease, peripheral artery disease and stroke. Effects of the peptides of the invention on these conditions may be mediated in whole or in part via an effect on body weight, or may be independent thereof.

In certain embodiments, there is provided a peptide or pharmaceutical composition of the invention for treating, preventing, and/or reducing the severity of a disease, condition or disorder selected from Alzheimer's disease, diabetes, type 1 diabetes, type 2 diabetes, pre-diabetes, insulin resistance syndrome, impaired glucose tolerance (IGT), disease states associated with elevated blood glucose levels, metabolic disease including metabolic syndrome, hyperglycemia, hypertension, atherogenic dyslipidemia, non-alcoholic steatohepatitis (NASH), hepatic steatosis ("fatty liver"), arteriosclerosis (e.g. atherosclerosis), diabetic heart (including diabetic cardiomyopathy and heart failure as a diabetic complication), coronary heart disease, peripheral artery disease and stroke.

In certain embodiments, there is provided a peptide or pharmaceutical composition of the invention for treating, preventing, and/or reducing the severity of a disease, condition, or disorder selected from obesity, morbid obesity, morbid obesity prior to surgery, obesity-linked inflammation, obesity-linked gall bladder disease, obesity-induced sleep apnea and respiratory problems, degeneration of cartilage and osteoarthritis, and reproductive health complications such as infertility.

The peptide or pharmaceutical composition of the invention may be used in preventing weight gain or promoting weight loss.

The term "therapeutically effective amount" as used herein in the context of the above-described methods of

treatment or other therapeutic interventions according to the invention refers to an amount that is sufficient to cure, ameliorate, alleviate or partially arrest the clinical manifestations of the particular disease, disorder or condition that is the object of the treatment or other therapeutic intervention in question e.g. as measured by established clinical endpoints or other biomarkers (established or experimental). A therapeutically relevant amount may be determined empirically by one skilled in the art based on the indication being treated or prevented and the subject to whom the therapeutically relevant amount is being administered. For example, the skilled worker may measure one or more of the clinically relevant indicators of bioactivity described herein, e.g. plasma lipid levels, blood glucose levels or insulin release. The skilled worker may determine a clinically relevant amount through *in vitro* or *in vivo* measurements. Other exemplary measures include weight gain, weight loss, and change in blood pressure.

An amount adequate to accomplish any or all of these effects is defined as a therapeutically effective amount. The administered amount and the method of administration can be tailored to achieve optimal efficacy. An amount effective for a given purpose will depend, *inter alia,* on the severity of the disease, disorder or condition that is the object of the particular treatment or other therapeutic intervention, on the body weight and general condition of the subject in question, on diet, on possible concurrent medication, and on other factors well known to those skilled in the medical arts. Determination of an appropriate dosage size and dosing regimen most appropriate for administration of a peptide or pharmaceutically acceptable salt or solvate thereof according to the invention to a human may be guided by the results obtained by the present invention, and may be confirmed in properly designed clinical trials. An effective dosage and treatment protocol may be determined by conventional means, starting with a low dose in laboratory animals and then increasing the dosage while monitoring the effects, and systematically varying the dosage regimen as well. Numerous factors may be taken into consideration by a clinician when determining an optimal dosage for a given subject. Such considerations are well known to the skilled person.

The terms "treatment" and grammatical variants thereof (e.g. "treated", "treating", "treat") as employed in the present context refer to an approach for obtaining beneficial or desired clinical results. For the purposes of this invention, beneficial or desired clinical results include, but are not limited to, alleviation of symptoms, diminishment of extent of disease, stabilization (i.e. not worsening) of state of disease, delay or slowing of disease progression, amelioration or palliation of the disease state, and remission (whether partial or total), whether detectable or undetectable. "Treatment" can also mean prolonging survival relative to expected survival time if not receiving treatment. A subject (e.g. a human) in need of treatment may thus be a subject already afflicted with the disease or disorder in question. The term "treatment" includes inhibition or reduction of an increase in severity of a pathological state or symptoms (e.g. weight gain or hyperglycemia) relative to the absence of treatment, and is not necessarily meant to imply complete cessation of the relevant disease, disorder or condition.

The terms "prevention" and grammatical variants thereof (e.g., "prevented", "preventing", "prevent") as employed in the present context refer to an approach for hindering or preventing the development of, or altering the pathology of, a condition, disease or disorder. Accordingly, "prevention" may refer to prophylactic or preventive measures. For the purposes of this invention, beneficial or desired clinical results include, but are not limited to, prevention or slowing of symptoms, progression or development of a disease, whether detectable or undetectable. A subject (e.g. a human) in need of "prevention" may thus be a subject not yet afflicted with the disease or disorder in question. The term "prevention" thus includes slowing the onset of disease relative to the absence of treatment, and is not necessarily meant to imply permanent prevention of the relevant disease, disorder or condition.

In some embodiments of the invention, a subject in need of the particular treatment or other therapeutic intervention referred to in connection with the various aspects of the invention described above is a mammal. In further embodiments, the mammal is a human.

## Synthesis of compounds (peptides) of the invention

The invention further provides a method of synthesis of a compound of the invention. The compounds (which may also be referred to as peptides) of the invention may suitably be manufactured by standard synthetic methods. Thus, the peptides may be synthesized by, e.g., methods comprising synthesizing the peptide by standard solid-phase or liquid-phase methodology, either stepwise or by fragment assembly, and optionally isolating and purifying the final peptide product. In this context, reference may be made to WO 98/11125 or, *inter alia,* Fields, G.B. et al., "Principles and Practice of Solid-Phase Peptide Synthesis"; in: Synthetic Peptides, Gregory A. Grant (ed.), Oxford University Press (2nd edition, 2002) and the synthesis examples herein. The method may further comprise the step of forming a disulfide bond between the thiol groups of the cysteine side chains at positions 2 and 7, e.g. by oxidative cyclization. In the case of solid phase synthesis, cyclization may be performed in situ on the solid phase (e.g. resin), i.e. before removal of the peptide from the solid phase.

N-alkyl groups

N-alkyl groups that may be present in an N-alkylated peptide bond in the context of compounds of the present invention include, but are not limited to, straight-chain and branched $C_{1-4}$ alkyl groups, e.g. $C_{1-3}$ alkyl groups, such as methyl, ethyl, 1-propyl or 2-propyl.

$C_{1-4}$ acyl groups

$C_{1-4}$ acyl groups that may be present as a group $R^1$ in the context of compounds of the present invention include formyl (i.e. methanoyl), acetyl (i.e. ethanoyl), propanoyl, 1-butanoyl and 2-methylpropanoyl groups.

$C_{1-4}$ alkyl groups

$C_{1-4}$ alkyl groups that may be present as a group $R^1$ in the context of compounds of the present invention include, but are not limited to, $C_{1-3}$ alkyl groups, such as methyl, ethyl, 1-propyl or 2-propyl.

$C_{1-3}$ alkyl groups

$C_{1-3}$ alkyl groups that may be present as a group $R^3$ in the context of compounds of the present invention include methyl, ethyl, 1-propyl and 2-propyl.

Half-life extending moieties M

As described herein, the N-terminal moiety $R^1$ in a compound of the invention may be a half-life extending moiety (sometimes referred to in the literature as, *inter alia,* a duration enhancing moiety or albumin binding moiety), optionally linked (covalently attached) to the peptide moiety Z via a linker moiety L. Among suitable half-life extending moieties are certain types of lipophilic substituents. Without wishing to be bound by any particular theory, it is thought that such lipophilic substituents (and other classes of half-life extending moieties) bind albumin in the blood stream, thereby shielding the compound of the invention from enzymatic degradation and thus possibly enhancing the half-life of the compound *in vivo.* The lipophilic substituent may also modulate the potency of the compound as an agonist to the amylin (calcitonin) receptor.

The lipophilic substituent may be attached to the N-terminal amino acid residue or to the linker L via an ester, a sulfonyl ester, a thioester, an amide, an amine or a sulfonamide. Accordingly it will be understood that preferably the lipophilic substituent includes an acyl group, a sulfonyl group, an N atom, an O atom or an S atom which forms part of the ester, sulfonyl ester, thioester, amide, amine or sulfonamide. Preferably, an acyl group in the lipophilic substituent forms part of an amide or ester with the amino acid residue or the linker.

The lipophilic substituent may comprise a hydrocarbon chain having from 10 to 24 C atoms, e.g. from 14 to 22 C atoms, e.g. from 16 to 20 C atoms. Preferably it has at least 14 C atoms, and preferably has 20 C atoms or fewer. For example, the hydrocarbon chain may contain 14, 15, 16, 17, 18, 19 or 20 carbon atoms. The hydrocarbon chain may be linear or branched, and may be saturated or unsaturated. Furthermore, it can include a functional group at the end of the lipophilic chain, e.g. a carboxylic acid group which may or may not be protected during synthesis. From the discussion above it will be understood that the hydrocarbon chain is preferably substituted with a moiety which forms part of the attachment to the N-terminal amino acid residue of the peptide moiety Z or to the linker L, for example an acyl group, a sulfonyl group, an N atom, an O atom or an S atom.

Most preferably, the hydrocarbon chain is substituted with an acyl group, and accordingly the hydrocarbon chain may be part of an alkanoyl group, for example a dodecanoyl, 2-butyloctanoyl, tetradecanoyl, hexadecanoyl, heptadecanoyl, octadecanoyl, nonadecanoyl or eicosanoyl group. Examples of functionalized hydrocarbon chains are 15-carboxypentadecanoyl, 17-carboxy-heptadecanoyl and 19-carboxy-nonadecanoyl.

As mentioned above, a lipophilic substituent M may be linked to the N-terminal amino acid residue (X0 or X1 as appropriate) of Z via a linker L. In embodiments, the linker moiety L may itself comprise one, two, three or more linked sub-moieties $L^1$, $L^2$, $L^3$, ..etc. When the linker L comprises only one such moiety, it is attached to the lipophilic substituent and to the N-terminal amino acid residue of Z. The linker may then be attached to the lipophilic substituent and to the N-terminal amino acid residue of Z independently by means of an ester, a sulfonyl ester, a thioester, an amide, an amine or a sulfonamide bond. Accordingly, it may include two moieties independently selected from acyl, sulfonyl, an N atom, an O atom and an S atom. The linker may consist of a linear or branched $C_{1-10}$ hydrocarbon chain or more preferably a linear $C_{1-5}$ hydrocarbon chain. Furthermore the linker can be substituted with one or more substituents selected from $C_{1-6}$ alkyl, $C_{1-6}$ alkylamine, $C_{1-6}$ alkylhydroxy and $C_{1-6}$ alkylcarboxy.

In some embodiments the linker may be, for example, a residue of any naturally occurring or non-naturally

occurring amino acid. For example, the linker may be a residue of Gly, Pro, Ala, Val, Leu, Ile, Met, Cys, Phe, Tyr, Trp, His, Lys, Arg, Gin, Asn, α-Glu, γ-Glu, ε-Lys, Asp, Ser, Thr, Gaba, Aib, β-Ala (i.e. 3-aminopropanoyl), 4-aminobutanoyl, 5-aminopentanoyl, 6-aminohexanoyl, 7-aminoheptanoyl, 8-aminooctanoyl, 9- aminononanoyl, 10-aminodecanoyl or 8Ado (i.e. 8-amino-3,6-dioxaoctanoyl). In certain embodiments, the linker is a residue of Glu, γ-Glu, ε -Lys, β-Ala, 4-aminobutanoyl, 8- aminooctanoyl or 8Ado. In the context of the present invention, γ-Glu and isoGlu are used interchangeably.

An example of a lipophilic substituent comprising a lipophilic moiety M and linker L is shown in the formula below:

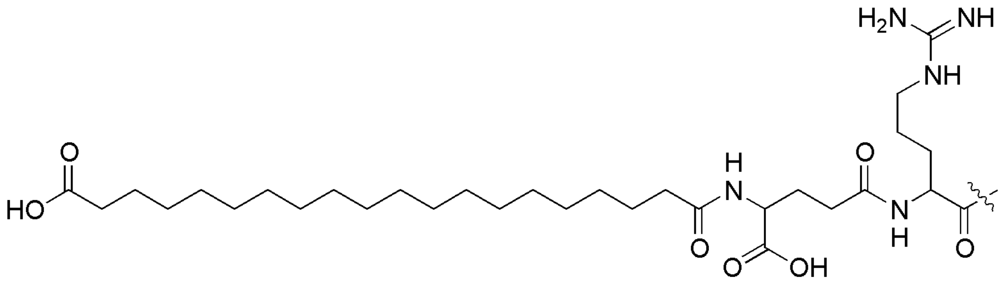

Here, the backbone nitrogen of an Arg residue is covalently attached to a γ-Glu linker (L) via an amide linkage. A 19-carboxy-nonadecanoyl group is covalently attached to the γ-Glu linker via an amide linkage. This combination of lipophilic moiety and linker, attached to an Arg residue, may be referred to by the shorthand notation [CD]-isoGluR, e.g. when shown in formulae of specific compounds.

The skilled person will be well aware of suitable techniques for preparing the compounds employed in the context of the invention. For examples of suitable chemistry, see, e.g., WO98/08871, WO00/55184, WO00/55119, Madsen et al (J. Med. Chem. 2007, 50, 6126-32), and Knudsen et al. 2000 (J. Med Chem. 43, 1664-1669).

The hydrocarbon chain in a lipophilic substituent may be further substituted. For example, it may be further substituted with up to three substituents selected from $NH_2$, OH and COOH. If the hydrocarbon chain is further substituted, it is preferably further substituted with only one substituent. Alternatively or additionally, the hydrocarbon chain may include a cycloalkane or heterocycloalkane moiety, for example as shown below:

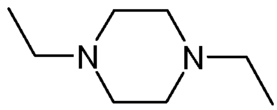

In some embodiments, the cycloalkane or heterocycloalkane moiety is a six-membered ring, e.g a piperidine ring.

In alternative embodiments of the present invention, the N-terminal amino acid (X0 or X1) of Z in a compound of the invention may be linked (covalently attached) to a biotinylic substituent, optionally via a linker moiety L.. Without wishing to be bound by any particular theory, it is likewise believed that such biotinylic substituents bind to albumin in the blood stream, thereby shielding the compound of the invention from enzymatic degradation and thus possibly enhancing the half-life of the compound *in vivo.* A linker, when present, may provide spacing between the peptide moiety Z and the biotinylic substituent.

The biotinylic substituent may be attached to the N-terminal amino acid residue (X0 or X1) or to the linker via an maleimide ester bond, a sulfonyl ester bond, a thioester bond, an amide bond, an amine bond or a sulfonamide bond. Accordingly it will be understood that the biotinylic substituent preferably comprises an maleimido group, an acyl group, a sulfonyl group, an N atom, an O atom or an S atom which forms part of the ester, sulfonyl ester, thioester, amide, amine or sulfonamide bond in question.

Examples of biotinylic substituents may include

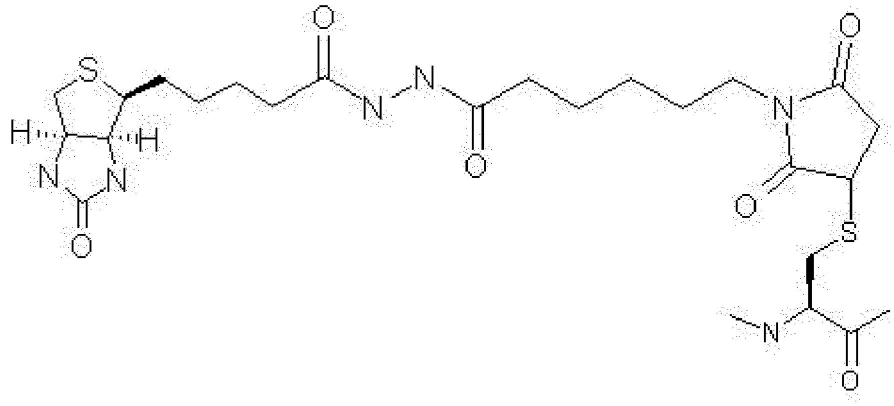

Biotin is known as Vitamin H or Coenzyme R, and is a water-soluble B-complex vitamin (vitamin B7). It has been shown to increase oral uptake of certain drugs.

Efficacy of compounds

The compounds of the invention are amylin receptor agonists, i.e. they are capable of binding to, and inducing signalling by, one or more receptors or receptor complexes regarded as physiological receptors for human amylin. These include the human calcitonin receptor hCTR2, as well as complexes comprising the human calcitonin receptor hCTR2 and at least one of the human receptor activity modifying proteins designated hRAMP1, hRAMP2 and hRAMP3. Complexes between hCTR2 and hRAMP1, hRAMP2 and hRAMP3 are designated hAMYR1, hAMYR2 and hAMYR3 (i.e. human amylin receptors 1, 2 and 3) respectively.

Without wishing to be bound by theory, a compound may be considered an amylin receptor agonist if it has agonist activity at one or more of hAMYR1, hAMYR2 and hAMYR3, e.g. against hAMYR1 and/or hAMYR3, e.g. at hAMYR3.

Typically an amylin receptor agonist will also have agonist activity at hCTR2 when expressed in the absence of hRAMP1, hRAMP2 and hRAMP3. Typically, the agonist will have activity at hCTR2 (when expressed in the absence of hRAMP1, hRAMP2 and hRAMP3) which is less than 10-fold higher than its activity at any one of hAMYR1, hAMYR2 and hAMYR3 (i.e. its activity at all of these receptors) in a comparable assay. Agonist activity at hCTR2 may be less than 5-fold higher than agonist activity at hAMYR1, hAMYR2 and hAMYR3, substantially equal to (e.g. +/- 10%) agonist activity at hAMYR1, hAMYR2 and hAMYR3, or less than agonist activity at hAMYR1, hAMYR2 and hAMYR3. In this regard, it may be sufficient just to compare activity between hCTR2 and hAMYR3.

The ability to induce cAMP formation (i.e. to induce adenylate cyclase activity) as a result of binding to the relevant receptor or receptor complex is typically regarded as indicative of agonist activity. Other intracellular signaling pathways or events may also be used as readouts for amylin receptor agonist activity. These may include calcium release, β-arrestin recruitment, receptor internalization, kinase activation or inactivation, lipase activation, inositol phosphate release, diacylglycerol release or nuclear transcription factor translocation.

A suitable comparable assay format would utilize cells which express hCTR2 and which differ only in their expression of hRAMP1, 2 and 3. For example, a "base" cell line which does not express any of hCTR2, hRAMP1, hRAMP2 and hRAMP3 may be engineered to generate cells which express (i) hCTR2, and (ii) one of hAMYR1, hAMYR2 and hAMYR3 (i.e. hCTR2 plus one of hRAMP1, hRAMP2 and hRAMP3), e.g. hAMYR3. The base cells will typically be mammalian cells and may be primate cells. They may be non-human primate cells. Preferably the base cell does not express any of CTR2, RAMP1, RAMP2 or RAMP3 (whether human, or native to the base cell if the base cell is non-human). The base cells may be fibroblast cells. Suitable non-human fibroblast base cells include COS7 cells, from African green monkey, which do not express native CTR2 or RAMPs.

Comparative activity may be measured by any suitable means, such as via determination of $EC_{50}$ values as described below. It will be apparent that the same biological read-out must be for both receptor types.

Compounds of the present invention may exhibit a number of advantageous properties in relation to human amylin and existing analogues thereof, such as pramlinitide, IAPP-GI, and analogues described in WO2012/168430, WO2012/168431 and WO2012/168432. In particular, compounds of the invention exhibit improved potency at one or more of the receptors hCTR2, hAMRY1, hAMRy2 or hAMYR3 as compared to human amylin. Additionally, compounds of the invention may exhibit improved solubility in aqueous media, especially at pH values in the range from 4 to 7.5, or at a range of pH values across that range. Moreover, compounds of the present invention may exhibit reduced tendency to undergo fibrillation in pharmaceutically relevant aqueous media, especially at pH values in the range from 4 to 7, or at a range of pH values across that range. Furthermore, compounds of the present invention may exhibit improved

chemical stability (i.e. reduced tendency to undergo chemical degradation) in aqueous media, especially at pH values in the range from 4 to 9, or at a range of pH values across that range.

Compounds of the invention may thus be well suited for formulation in acidic media (e.g. pH 4) and in neutral or near-neutral media (e.g. pH 7 or 7.4). In contrast to pramlintide, for example, which generally exhibits poor chemical stability and rapid fibrillation in pharmaceutically relevant aqueous media at neutral pH, compounds of the invention may be thus well suited for co-formulation with, for example, insulin, various insulin analogues and/or other therapeutic (e.g. anti-diabetic or anti-obesity) agents that require a neutral or near-neutral formulation pH.

In general it is preferred to use a biological assay which measures intracellular signalling caused by binding of the compound to the relevant receptor, as discussed above. Activation of the calcitonin/amylin receptor by compounds of the invention (which behave as agonists of the receptor) induces cAMP formation and activation of other intracellular signaling pathways and events. Thus, production of cAMP or any other suitable parameter in suitable cells expressing the receptor can be used to monitor agonist activity towards the receptor.

The skilled person will be aware of suitable assay formats, and examples are provided below. For example, the assays may make use of the human calcitonin receptor (hCTR2) having primary accession number GI: 4502547 (NP_001733.1) or the hAMYR3 receptor (see Example 2, below). Where sequences of precursor proteins are referred to, it should be understood that assays may make use of the mature protein, lacking the signal sequence.

$EC_{50}$ values may be used as a numerical measure of agonist potency at a given receptor. An $EC_{50}$ value is a measure of the concentration of a compound required to achieve half of that compound's maximal activity in a particular assay. Thus, for example, a compound having $EC_{50}$ [hCTR2] lower than the $EC_{50}$ [hCTR2] of native amylin, or lower than that of pramlintide, in a particular assay may be considered to have higher potency or activity at the receptor than amylin, or higher than that of pramlintide, respectively.

In some embodiments of compounds of the present invention, the $EC_{50}$ towards hCTR2 is below 1.4 nM.

In some embodiments of compounds of the present invention, the $EC_{50}$ towards hCTR2 is below 0.8 nM.

In further embodiments of compounds of the present invention, the $EC_{50}$ towards hCTR2 is below 0.4 nM.

In still further embodiments of compounds of the present invention, the $EC_{50}$ towards hCTR2 is below 0.2 nM.

In some embodiments of compounds of the present invention, the $EC_{50}$ towards hAMYR3 is below 1 nM.

In some embodiments of compounds of the present invention, the $EC_{50}$ towards hAMYR3 is below 0.5 nM.

In some embodiments of compounds of the present invention, the $EC_{50}$ towards hAMYR3 is below 0.4 nM.

In some embodiments of compounds of the present invention, the $EC_{50}$ towards hAMYR3 is below 0.3 nM.

In some embodiments of compounds of the present invention, the $EC_{50}$ towards hAMYR3 is below 0.2 nM.

For example, the $EC_{50}$ at hCTR2 (when expressed in the absence of hRAMP1, hRAMP2 and hRAMP3) may be less than 10-fold lower than the $EC_{50}$ at any or all of hAMYR1, hAMYR2 and hAMYR3, e.g. at hAMYR3.

The $EC_{50}$ at hCTR2 (when expressed in the absence of hRAMP1, hRAMP2 and hRAMP3) may be less than 5-fold lower than the $EC_{50}$ at any or all of hAMYR1, hAMYR2 and hAMYR3, e.g. at hAMYR3.

The $EC_{50}$ at hCTR2 (when expressed in the absence of hRAMP1, hRAMP2 and hRAMP3) may be substantially equal to (e.g. +/- 10%) the $EC_{50}$ at any or all of hAMYR1, hAMYR2 and hAMYR3, e.g. at hAMYR3.

The $EC_{50}$ at hCTR2 (when expressed in the absence of hRAMP1, hRAMP2 and hRAMP3) may be higher than (e.g. +/- 10%) the $EC_{50}$ at any or all of hAMYR1, hAMYR2 and hAMYR3, e.g. at hAMYR3.

Such assays may be performed under the conditions described in Examples 2 and 3.

Additionally or alternatively, the compounds may show excellent solubility. For example, the compounds may show solubility of greater than or equal to 1 mg/ml at pH 4, pH 5, pH 6, pH 7 and/or pH 7.5, e.g. at 25°C, e.g. under the conditions described in Example 4.

Additionally or alternatively, the compounds may show excellent resistance to fibrillation. For example, they may show no detectable fibrillation after 96 hours at pH 4.0 and/or pH 7.0, e.g. at 40°C, e.g. under the conditions described in Example 5.

Additionally or alternatively, the compounds may show excellent chemical stability, i.e. resistance to degradation in solution. For example, they may retain at least 70% purity, at least 75% purity, at least 80% purity, at least 85% purity, at least 90% purity, or at least 95% purity after incubation at pH 4, pH 7.5, and/or pH 9 at 40°C for 7 days, e.g. under the conditions described in Example 6.

## Therapeutic uses

A number of therapeutic uses of peptides of the invention have already been mentioned above. The use of a peptide of the invention also encompasses the use of pharmaceutically acceptable salt or solvate thereof.

Exemplary therapeutic uses include, but are not limited to, the treatment of Alzheimer's disease, diabetes, type 1 diabetes, type 2 diabetes, pre-diabetes, insulin resistance syndrome, impaired glucose tolerance (IGT), disease states associated with elevated blood glucose levels, metabolic disease, hyperglycemia, hypertension, atherogenic dyslipidemia, arteriosclerosis (e.g. atherosclerosis), diabetic heart (including diabetic cardiomyopathy and heart failure as a

diabetic complication), coronary heart disease, heart failure, peripheral artery disease, stroke, microvascular disease, gastric disease, or metabolic syndrome.

The compounds of the invention may provide an attractive treatment option for, *inter alia,* obesity and metabolic diseases.

Metabolic syndrome is characterized by a group of metabolic risk factors in one person. They include abdominal obesity (excessive fat tissue around the abdominal internal organs), atherogenic dyslipidemia (blood fat disorders including high triglycerides, low HDL cholesterol and/or high LDL cholesterol, which foster plaque buildup in artery walls), elevated blood pressure (hypertension), insulin resistance and glucose intolerance, prothrombotic state (e.g. high fibrinogen or plasminogen activator inhibitor-1 in the blood), and proinflammatory state (e.g., elevated C-reactive protein in the blood).

Individuals with metabolic syndrome are at increased risk of coronary heart disease and other diseases related to other manifestations of arteriosclerosis (e.g. stroke and peripheral vascular disease). The dominant underlying risk factor for this syndrome appears to be abdominal obesity.

The compounds of the present invention may be used as pharmaceutical agents for preventing weight gain, promoting weight loss, reducing excess body weight or treating obesity (e.g. by control of appetite, feeding, food intake, calorie intake, and/or energy expenditure), including morbid obesity, as well as associated diseases and health conditions including but not limited to obesity linked inflammation, obesity linked gallbladder disease and obesity induced sleep apnea. The compounds of the invention may also be used for treatment or prevention of metabolic syndrome, hypertension, atherogenic dyslipidemia, non-alcoholic steatohepatitis (NASH), hepatic steatosis ("fatty liver"), atherosclerosis, arteriosclerosis, coronary heart disease and stroke, all of which can be associated with obesity. The effects of the compounds of the invention on these conditions may be mediated in whole or in part via an effect on body weight, or may be independent thereof.

## Pharmaceutical compositions

It is to be understood that the inclusion of a peptide of the invention (i.e. one or more peptides of the invention) in a pharmaceutical composition also encompasses inclusion of a pharmaceutically acceptable salt or solvate of a peptide of the invention.

The invention also provides a pharmaceutical composition comprising a peptide, or a pharmaceutically acceptable salt or solvate thereof, according to the invention.

The invention also provides a pharmaceutical composition comprising a peptide, or a pharmaceutically acceptable salt or solvate thereof, for treating a variety of conditions, diseases, or disorders as disclosed herein elsewhere (see, e.g., therapeutic uses, supra).

The peptides of the present invention may be formulated as pharmaceutical compositions which are suited for administration with or without storage, and which typically comprise a therapeutically effective amount of at least one peptide of the invention, together with a pharmaceutically acceptable carrier, excipient or vehicle.

The term "pharmaceutically acceptable carrier" includes any of the standard pharmaceutical carriers. Pharmaceutically acceptable carriers for therapeutic use are well known in the pharmaceutical art and are described, for example, in "Remington's Pharmaceutical Sciences", 17th edition, Alfonso R. Gennaro (Ed.), Mark Publishing Company, Easton, PA, USA, 1985. For example, sterile saline and phosphate-buffered saline at slightly acidic or physiological pH may be used. Suitable pH-buffering agents may, e.g., be phosphate, citrate, acetate, tris(hydroxymethyl)aminomethane (TRIS), N-tris(hydroxymethyl)methyl-3-aminopropanesulfonic acid (TAPS), ammonium bicarbonate, diethanolamine, histidine, arginine, lysine or acetate (e.g. as sodium acetate), or mixtures thereof. The term further encompasses any carrier agents listed in the US Pharmacopeia for use in animals, including humans.

A pharmaceutical composition of the invention may be in unit dosage form. In such form, the composition is divided into unit doses containing appropriate quantities of the active component or components. The unit dosage form may be presented as a packaged preparation, the package containing discrete quantities of the preparation, for example, packaged tablets, capsules or powders in vials or ampoules. The unit dosage form may also be, e.g., a capsule, cachet or tablet in itself, or it may be an appropriate number of any of these packaged forms. A unit dosage form may also be provided in single-dose injectable form, for example in the form of a pen device containing a liquid-phase (typically aqueous) composition. Compositions may be formulated for any suitable route and means of administration. Pharmaceutically acceptable carriers or diluents include those used in formulations suitable for e.g. oral, intravitreal, rectal, vaginal, nasal, topical, enteral or parenteral (including subcutaneous (sc), intramuscular (im), intravenous (iv), intradermal and transdermal) administration or administration by inhalation. The formulations may conveniently be presented in unit dosage form and may be prepared by any of the methods well known in the art of pharmaceutical formulation.

Subcutaneous or transdermal modes of administration may in some cases be suitable for peptides of the invention.

Further embodiments relate to devices, dosage forms and packages used to deliver the pharmaceutical formulations of the present invention. Thus, at least one peptide in a stable or preserved formulation or solution described herein can be administered to a patient in accordance with the present invention via a variety of delivery methods, including by sc or im injection, or by transdermal, pulmonary or transmucosal administration, or by implant, or by use of an osmotic pump, cartridge, micro-pump or other means recognized by a person of skill in the art.

Still further embodiments relate to oral formulations and oral administration. Formulations for oral administration may rely on the co-administration of adjuvants (e.g. resorcinols and/or nonionic surfactants such as polyoxyethylene oleyl ether and n-hexadecylpolyethylene ether) to artificially increase the permeability of the intestinal walls, and/or the co-administration of enzymatic inhibitors (e.g. pancreatic trypsin inhibitors, diisopropylfluorophosphate (DFF) or trasylol) to inhibit enzymatic degradation. The active constituent compound of a solid-type dosage form for oral administration can be mixed with at least one additive, such as sucrose, lactose, cellulose, mannitol, trehalose, raffinose, maltitol, dextran, starches, agar, alginates, chitins, chitosans, pectins, gum tragacanth, gum arabic, gelatin, collagen, casein, albumin, synthetic or semisynthetic polymer, or glyceride. These dosage forms can also contain other type(s) of additives, e.g. an inactive diluting agent, a lubricant (such as magnesium stearate), a paraben, a preserving agent (such as sorbic acid, ascorbic acid or alpha-tocopherol), an antioxidant (such as cysteine),a disintegrant, binder, thickener, buffering agent, pH-adjusting agent, sweetening agent, flavoring agent or perfuming agent.

Dosages

A typical dosage of a peptide of the invention as employed in the context of the present invention may be in the range from about 0.0001 to about 100 mg/kg body weight per day, such as from about 0.0005 to about 50 mg/kg body weight per day, such as from about 0.001 to about 10 mg/kg body weight per day, e.g. from about 0.01 to about 1 mg/kg body weight per day, administered in one or more doses, such as from one to three doses. The exact dosage employed will depend, *inter alia,* on: the nature and severity of the disease or disorder to be treated, on the sex, age, body weight and general condition of the subject to be treated, on possible other, concomitant, disease or disorder that is undergoing or is to undergo treatment, as well as on other factors that will be known to a medical practitioner of skill in the art.

A peptide of the invention may be administered continuously (e.g. by intravenous administration or another continuous drug administration method), or may be administered to a subject at intervals, typically at regular time intervals, depending on the desired dosage and the pharmaceutical composition selected by the skilled practitioner for the particular subject. Regular administration dosing intervals include, e.g., once daily, twice daily, once every two, three, four, five or six days, once or twice weekly, once or twice monthly, and the like. Such regular peptide administration regimens may, in certain circumstances such as, e.g., during chronic long-term administration, be advantageously interrupted for a period of time so that the medicated subject reduces the level of, or stops taking, the medication, often referred to as taking a "drug holiday." Drug holidays are useful for, e.g., maintaining or regaining sensitivity to a drug especially during long-term chronic treatment, or to reduce unwanted side-effects of long-term chronic treatment of the subject with the drug. The timing of a drug holiday depends on the timing of the regular dosing regimen and the purpose for taking the drug holiday (e.g., to regain drug sensitivity and/or to reduce unwanted side effects of continuous, long- term administration). In some embodiments, the drug holiday may be a reduction in the dosage of the drug (e.g. to below the therapeutically effective amount for a certain interval of time). In other embodiments, administration of the drug is stopped for a certain interval of time before administration is started again using the same or a different dosing regimen (e.g. at a lower or higher dose and/or frequency of administration). A drug holiday of the invention may thus be selected from a wide range of time-periods and dosage regimens. An exemplary drug holiday is two or more days, one or more weeks, or one or more months, up to about 24 months of drug holiday. So, for example, a regular daily dosing regimen with a peptide of the invention may, for example, be interrupted by a drug holiday of a week, or two weeks, or four weeks, after which time the preceding, regular dosage regimen (e.g. a daily or a weekly dosing regimen) is resumed. A variety of other drug holiday regimens are envisioned to be useful for administering peptides of the invention.

Thus, the peptide may be delivered via an administration regime which comprises two or more administration phases separated by respective drug holiday phases.

During each administration phase, the peptide is administered to the recipient subject in a therapeutically effective amount according to a pre-determined administration pattern. The administration pattern may comprise continuous administration of the drug to the recipient subject over the duration of the administration phase. Alternatively, the administration pattern may comprise administration of a plurality of doses of the peptide to the recipient subject, wherein said doses are spaced by dosing intervals.

A dosing pattern may comprise at least two doses per administration phase, at least five doses per administration phase, at least 10 doses per administration phase, at least 20 doses per administration phase, at least 30 doses per administration phase, or more.

Said dosing intervals may be regular dosing intervals, which may be as set out above, including once daily, twice daily, once every two, three, four, five or six days, once or twice weekly, once or twice monthly, or a regular and

even less frequent dosing interval, depending on the particular dosage formulation, bioavailability, and pharmacokinetic profile of the peptide.

An administration phase may have a duration of at least two days, at least a week, at least 2 weeks, at least 4 weeks, at least a month, at least 2 months, at least 3 months, at least 6 months, or more.

Where an administration pattern comprises a plurality of doses, the duration of a possible following drug holiday phase is longer than the dosing interval used in that administration pattern. Where the dosing interval is irregular, the duration of a drug holiday phase may be greater than the mean interval between doses over the course of the administration phase. Alternatively the duration of the drug holiday may be longer than the longest interval between consecutive doses during the administration phase.

The duration of a possible drug holiday phase may be at least twice that of the relevant dosing interval (or mean thereof), at least 3 times, at least 4 times, at least 5 times, at least 10 times, or at least 20 times that of the relevant dosing interval or mean thereof.

Within these constraints, a drug holiday phase may have a duration of at least two days, at least a week, at least 2 weeks, at least 4 weeks, at least a month, at least 2 months, at least 3 months, at least 6 months, or more, depending on the administration pattern during the previous administration phase.

An administration regime entailing the use of drug holiday comprises at least 2 administration phases. Consecutive administration phases are separated by respective drug holiday phases. Thus the administration regime may comprise at least 3, at least 4, at least 5, at least 10, at least 15, at least 20, at least 25, or at least 30 administration phases, or more, each separated by respective drug holiday phases.

Consecutive administration phases may utilise the same administration pattern, although this may not always be desirable or necessary. However, if other drugs or active agents are administered in combination with a peptide of the invention, then typically the same combination of drugs or active agents is given in consecutive administration phases. In certain embodiments, the recipient subject is a human.

Devices and kits

In some embodiments, the invention relates to a device comprising one or more peptides, or pharmaceutically acceptable salts or solvates thereof, of the invention, for delivery of the peptide to a subject. Thus, one or more peptides or pharmaceutically acceptable salts or solvates thereof can be administered to a patient in accordance with the present invention via a variety of delivery methods, including: intravenous, subcutaneous, intramuscular or intraperitoneal injection; oral administration; transdermal administration; pulmonary or transmucosal administration; administration by implant, osmotic pump, cartridge or micro pump; or by other means recognized by a person of skill in the art.

In some embodiments, the invention relates to a kit comprising one or more peptides, or pharmaceutically acceptable salts or solvates thereof, of the invention. In other embodiments, the kit comprises one or more pharmaceutical compositions comprising one or more peptides or pharmaceutically acceptable salts or solvates thereof. In certain embodiments, the kit further comprises packaging and/or instructions for use.

Combination therapy

As noted above, it will be understood that reference in the following to a peptide of the invention also extends to a pharmaceutically acceptable salt or solvate thereof, as well as to a composition comprising more than one different peptide of the invention.

A peptide of the invention may have some benefit if administered in combination with an anti-diabetic agent of known type, including, but not limited to, metformin, a sulfonylurea, a glinide, a DPP-IV inhibitor, a glitazone, a GLP-1 or a GLP-1 analogue, an exendin-4 or an exendin-4 analogue, any other GLP-1 receptor agonist including Liraglutide, Dulaglutide or Albiglutide or a glucagon-GLP-1 dual agonist (e.g. as described in WO2008/101017, WO2008/152403, WO2010/070252, WO2010/070253, WO2010/070255, WO2010/070251, WO2011/006497, WO2011/160630, WO2011/160633, WO2013/092703, WO2014/041195), an SGLT2 inhibitor (i.e. an inhibitor of sodium-glucose transport, e.g. a gliflozin such as empagliflozin, canagliflozin, dapagliflozin or ipragliflozin), a GPR40 agonist (FFAR1/FFA1 agonist, e.g. fasiglifam), or an insulin or an insulin analogue. Examples of appropriate insulin analogues include, but are not limited to, Lantus™, Novorapid™, Humalog™, Novomix™, Actraphane™ HM, Levemir™ Degludec™ and Apidra™. Other relevant anti-diabetic agents in this connection include GLP-1 receptor agonists, such as exenatide (Byetta™ and Bydureon™ exendin-4) and Byetta LAR™, lixisenatide (Lyxumia™) and liraglutide (Victoza™).

A peptide of the invention may be administered as part of a combination therapy together with another active agent for the treatment of the disease or disorder in question, e.g. obesity, metabolic syndrome, dyslipidemia or hypertension, and in such cases, the two active agents may be given together or separately, e.g. as constituents in the same pharmaceutical composition or formulation, or as separate formulations.

Moreover, a peptide of the invention may be used in combination with an anti-obesity agent of known type,

including, but not limited to, peptide YY or an analogue thereof, neuropeptide Y (NPY) or an analogue thereof, a cannabinoid receptor 1 antagonist, a lipase inhibitor, Human prolslet Peptide (HIP), a melanocortin receptor 4 agonist, liraglutide (Victoza™), Orlistat™, Sibutramine™, phentermine, a melanin concentrating hormone receptor 1 antagonist, CCK, amylin, pramlintide and leptin, as well as analogues thereof.

A peptide of the invention may further be used in combination with an anti-hypertension agent of a known type, including, but not limited to, an angiotensin-converting enzyme inhibitor, an angiotensin II receptor blocker, a diuretic, a beta-blocker and a calcium channel blocker.

A peptide of the invention may still further be used in combination with an anti-dyslipidemia agent of known type, including, but not limited to, a statin, a fibrate, a niacin and a cholesterol absorption inhibitor.

A peptide of the invention may also be used in combination with a proton pump inhibitor (i.e. a pharmaceutical agent possessing pharmacological activity as an inhibitor of $H^+/K^+$-

ATPase) of known type, including, but not limited to, an agent of the benzimidazole derivative type or of the imidazopyridine derivative type, such as Omeprazole™, Lansoprazole™, Dexlansoprazole™, Esomeprazole™, Pantoprazole™, Rabeprazole™, Zolpidem™, Alpidem™, Saripidem™ or Necopidem™.

In addition, with regard to anti-inflammatory treatment, a peptide of the invention may be beneficial if administered in combination with an anti-inflammatory agent of known type, including, but not limited to:

steroids and corticosteroids, such as beclomethasone, methylprednisolone, betamethasone, prednisone, dexamethasone, and hydrocortisone;

non-steroidal antiinflammatory agents (NSAIDs), such as propionic acid derivatives (e.g. alminoprofen, benoxaprofen, bucloxic acid, carprofen, fenbufen, fenoprofen, fluprofen, flurbiprofen, ibuprofen, indoprofen, ketoprofen, miroprofen, naproxen, oxaprozin, pirprofen, pranoprofen, suprofen, tiaprofenic acid and tioxaprofen); acetic acid derivatives (e.g. indomethacin, acemetacin, alclofenac, clidanac, diclofenac, fenclofenac, fenclozic acid, fentiazac, furofenac, ibufenac, isoxepac, oxpinac, sulindac, tiopinac, tolmetin, zidometacin and zomepirac); fenamic acid derivatives (e.g. flufenamic acid, meclofenamic acid, mefenamic acid, niflumic acid and tolfenamic acid); biphenylcarboxylic acid derivatives (e.g. diflunisal and flufenisal); oxicams (e.g. isoxicam, piroxicam, sudoxicam and tenoxicam); salicylates (e.g. acetylsalicylic acid and sulfasalazine); and pyrazolones (e.g. apazone, bezpiperylon, feprazone, mofebutazone, oxyphenbutazone and phenylbutazone);

COX II inhibitors, such as rofecoxib and celecoxib; preparations of interferon beta (e.g. interferon beta-1a or interferon beta-1b);

and certain other compounds, such as 5-aminosalicylic acid and prodrugs and pharmaceutically acceptable salts thereof.

Metformin has also been demonstrated to have anti-inflammatory properties (see, e.g., Haffner et al., Diabetes 54: 1566-1572 (2005)) and as such may also be useful in combination with the peptides of the invention.

## EXAMPLES

The following examples demonstrate certain specific embodiments of the present invention. The following examples were carried out using standard techniques that are well known and routine to those of skill in the art, except where otherwise described in detail. It is to be understood that these examples are for illustrative purposes only and do not purport to be wholly definitive as to conditions or scope of the invention. As such, they should not be construed as limiting the scope of the present invention in any way.

Abbreviations employed in the examples include:

| | |
|---|---|
| Acm: | acetaminomethyl |
| DCM: | dichloromethane |
| DMF: | N,N-dimethylformamide |
| HATU: | 2-(7-aza-1H-benzotriazole-1-yl)-1,1,3,3-tetramethyluronium hexafluorophosphate |
| DIPEA: | diisopropylethylamine |
| DODT: | 3,6-dioxa-1,8-octanedithiol |
| EtOH: | ethanol |
| $Et_2O$: | diethyl ether |
| TFA: | trifluoroacetic acid |
| TIS: | triisopropylsilane |

| | |
|---|---|
| MeCN: | acetonitrile |
| HPLC: | high performance liquid chromatography |
| RP-HPLC: | reverse phase high performance liquid chromatography |
| MS: | mass spectrometry |
| ESI-MS: | electron spray ionization mass spectrometry |
| IBMX: | 3-isobutyl-1-methylxanthine |
| BSA: | bovine serum albumin |
| cAMP: | cyclic adenosine monophosphate |
| DMEM: | Dulbecco's Modified Eagle Medium |
| FCS: | fetal calf serum |
| HEPES: | N-2-hydroxyethylpiperazine-N'-2-ethanesulfonic acid |
| p-ERK: | phosphorylated extracellular regulated kinase |
| PBS: | phosphate-buffered saline |
| Boc: | tert-butoxycarbonyl |
| Fmoc: | 9-fluorenylmethoxycarbonyl |
| Trt: | trityl (i.e. triphenylmethyl) |
| NEP: | N-ethylpyrrolidone |
| NMP | N-methylpyrrolidone |
| v/v: | volume/volume |
| w/v: | weight/volume |

The following examples are provided to illustrate certain embodiments of the invention and are not intended to limit the scope of the invention.

Measurement of physiological parameters

Whole-blood glucose levels were determined on tail-vein blood samples by the Biosen (EKF Diagnostic, Germany) enzyme-based electrode method. Blood samples were analyzed for glycated hemoglobin (HbA1c) using a Cobas c111 analyzer (Roche Diagnostics, Mannheim, Germany). Plasma insulin levels were measured using a Meso Scale Discovery (MSD) system (Rockville, MD, USA). Liver fat content was determined by magnetic resonance (MR) scanning using an Echo systems MR scanner. Fat depots were measured by weighing of excised fat.

The following compounds are disclosed. Some represent compounds of the invention, as listed elsewhere in this specification, while others provide useful background.

| | |
|---|---|
| [19CD]-isoGlu-RC()NTATC()ATQRLAEFLHHSSNNF-Gly(Me)-A-Ile(Me)-LSSTNVGSNTP-NH2 | Compd. 1 |
| [19CD]-isoGlu-RC()NTATC()ATQRLAEFLHHSSNNF-Gly(Me)-Ile(Me)-LSSTNVGSNTP-NH2 | Compd. 2 |
| [19CD]-isoGlu-KC()NTATC()ATQRLAEFLHHSSNNF-Gly(Me)-A-Ile(Me)-LSSTNVGSHTY-NH2 | Compd. 3 |
| [19CD]-isoGlu-KC()NTATC()ATQRLAEFLHHSSNNF-Gly(Me)-Ile(Me)-LSSTNVGSHTY-NH2 | Compd. 4 |
| [19CD]-isoGlu-AC()NTATC()ATQRLAEFLHHSSNNF-Gly(Me)-A-Ile(Me)-LSSTNVGSNTP-NH2 | Compd. 5 |
| [19CD]-isoGlu-RC()NTATC()ATQR-DLeu-AEFLHHSSNNF-Gly(Me)-A-Ile(Me)-LSSTNVGSNTP-NH2 | Compd. 6 |
| [19CD]-isoGlu-RC()TATC()ATQRL-DAla-EFLHHSSNNF-Gly(Me)-A-Ile(Me)-LSSTNVGSNTP-NH2 | Compd. 7 |
| [19CD]-isoGlu-RC()NTATC()ATQRLA-DGlu-FLHHSSNNF-Gly(Me)-A-Ile(Me)-LSSTNVGSNTP-NH2 | Compd. 8 |
| [19CD]-isoGlu-RC()NTATC()ATQRLAE-DPhe-LHHSSNNF-Gly(Me)-A-Ile(Me)-LSSTNVGSNTP-NH2 | Compd. 9 |
| [19CD]-isoGlu-RC()NTATC()ATQRLAEF-DLeu-HHSSNNF-Gly(Me)-A-Ile(Me)-LSSTNVGSNTP-NH2 | Compd. 10 |
| [19CD]-isoGlu-RC()NTATC()ATQRLAEFL-DHis-HSSNNF-Gly(Me)-A-Ile(Me)-LSSTNVGSNTP-NH2 | Compd. 11 |
| [19CD]-isoGlu-RC()NTATC()ATQRLAEFLH-DHis-SSNNF-Gly(Me)-A-Ile(Me)-LSSTNVGSNTP-NH2 | Compd. 12 |
| [19CD]-isoGlu-RC()NTATC()ATQRLAEFLHHSSNN-DPhe-Gly(Me)-A-Ile(Me)-LSSTNVGSNTP-NH2 | Compd. 13 |
| [19CD]-isoGlu-RC()NTATC()ATQRLAEFLHHSSNNF-Gly(Me)-DAla-Ile(Me)-LSSTNVGSNTP-NH2 | Compd. 14 |

(continued)

| | |
|---|---|
| [19CD]-isoGlu-RC()NTA TC()ATQRLAEFLHHSSNNF-Gly(Me)-A-Ile(Me)-DLeu-SSTNVGSNTP-NH2 | Compd. 15 |
| [19CD]-isoGlu-RC()NTATC()ATQRLAEFLHHSSNNFAA-Ile(Me)-LSSTNVGSNTP-NH2 | Compd. 16 |
| [19CD]-isoGlu-RC()NTATC()ATQRLAEFLHHSSNNF-DAla-A-Ile(Me)-LSSTNVGSNTP-NH2 | Compd. 17 |
| [19CD]-isoGlu-RC()NTATC()ATQRLAEFLHRSSNNF-Gly(Me)-A-Ile(Me)-LSSTNVGSNTP-NH2 | Compd. 18 |
| [19CD]-isoGlu-KC()NTATC()ATQRLAEFLHHSSNNF-Gly(Me)-A-Ile(Me)-LSSTNVGSNTP-NH2 | Compd. 19 |
| [19CD]-isoGlu-KC()NTATC()ATQRLAEFLHHSSNNF-Gly(Me)-Ile(Me)-LSSTNVGSNTP-NH2 | Compd. 20 |
| [19CD]-isoGlu-RC()NTATC()ATQRLAEFLRHSSNNF-Gly(Me)-A-Ile(Me)-LSSTNVGSNTP-NH2 | Compd. 21 |
| [19CD]-isoGlu-RC()NTATC()ATQRLAEFLRHSSNNF-Gly(Me)-Ile(Me)-LSSTNVGSNTP-NH2 | Compd. 22 |
| [19CD]-isoGlu-RC()NTATC()ATQRLAEFLHHSSNNF-Gly(Me)-A-Ile(Me)-L-DSer-STNVGSNTP-NH2 | Compd. 23 |
| [19CD]-isoGlu-RC()NTATC()ATQRLAEFLHHSSNNF-Gly(Me)-Ile(Me)-L-DSer-STNVGSNTP-NH2 | Compd. 24 |
| [19CD]-isoGlu-RC()NTATC()ATQRLAEFLHHSSNNF-Gly(Me)-A-Ile(Me)-LSTNVGSNTP-NH2 | Compd. 25 |
| [19CD]-isoGlu-RC()NTATC()ATQRLAEFLHHSSNNF-Gly(Me)-Ile(Me)-LSTNVGSNTP-NH2 | Compd. 26 |
| [19CD]-isoGlu-RC()NTATC()ATQRLAEFLHHSSNNF-Gly(Me)-ALSSTNVGSNTP-NH2 | Compd. 27 |
| [19CD]-isoGlu-RC()NTATC()ATQRLAEFLHHSSNNF-Gly(Me)-A-DIle-LSSTNVGSNTP-NH2 | Compd. 28 |
| [19CD]-isoGlu-RC()NTATC()ATQRLAEFLHH-DSer-SNNF-Gly(Me)-A-Ile(Me)-LSSTNVGSNTP-NH2 | Compd. 29 |
| [19CD]-isoGlu-RC()NTATC()ATQRLAEFLHH-DSer-SNNF-Gly(Me)-Ile(Me)-LSSTNVGSNTP-NH2 | Compd. 30 |
| [19CD]-isoGlu-RC()NTATC()ATQRLAEFLHHS-DSer-NNF-Gly(Me)-A-Ile(Me)-LSSTNVGSNTP-NH2 | Compd. 31 |
| [19CD]-isoGlu-RC()NTATC()ATQRLAEFLHHSS-DAsn-NF-Gly(Me)-A-Ile(Me)-LSSTNVGSNTP-NH2 | Compd. 32 |
| [19CD]-isoGlu-RC()NTATC()ATQRLAEFLHHSSN-DAsn-F-Gly(Me)-A-Ile(Me)-LSSTNVGSNTP-NH2 | Compd. 33 |
| [19CD]-isoGlu-RC()NTATC()ATQRLAEFLHHS-DSer-NNF-Gly(Me)-Ile(Me)-LSSTNVGSNTP-NH2 | Compd. 34 |
| [19CD]-isoGlu-RC()NTATC()ATQRLAEFLHHSS-DAsn-NF-Gly(Me)-Ile(Me)-LSSTNVGSNTP-NH2 | Compd. 35 |
| [19CD]-isoGlu-RC()NTATC()ATQRLAEFLHHSSN-DAsn-F-Gly(Me)-Ile(Me)-LSSTNVGSNTP-NH2 | Compd. 36 |
| [19CD]-isoGlu-RC()NTATC()ATQRLAEFLHHSSNNF-Gly(Me)-A-Ile(Me)-LSSNVGSNTP-NH2 | Compd. 37 |
| [19CD]-isoGlu-RC()NTATC()ATQRLAEFLHHSSNF-Gly(Me)-Ile(Me)-LSSTNVGSNTP-NH2 | Compd. 38 |
| [19CD]-isoGlu-RC()NTATC()ATQRLAEFLHSSNNF-Gly(Me)-A-Ile(Me)-LSSTNVGSNTP-NH2 | Compd. 39 |
| [19CD]-isoGlu-RC()NTATC()ATQRLAEFLHSSNNF-Gly(Me)-Ile(Me)-LSSTNVGSNTP-NH2 | Compd. 40 |
| [19CD]-isoGlu-RC()NTATC()ATQRLAEFLSSNNF-Gly(Me)-A-Ile(Me)-LSSTNVGSNTP-NH2 | Compd. 41 |
| [19CD]-isoGlu-RC()NTATC()ATQRLAEFLSSNNF-Gly(Me)-Ile(Me)-LSSTNVGSNTP-NH2 | Compd. 42 |
| [19CD]-isoGlu-HarC()NTATC()ATQRLAEFLHHSSNNF-Gly(Me)-A-Ile(Me)-LSSTNVGSNTP-NH2 | Compd. 43 |
| [19CD]-isoGlu-RC()NTATC()ATQRLAEFLHHSSNF-Gly(Me)-A-Ile(Me)-LSSTNVGSNTP-NH2 | Compd. 44 |
| [19CD]-isoGlu-RC()NTATC()ATQRLAEFLHHSNNF-Gly(Me)-A-Ile(Me)-LSSTNVGSNTP-NH2 | Compd. 45 |
| [19CD]-isoGlu-RC()NTATC()ATQRLAEFLHHSNNF-Gly(Me)-Ile(Me)-LSSTNVGSNTP-NH2 | Compd. 46 |
| [19CD]-isoGlu-RC()NTATC()ATQRLAEFLHHNNF-Gly(Me)-A-Ile(Me)-LSSTNVGSNTP-NH2 | Compd. 47 |
| [19CD]-isoGlu-RC()NTATC()ATQRLAEFLHHNNF-Gly(Me)-Ile(Me)-LSSTNVGSNTP-NH2 | Compd. 48 |
| [19CD]-isoGlu-RC()NTATC()ATQRLAEFLHHSSNNFA-Ile(Me)-LSSTNVGSNTP-NH2 | Compd. 49 |
| [19CD]-isoGlu-RC()NTATC()ATQRLAEFLHHSSNN-Gly(Me)-A-Ile(Me)-LSSTNVGSNTP-NH2 | Compd. 50 |
| [19CD]-isoGlu-RC()NTATC()ATQRLAEFLHHSSNN-Gly(Me)-Ile(Me)-LSSTNVGSNTP-NH2 | Compd. 51 |
| [19CD]-isoGlu-RC()NTATC()ATQRLAEFLHHSSF-Gly(Me)-A-Ile(Me)-LSSTNVGSNTP-NH2 | Compd. 52 |
| [19CD]-isoGlu-RC()NTATC()ATQRLAEFLHHSSF-Gly(Me)-Ile(Me)-LSSTNVGSNTP-NH2 | Compd. 53 |
| [19CD]-isoGlu-RC()NTATC()ATQRLAEFLHHSSNNF-Gly(Me)-A-Ile(Me)-SSTNVGSNTP-NH2 | Compd. 54 |
| [19CD]-isoGlu-RC()NTATC()ATQRLAEFLHHSSNNF-Gly(Me)-Ile(Me)-SSTNVGSNTP-NH2 | Compd. 55 |
| [19CD]-isoGlu-RC()NTATC()ATQRLAEFLHHSSNNF-Ala(Me)-A-Ile(Me)-LSSTNVGSNTP-NH2 | Compd. 56 |
| [19CD]-isoGlu-KC()NTATC()ATQRLAEFLHRSSNNF-Gly(Me)-A-Ile(Me)-LSSTNVGSNTP-NH2 | Compd. 57 |
| [19CD]-isoGlu-KC()NTATC()ATQRLAEFLHRSSNNF-Gly(Me)-Ile(Me)-LSSTNVGSNTP-NH2 | Compd. 58 |

(continued)

| | |
|---|---|
| [19CD]-isoGlu-RC()NTATC()ATQRLAEFLHHSSNN-DPhe-Gly(Me)-A-Ile(Me)-L-DSer-STNVGSNTP-NH2 | Compd. 59 |
| [19CD]-isoGlu-RC()NTATC()ATQRLAEFLHHSSNN-DPhe-Gly(Me)-Ile(Me)-L-DSer-STNVGSNTP-NH2 | Compd. 60 |
| [19CD]-isoGlu-RC()NTATC()ATQRLAEFLHHSSNNF-Gly(Me)-A-Ile(Me)-LSSTNVGSNT-Tyr (Me)-NH2 | Compd. 61 |
| [19CD]-isoGlu-RC()NTATC()ATQRLAEFLHHSSNNF-Gly(Me)-Ile(Me)-LSSTNVGSNT-Tyr (Me)-NH2 | Compd. 62 |
| [19CD]-isoGlu-Dab-C()NTATC()ATQRLAEFLHHSSNNF-Gly(Me)-A-Ile(Me)-LSSTNVGSNTP-NH2 | Compd. 63 |
| [19CD]-isoGlu-Dab-C()NTATC()ATQRLAEFLHHSSNNF-Gly(Me)-Ile(Me)-LSSTNVGSNTP-NH2 | Compd. 64 |
| [19CD]-isoGlu-Orn-C()NTATC()ATQRLAEFLHHSSNNF-Gly(Me)-A-Ile(Me)-LSSTNVGSNTP-NH2 | Compd. 65 |
| [19CD]-isoGlu-Orn-C()NTATC()ATQRLAEFLHHSSNNF-Gly(Me)-Ile(Me)-LSSTNVGSNTP-NH2 | Compd. 66 |
| [19CD]-isoGlu-Dap-C()NTATC()ATQRLAEFLHHSSNNF-Gly(Me)-A-Ile(Me)-LSSTNVGSNTP-NH2 | Compd. 67 |
| [19CD]-isoGlu-KC()NTATC()ATQRLAEFLHRSSNNF-Gly(Me)-A-Ile(Me)-L-DSer-STNVGSNTP-NH2 | Compd. 68 |
| [19CD]-isoGlu-Dap-C()NTATC()ATQRLAEFLHHSSNNF-Gly(Me)-Ile(Me)-LSSTNVGSNTP-NH2 | Compd. 69 |
| [19CD]-isoGlu-KC()NTATC()ATQRLAEFLHRSSNNF-Gly(Me)-Ile(Me)-L-DSer-STNVGSNTP-NH2 | Compd. 70 |
| [19CD]-isoGlu-KC()NTATC()ATQRLAEFLHRSSNN-DPhe-Gly(Me)-A-Ile(Me)-LSSTNVGSNTP-NH2 | Compd. 71 |
| [19CD]-isoGlu-KC()NTATC()ATQRLAEFLHRSSNN-DPhe-Gly(Me)-A-Ile(Me)-L-DSer-STNVGSNTP | Compd. 72 |
| [19CD]-isoGlu-RC()NTATC()ATQRLAEFLHRSSSSF-Gly(Me)-A-Ile(Me)-LSSTDVGSDTP-NH2 | Compd. 73 |
| [19CD]-isoGlu-RC()NTATC()ATQRLAEFLHRSSSSF-Gly(Me)-A-Ile(Me)-LSSTDVGSETP-NH2 | Compd. 74 |
| [19CD]-isoGlu-RC()NTATC()ATQRLAEFLHRSSSSF-Gly(Me)-Ile(Me)-LSSTDVGSETP-NH2 | Compd. 75 |
| [19CD]-isoGlu-RC()NTATC()ATQRLAEFLHRSSSSF-Gly(Me)-A-Ile(Me)-LSSTEVGSETP-NH2 | Compd. 76 |
| [19CD]-isoGlu-RC()NTATC()ATQRLAEFLHRSSSSF-Gly(Me)-Ile(Me)-LSSTEVGSETP-NH2 | Compd. 77 |
| [19CD]-isoGlu-RC()NTATC()ATQRLAEFLHRSSF-Gly(Me)-A-Ile(Me)-LSSTEVGSDTP-NH2 | Compd. 78 |
| [19CD]-isoGlu-RC()NTATC()ATQRLAEFLHRSSF-Gly(Me)-Ile(Me)-LSSTEVGSDTP-NH2 | Compd. 79 |
| [19CD]-isoGlu-RC()NTATC()ATQRLAEFLHRSSF-Gly(Me)-A-Ile(Me)-LSSTDVGSETP-NH2 | Compd. 80 |
| [19CD]-isoGlu-RC()NTATC()ATQRLAEFLHRSSF-Gly(Me)-A-Ile(Me)-LSSTDVGSDTP-NH2 | Compd. 81 |
| [19CD]-isoGlu-RC()NTATC()ATQRLAEFLHRSSF-Gly(Me)-A-Ile(Me)-LSSTEVGSETP-NH2 | Compd. 82 |
| [19CD]-isoGlu-RRC()NTATC()ATQRLAEFLHRSSNNF-Gly(Me)-A-Ile(Me)-LSSTNVGSNTP-NH2 | Compd. 83 |
| [19CD]-isoGlu-KRC()NTATC()ATQRLAEFLHRSSNNF-Gly(Me)-A-Ile(Me)-LSSTNVGSNTP-NH2 | Compd. 84 |
| [19CD]-isoGlu-HRC()NTATC()ATQRLAEFLHRSSNNF-Gly(Me)-A-Ile(Me)-LSSTNVGSNTP-NH2 | Compd. 85 |
| [19CD]-isoGlu-RKC()NTATC()ATQRLAEFLHRSSNNF-Gly(Me)-A-Ile(Me)-LSSTNVGSNTP-NH2 | Compd. 86 |
| [19CD]-isoGlu-KKC()NTATC()ATQRLAEFLHRSSNNF-Gly(Me)-A-Ile(Me)-LSSTNVGSNTP-NH2 | Compd. 87 |
| [19CD]-isoGlu-HKC()NTATC()ATQRLAEFLHRSSNNF-Gly(Me)-A-Ile(Me)-LSSTNVGSNTP-NH2 | Compd. 88 |
| [17CD]-isoGlu-RC()NTATC()ATQRLAEFLHRSSNNF-Gly(Me)-A-Ile(Me)-LSSTNVGSNTP-NH2 | Compd. 89 |
| [15CD]-isoGlu-RC()NTATC()ATQRLAEFLHRSSNNF-Gly(Me)-A-Ile(Me)-LSSTNVGSNTP-NH2 | Compd. 90 |
| [Tetradecanoyl]-isoGlu-RC()NTATC()ATQRLAEFLHRSSNNF-Gly(Me)-A-Ile(Me)-LSSTNVGSNTP-NH2 | Compd. 91 |
| [Hexadecanoyl]-isoGlu-RC()NTATC()ATQRLAEFLHRSSNNF-Gly(Me)-A-Ile(Me)-LSSTNVGSNTP-NH2 | Compd. 92 |
| [Octadecanoyl]-isoGlu-RC()NTATC()ATQRLAEFLHRSSNNF-Gly(Me)-A-Ile(Me)-LSSTNVGSNTP-NH2 | Compd. 93 |
| [Eicosanoyl]-isoGlu-RC()NTATC()ATQRLAEFLHRSSNNF-Gly(Me)-A-Ile(Me)-LSSTNVGSNTP-NH2 | Compd. 94 |
| [19CD]-isoGlu-RC()NTATC()ATQRLAEFLHRSSF-Gly(Me)-Ile(Me)-LSSTEVGSETP-NH2 | Compd. 95 |
| [19CD]-isoGlu-RC()NTATC()ATQRLAEFLHRSSNNF-Gly(Me)-A-Ile(Me)-LSSTNVGSNTG-NH2 | Compd. 96 |
| [19CD]-isoGlu-RC()NTATC()ATQRLAEFLHRSSNNF-Gly(Me)-A-Ile(Me)-LSSTNVGSNTA-NH2 | Compd. 97 |
| [19CD]-isoGlu-RC()NTATC()ATQRLAEFLHRSSNNF-Gly(Me)-A-Ile(Me)-LSSTNVGSNTV-NH2 | Compd. 98 |
| [19CD]-isoGlu-RC()NTATC()ATQRLAEFLHRSSNNF-Gly(Me)-A-Ile(Me)-LSSTNVGSNTI-NH2 | Compd. 99 |

(continued)

| | |
|---|---|
| [19CD]-isoGlu-RC()NTATC()ATQRLAEFLHRSSNNF-Gly(Me)-A-Ile(Me)-LSSTNVGSNTL-NH2 | Compd. 100 |
| [19CD]-isoGlu-RC()NTATC()ATQRLAEFLHRSSNNF-Gly(Me)-A-Ile(Me)-LSSTNVGSNT-Gly (Me)-NH2 | Compd. 101 |
| [19CD]-isoGlu-RC()NTATC()ATQRLAEFLHRSSNNF-Gly(Me)-A-Ile(Me)-LSSTNVGSNT-Ala (Me)-NH2 | Compd. 102 |
| [19CD]-isoGlu-RC()NTATC()ATQRLAEFLHRSSNNF-Gly(Me)-A-Ile(Me)-LSSTNVGSNT-Ile (Me)-NH2H2 | Compd. 103 |
| [19CD]-isoGlu-RC()NTATC()ATQRLAEFLHRSSNNF-Gly(Me)-A-Ile(Me)-LSSTNVGSNT-DPro-NH2 | Compd. 104 |
| [19CD]-isoGlu-RC()NTATC()ATQRLAEFLHRSSNNF-Gly(Me)-A-Ile(Me)-LSSTNVGSNT-Hyp-NH2 | Compd. 105 |
| [19CD]-isoGlu-RC()NTATC()ATQRLAEFLHRSSNNF-Gly(Me)-Ile(Me)-LSSTNVGSNT-Hyp-NH2 | Compd. 106 |
| [19CD]-isoGlu-RC()NTATC()ATQRLAEFLHRSSNNF-Gly(Me)-A-Ile(Me)-LSSTNVGSNT-Aze-NH2 | Compd. 107 |
| [19CD]-isoGlu-RC()NTATC()ATQRLAEFLHRSSNNF-Gly(Me)-Ile(Me)-LSSTNVGSNT-Aze-NH2 | Compd. 108 |
| [19CD]-isoGlu-RC()NTATC()ATQRLAEFLHRSSNNF-Gly(Me)-A-Ile(Me)-LSSTNVGSNT-Pip-NH2 | Compd. 109 |
| [19CD]-isoGlu-RC()NTATC()ATQRLAEFLHRSSNNF-Gly(Me)-A-Ile(Me)-LSSTNVGSNT-Apr-NH2 | Compd. 110 |
| [19CD]-isoGlu-RC()NTATC()ATQRLAEFLHRSSNNF-Gly(Me)-Ile(Me)-LSSTNVGSNT-Apr-NH2 | Compd. 111 |
| [19CD]-isoGlu-RC()NTATC()ATQRLAEFLHRSSNNF-Gly(Me)-A-Ile(Me)-LSSTNVGSNT-Php-NH2 | Compd. 112 |
| [19CD]-isoGlu-RC()NTATC()ATQRLAEFLHRSSNNF-Gly(Me)-Ile(Me)-LSSTNVGSNT-Php-NH2 | Compd. 113 |
| [19CD]-isoGlu-RC()NTATC()ATQRLAEFLHRSSNNF-Gly(Me)-A-Ile(Me)-LSSTNVGSNT-Bep-NH2 | Compd. 114 |
| [19CD]-isoGlu-RC()NTATC()ATQRLAEFLHRSSNNF-Gly(Me)-A-Ile(Me)-LSSTNVGSNTF-NH2 | Compd. 115 |
| [19CD]-isoGlu-RC()NTATC()ATQRLAEFLHRSSNNF-Gly(Me)-A-Ile(Me)-LSSTNVGSNTS-NH2 | Compd. 116 |
| [19CD]-isoGlu-RC()NTATC()ATQRLAEFLHRSSNNF-Gly(Me)-A-Ile(Me)-LSSTNVGSNTT-NH2 | Compd. 117 |
| [19CD]-isoGlu-RC()NTATC()ATARLAEFLHRSSSSF-Gly(Me)-A-Ile(Me)-LSSTEVGSETP-NH2 | Compd. 118 |
| [19CD]-isoGlu-RC()NTATC()ATERLAEFLHRSSSSF-Gly(Me)-A-Ile(Me)-LSSTEVGSETP-NH2 | Compd. 119 |
| [19CD]-isoGlu-RC()NTATC()ATDRLAEFLHRSSSSF-Gly(Me)-A-Ile(Me)-LSSTEVGSETP-NH2 | Compd. 120 |
| [19CD]-isoGlu-RC()NTATC()ATLRLAEFLHRSSSSF-Gly(Me)-A-Ile(Me)-LSSTEVGSETP-NH2 | Compd. 121 |
| [19CD]-isoGlu-RC()NTATC()ATGRLAEFLHRSSSSF-Gly(Me)-A-Ile(Me)-LSSTEVGSETP-NH2 | Compd. 122 |
| [19CD]-isoGlu-RC()NTATC()ATTRLAEFLHRSSSSF-Gly(Me)-A-Ile(Me)-LSSTEVGSETP-NH2 | Compd. 123 |
| [19CD]-isoGlu-RC()NTATC()ATSRLAEFLHRSSSSF-Gly(Me)-A-Ile(Me)-LSSTEVGSETP-NH2 | Compd. 124 |
| [19CD]-isoGlu-WC()NTATC()ATQRLAEFLHRSSNNF-Gly(Me)-A-Ile(Me)-LSSTNVGSNTP-NH2 | Compd. 125 |
| [19CD]-isoGlu-RC()STATC()ATQRLAEFLHRSSSSF-Gly(Me)-A-Ile(Me)-LSSTEVGSETP-NH2 | Compd. 126 |
| [19CD]-isoGlu-RC()DTATC()ATQRLAEFLHRSSSSF-Gly(Me)-A-Ile(Me)-LSSTEVGSETP-NH2 | Compd. 127 |
| [19CD]-isoGlu-RC()ETATC()ATQRLAEFLHRSSSSF-Gly(Me)-A-Ile(Me)-LSSTEVGSETP-NH2 | Compd. 128 |
| [19CD]-isoGlu-RC()PTATC()ATQRLAEFLHRSSSSF-Gly(Me)-A-Ile(Me)-LSSTEVGSETP-NH2 | Compd. 129 |
| [19CD]-isoGlu-RC()GTATC()ATQRLAEFLHRSSSSF-Gly(Me)-A-Ile(Me)-LSSTEVGSETP-NH2 | Compd. 130 |
| [19CD]-isoGlu-RC()GTATC()ATGRLAEFLHRSSSSF-Gly(Me)-A-Ile(Me)-LSSTEVGSETP-NH2 | Compd. 131 |
| [19CD]-isoGlu-RC()STATC()ATGRLAEFLHRSSSSF-Gly(Me)-A-Ile(Me)-LSSTEVGSETP-NH2 | Compd. 132 |
| [19CD]-isoGlu-RC()STATC()ATSRLAEFLHRSSSSF-Gly(Me)-A-Ile(Me)-LSSTEVGSETP-NH2 | Compd. 133 |
| [19CD]-isoGlu-RC()ETATC()ATERLAEFLHRSSSSF-Gly(Me)-A-Ile(Me)-LSSTEVGSETP-NH2 | Compd. 134 |
| [19CD]-isoGlu-RC()STATC()ATERLAEFLHRSSSSF-Gly(Me)-A-Ile(Me)-LSSTEVGSETP-NH2 | Compd. 135 |
| [19CD]-isoGlu-RC()PTATC()ATERLAEFLHRSSSSF-Gly(Me)-A-Ile(Me)-LSSTEVGSETP-NH2 | Compd. 136 |
| [19CD]-isoGlu-RC()DTATC()ATERLAEFLHRSSSSF-Gly(Me)-A-Ile(Me)-LSSTEVGSETP-NH2 | Compd. 137 |
| [19CD]-isoGlu-RC()DTATC()ATSRLAEFLHRSSSSF-Gly(Me)-A-Ile(Me)-LSSTEVGSETP-NH2 | Compd. 138 |
| [19CD]-isoGlu-RC()ETATC()ATSRLAEFLHRSSSSF-Gly(Me)-A-Ile(Me)-LSSTEVGSETP-NH2 | Compd. 139 |
| [19CD]-isoGlu-RC()PTATC()ATSRLAEFLHRSSSSF-Gly(Me)-A-Ile(Me)-LSSTEVGSETP-NH2 | Compd. 140 |
| [19CD]-isoGlu-RC()GTATC()ATSRLAEFLHRSSSSF-Gly(Me)-A-Ile(Me)-LSSTEVGSETP-NH2 | Compd. 141 |
| [19CD]-isoGlu-RC()NTATC()ATPRLAEFLHRSSSSF-Gly(Me)-A-Ile(Me)-LSSTEVGSETP-NH2 | Compd. 142 |
| [19CD]-isoGlu-RC()PTATC()ATPRLAEFLHRSSSSF-Gly(Me)-A-Ile(Me)-LSSTEVGSETP-NH2 | Compd. 143 |
| [19CD]-isoGlu-RC()STATC()ATARLAEFLHRSSSSF-Gly(Me)-A-Ile(Me)-LSSTEVGSETP-NH2 | Compd. 144 |
| [19CD]-isoGlu-RC()DTATC()ATARLAEFLHRSSSSF-Gly(Me)-A-Ile(Me)-LSSTEVGSETP-NH2 | Compd. 145 |
| [19CD]-isoGlu-RC()ETATC()ATARLAEFLHRSSSSF-Gly(Me)-A-Ile(Me)-LSSTEVGSETP-NH2 | Compd. 146 |

(continued)

| | |
|---|---|
| [19CD]-isoGlu-RC()PTATC()ATARLAEFLHRSSSSF-Gly(Me)-A-Ile(Me)-LSSTEVGSETP-NH2 | Compd. 147 |
| [19CD]-isoGlu-RC()STATC()ATDRLAEFLHRSSSSF-Gly(Me)-A-Ile(Me)-LSSTEVGSETP-NH2 | Compd. 148 |
| [19CD]-isoGlu-RC()DTATC()ATDRLAEFLHRSSSSF-Gly(Me)-A-Ile(Me)-LSSTEVGSETP-NH2 | Compd. 149 |
| [19CD]-isoGlu-RC()ETATC()ATDRLAEFLHRSSSSF-Gly(Me)-A-Ile(Me)-LSSTEVGSETP-NH2 | Compd. 150 |
| [19CD]-isoGlu-RC()PTATC()ATDRLAEFLHRSSSSF-Gly(Me)-A-Ile(Me)-LSSTEVGSETP-NH2 | Compd. 151 |
| [19CD]-isoGlu-RC()NTATC()ATQRLAEFLHRSSNNF-Gly(Me)-A-Ile(Me)-LSSTNVGSNTP-NHMe | Compd. 152 |
| [19CD]-isoGlu-RC()NTATC()ATQRLAEFLHRSSNNF-Gly(Me)-Ile(Me)-LSSTNVGSNTP-NHMe | Compd. 153 |
| [19CD]-isoGlu-RC()NTATC()ATQRLAEFLHRSSNNF-Gly(Me)-A-Ile(Me)-LSSTNVGSNTY-NHMe | Compd. 154 |
| [19CD]-isoGlu-RC()NTATC()ATQRLAEFLHRSSNNF-Gly(Me)-Ile(Me)-LSSTNVGSNTY-NHMe | Compd. 155 |
| [19CD]-isoGlu-RC()NTATC()ATQRLAEFLHRSSNNF-Gly(Me)-A-Ile(Me)-LSSTNVGSNTY-NH2 | Compd. 156 |
| [19CD]-isoGlu-WC()GTATC()ATDRLAEFLHRSSF-Gly(Me)-A-Ile(Me)-LSSTDVGSDTP-NH2 | Compd. 157 |
| [19CD]-isoGlu-RC()GTATC()ATDRLAEFLHRSSF-Gly(Me)-A-Ile(Me)-LSSTDVGSDTP-NH2 | Compd. 158 |
| [19CD]-isoGlu-KKC()NTATC()ATERLAEFLHRSSSSF-Gly(Me)-A-Ile(Me)-LSSTDVGSDTP-NH2 | Compd. 159 |
| [19CD]-isoGlu-RC()NTATC()ATERLAEFLHRSSSSF-Gly(Me)-A-Ile(Me)-LSSTDVGSDTP-NH2 | Compd. 160 |
| [19CD]-isoGlu-WC()GTATC()ATERLAEFLHRSSF-Gly(Me)-A-Ile(Me)-LSSTEVGSETP-NH2 | Compd. 161 |
| [19CD]-isoGlu-RC()GTATC()ATERLAEFLHRSSF-Gly(Me)-A-Ile(Me)-LSSTEVGSETP-NH2 | Compd. 162 |
| [19CD]-isoGlu-KKC()ETATC()ATERLAEFLHRSSSSF-Gly(Me)-A-Ile(Me)-LSSTEVGSETP-NH2 | Compd. 163 |
| [19CD]-isoGlu-WC()GTATC()ATERLAEFLHRSSF-Gly(Me)-A-Ile(Me)-LSSTEVGSET-Hyp-NH2 | Compd. 164 |
| [19CD]-isoGlu-WC()GTATC()ATERLAEFLHRSSF-Gly(Me)-Ile(Me)-LSSTEVGSET-Hyp-NH2 | Compd. 165 |
| [19CD]-isoGlu-RC()GTATC()ATERLAEFLHRSSF-Gly(Me)-A-Ile(Me)-LSSTEVGSET-Hyp-NH2 | Compd. 166 |
| [19CD]-isoGlu-RC()GTATC()ATERLAEFLHRSSF-Gly(Me)-Ile(Me)-LSSTEVGSET-Hyp-NH2 | Compd. 167 |
| [19CD]-isoGlu-KKC()ETATC()ATERLAEFLHRSSSSF-Gly(Me)-A-Ile(Me)-LSSTEVGSET-Hyp-NH2 | Compd. 168 |
| [19CD]-isoGlu-KKC()ETATC()ATERLAEFLHRSSSSF-Gly(Me)-Ile(Me)-LSSTEVGSET-Hyp-NH2 | Compd. 169 |
| [19CD]-isoGlu-RC()ETATC()ATERLAEFLHRSSSSF-Gly(Me)-A-Ile(Me)-LSSTEVGSET-Hyp-NH2 | Compd. 170 |
| [19CD]-isoGlu-RC()ETATC()ATERLAEFLHRSSSSF-Gly(Me)-Ile(Me)-LSSTEVGSET-Hyp-NH2 | Compd. 171 |
| [19CD]-isoGlu-RC()PTATC()ATDRLAEFLHRSSF-Gly(Me)-A-Ile(Me)-LSSTDVGSDTP-NH2 | Compd. 172 |
| [19CD]-isoGlu-RC()PTATC()ATERLAEFLHRSSSSF-Gly(Me)-A-Ile(Me)-LSSTDVGSDTP-NH2 | Compd. 173 |
| [19CD]-isoGlu-RWC()NTATC()ATQRLAEFLHRSSNNF-Gly(Me)-A-Ile(Me)-LSSTNVGSNTP-NH2 | Compd. 174 |
| [19CD]-isoGlu-RC()NTATC()ATQRLAEFLHHSSNNF-Gly(Me)-P-Ile(Me)-LPPTNVGSNTP-$NH_2$ | Compd. 175 |
| [19CD]-isoGlu-RC()NTATC()ATQRLAEFLHHSSNNF-Gly(Me)-Ile(Me)-LPPTNVGSNTP-$NH_2$ | Compd. 176 |

[19CD] represents [19-carboxynonadecanoyl],
[17CD] represents [17-carboxyheptadecanoyl, and
[15CD] represents [15-carboxypentadecanoyl];

Example 1: Synthesis of compounds

Unless otherwise specified, reagents and solvents employed in the following were available commercially in standard laboratory reagent or analytical grade, and were used without further purification.

General procedure for solid-phase synthesis of peptides

A CEM Liberty Peptide Synthesizer was employed, using standard Fmoc chemistry. TentaGel™ S Ram resin (1 g; 0.25 mmol/g) was swelled in DMF (10 ml) prior to use and transferred between tube and reaction vessel using DCM and DMF. Pseudoprolines [i.e. dipeptides employed to minimize aggregation during peptide synthesis, such as Fmoc-Phe-Thr(ψ-Me,Me-Pro)-OH and Fmoc-Asp-Ser(ψ-Me,Me-Pro)-OH and Fmoc-Ser-Ser(ψ-Me,Me-Pro)-OH] were used where appropriate, and non-naturally occurring amino acids and other suitable building blocks were employed without any changes to the general procedure.

The following optical isomers of non-naturally occurring amino acids were employed in the synthesis of the compounds:

Apr: (2S,4R)-4-aminoproline [also denoted (4R)-4-amino-L-proline];
Aze: (2S)-azetidine-2-carboxylic acid;

Bep: (2S,4R)-4-benzylproline [also denoted (4R)-4-benzyl-L-proline];
Hyp: (2S,4R)-4-hydroxyproline [also denoted (4R)-4-hydroxy-L-proline];
Php: (2S,4S)-4-phenylproline [also denoted (4S)-4-phenyl-L-proline];
Pip: (2S)-piperidine-2-carboxylic acid [also denoted (S)-pipecolic acid].

*Coupling:*

An Fmoc-amino acid in DMF/DCM (2:1; 0.2 M; 5 ml) was added to the resin in a CEM Discover microwave unit together with COMU/DMF (0.5 M; 2 ml) and DIPEA/DMF (2.0 M; 1 ml). The coupling mixture was heated to 75°C for 5 min. while nitrogen was bubbled through the mixture. The resin was then washed with DMF (4 x 10 ml). Alternatively the coupling was done without heating and the reaction time extended to 60 min in this case.

In the case of difficult couplings (e.g. coupling of a residue immediately after an N-methylated amino acid residue or other sterically hindered amino acid residue as recognized by a person of skill in the art) the coupling was repeated one or more times.

*Deprotection:*

Piperidine/DMF (1:4, i.e. 1 part piperidine to 4 parts DMF by volume; 10 ml) was added to the resin for initial deprotection, and the mixture was microwave-heated (40°C; 30 sec.). The reaction vessel was drained and a second portion of piperidine/DMF (1:4; 10 ml) was added and heated (75°C; 3 min) again. The resin was then washed with DMF (6 x 10 ml).

*Oxidative cyclization*

Intramolecular ring formation (disulfide bridge formation) between the Cys residues in positions 2 and 7 (initially coupled in the form of Acm-protected cysteines) was performed with the peptide still attached to the resin, using 163 mg thallium(III) trifluoroacetate [Tl(TFA)3] in 5 mL NMP in a simultaneous Acm-deprotection and disulfide-formation step.

*Cleavage:*

The resin was washed with EtOH (3 x 10 ml) and $Et_2O$ (3 x 10 ml) and dried to constant weight at room temperature (r.t.). The crude peptide was cleaved from the resin by treatment with TFA/TIS/$H_2O$ (90:5:5; 40 ml; 2 h; room temp.) or alternatively with TFA/DODT (95:5; 40 ml; 2h; room temp.). Most of the TFA was removed under reduced pressure, and the crude peptide was precipitated and washed three times with $Et_2O$ and dried to constant weight at room temperature.

*Purification and characterisation:*

The crude peptide was purified by preparative reverse phase HPLC using a PerSeptive Biosystems VISION Workstation or a Gilson system (Pumps: "Pump 305", "331 Pump", "332 Pump", "402 Syringe Pump"; column changer "Valvemate® II"UV detector "UV/Vis-155"; and the fraction collector "GX 281" equipped with a suitable column and a fraction collector, and run with a gradient of buffer A (0.1% aqueous TFA) and buffer B (0.1% TFA, 90% MeCN, water). Fractions were analysed by analytical HPLC and MS, and relevant fractions were pooled and lyophilised. The final product was characterized by HPLC and MS.

In many cases it was possible to isolate a peptide with a deletion of the amino acid in position 25 in addition to the full length peptide.

One of skill in the art will appreciate that standard methods of peptide synthesis may be used to generate the compounds of the invention.

Synthesis of Compound 5

Compound 5 (SEQ ID NO: 7) was synthesized on a CEM Liberty Peptide Synthesizer using Tentagel S PHB-Thr(tBu) resin (1.23 g, 0.24 mmol/g), COMU as coupling reagent, DMF as the solvent, and Fmoc-chemistry as described above. Pseudoprolines Fmoc-Ala-Thr(Ψ Me, Me-Pro)-OH (for position 8/9), Fmoc-Ser-Ser(Ψ, Me, Me Pro)-OH (for position 19/20), and Fmoc-Leu-Ser(Ψ, Me, Me-Pro)-OH (for position 27/28), were used in the sequence. Additional unusual building blocks were Fmoc-Gly(Me)-OH (for position 24), Fmoc-Ile(Me)-OH (for position 25), Eicosanedioic acid, 1-(1,1-dimethyl)ethyl ester (for moiety M), Fmoc-L-glutamic acid α-tert-butyl ester (CAS No. 84793-07) (for moiety L), and Fmoc-Cys(Acm)-OH (for positions 2 and 7). Eicosanedioic acid, 1-(1,1-dimethyl)ethyl ester, Fmoc-L-glutamic acid

α -tert-butyl ester, Fmoc-Cys(Acm)-OH and Fmoc-His(Trt)-OH were coupled without heat, and double couplings were employed after Fmoc-Gly(Me)-OH and Fmoc-Ile(Me)-OH, respectively, had been attached to the peptide chain.

Intramolecular ring formation (cyclization/disulfide bridge formation) was performed as described above, and the peptide was then cleaved from the resin as described above using TFA/DODT. Purification was performed on a Gemini-NX column (5 cm, C18, 10 micron) with a 35 ml/min flow of a mixture of buffer A and buffer B (*vide supra*). The product was eluted with a linear gradient of 35-60% buffer B over 47 min., and relevant fractions were analyzed by analytical HPLC and MS. The pooled fractions were lyophilized to give 23.71 mg of product. The purity was 90% as determined by analytical HPLC (*vide supra*), and the monoisotopic mass was 4458.20 Da as determined by MS (calc. 4458.2101 Da). Compound 6 (SEQ ID NO: 8) was also isolated in addition to the full length peptide Compound 5. The pooled, eluted fractions containing Compound 6 were lyophilized to give 15.87 mg of product. The purity was 80% as determined by analytical HPLC (*vide supra*), and the monoisotopic mass was 4387.16 Da as determined by MS (calc. 4387.1729 Da).

Additional protocol for synthesis of Compound 43

Pyrrazolecarboxamidine•HCl (3 mg; 10 equiv.) was dissolved in NMP (22 μl) in a 1.5 mL Eppendorf tube, and DIPEA (8 μl; 20 equiv.) was added. Addition of this solution to Compound 19 (10 mg) led to formation of a solution with two phases (lower phase clear, upper phase cloudy). After adding NMP (70 μl) the solution became clear and monophasic, and the reaction was then continued for 16 h with occasional checks for reaction completion.

After no more starting material could be detected via ESI-MS the solution was transferred to a 50 ml Falcon tube and the volume adjusted to 10 ml using MilliQ™ water. The product was isolated by RP-HPLC purification, yielding 3.55 mg of Compound 43 (purity 90%).

Example 2

Generation of cell lines expressing (i) human calcitonin receptor (hCTR2), and (ii) human amylin receptor 3 (hAMYR3) consisting of hCTR2 co-expressed with human Receptor Activity Modifying Protein 3 (hRAMP3)

The cell line COS7, originating from African Green Monkey kidney fibroblast cells, was used for establishment of the stable cell lines described below. The COS7 cellular background was used due to it being RAMP naive, and as such it provides the best available system for testing of the monomeric hCTR and different heterodimeric amylin receptor subtypes functionally generated by introduction of the individual RAMPs.

Calcitonin receptor cell line:

A cell line expressing the human calcitonin receptor (hCTR2) was generated in the COS7 cellular background as stable clones. In brief, hCTR2 (GI: 4502547) was amplified by PCR using primers encoding terminal restriction sites for subcloning. The 5'-end primers additionally encoded a near Kozak consensus sequence to ensure efficient translation. The fidelity of the DNA encoding the receptor was confirmed by DNA sequencing. The PCR products encoding the receptor were subcloned into a mammalian expression vector containing a neomycin (G418) resistance marker. The mammalian expression vector encoding the receptor was transfected into COS7 cells by a standard liposome transfection method. 48 hours post-transfection, cells were seeded for limited dilution cloning and selected with 1 mg/ml G418 in the culture medium. After 3 weeks, surviving colonies of hCTR2-expressing cells were picked, propagated and tested in the amylin efficacy assay as described in Example 3 below. One hCTR2-expressing clone was chosen for compound profiling.

Amylin receptor cell line:

A cell line expressing the human amylin receptor 3 (hAMYR3) was generated in the COS7 cellular background as stable clones. In brief, hCTR2 (GI:4502547) and hRAMP3 (GI:118572586) were amplified by PCR using primers encoding terminal restriction sites for subcloning. The 5'-end primers additionally encoded a near Kozak consensus sequence to ensure efficient translation. The fidelity of the DNA encoding the receptor was confirmed by DNA sequencing. The PCR products encoding the receptor were subcloned into a proprietary tri-cistronic mammalian expression vector with RAMP3, hCTR2 and neomycin (G418) resistance marker. The mammalian expression vector encoding the receptor was transfected into COS7 cells by a standard liposome transfection method. 48 hours post-transfection, cells were seeded for limited dilution cloning and selected with 1 mg/ml G418 in the culture medium. After 3 weeks, surviving colonies of hAMY3R-expressing cells were picked, propagated and tested in the amylin efficacy assay as described in Example 3 below. The functional generation of the amylin receptor phenotype was verified in the efficacy assay by a left-shifted efficacy response to amylin relative to monomeric calcitonin receptor, and one hAMYR3-expressing clone

was chosen for compound profiling.

Example 3

hCTR2 and hAMYR3 assays

*In vitro* activity of test peptides at the hCTR2 and hAMYR3 receptors was assessed by measuring the induction of cAMP following stimulation of the receptor by pramlintide or peptides of the invention using the AlphaScreen® cAMP Assay kit from Perkin-Elmer.

Briefly, COS7 cells expressing hCTR2 (see Example 2, above) were seeded at 30-40,000 cells per well in 96-well microtiter plates coated with 0.01 % poly-L-lysine, and grown for 1 day in culture in 100 μl growth medium [DMEM, 10% FCS, Penicillin (100 IU/ml), Streptomycin (100 μg/ml)]. On the day of analysis, growth medium was removed and the cells were washed once with 200 μl Tyrode buffer [Tyrode's Salts (9.6 g/l), 10 mM HEPES, pH 7.4]. Cells were incubated in 100 μl Tyrode buffer containing increasing concentrations of test peptides, 100 μM IBMX and 0.1% casein for 15 min at 37° C. The reaction was stopped by carefully decanting off the compound/buffer medium and replacing it with lysis/detection buffer (80 μl 0.1 % w/v BSA, 5 mM HEPES, 0.3 % v/v Tween-20 in deionized water). After incubation at room temperature for 10 min., the cAMP content of the resulting cell lysate was estimated according to the AlphaScreen® cAMP Assay manufacturer's instructions. $EC_{50}$ values and relative efficacy values (in %) compared to reference peptide (Pramlintide) were estimated by computer-aided curve fitting using the 4-parameter logistic (4PL) non-linear model.

The *in vitro* activity results (expressed as $EC_{50}$ values) for peptides of the invention and for Pramlintide (reference peptide) are summarized in Table 2, below.

Example 4

Solubility assessment

A stock solution of the test peptide (2 mg/ml; determined from the weighed amount of peptide) in demineralized water adjusted to pH 2.5 with HCl was prepared, and aliquots were diluted 1:1 in 100 mM acetate buffer (pH 4.0 and pH 5.0), 100 mM histidine buffer (pH 6.0 and pH 7.0) and 100 mM phosphate buffer (pH 6.0, pH 7.0 and pH7.5), respectively, and loaded in a standard flat-bottom, non-sterile 96-well UV Microplate. The absorbance of samples (single samples, n=1) at 280 and 325 nm was measured in an absorbance-based plate reader, which was preheated to ambient temperature (typically 25°C). The turbidity absorbance criterion for a peptide solubility of ≥ 1 mg/ml was an absorbance at 325 nm of ≤ 0.02 absorbance units (which is 5 to 6 times the standard deviation of 8 buffer samples in a plate). Solubility data for peptides of the invention, pramlintide, and for a comparison pramlintide derivative denoted NN96 (see Example 5 below for details) are shown in Table 1, below.

Table 1. Solubility data for compounds of the invention.

| Compd. No. | Acetate buffer pH 4 | Acetate buffer pH 5 | Histidine buffer pH 6 | Histidine buffer pH 7 | Phosphate buffer pH 6 | Phosphate buffer pH 7 | Phosphate buffer pH 7.5 |
|---|---|---|---|---|---|---|---|
| pramlintide | SS* | SS | SS | SS | SS | SS | SS |
| NN96 | SS | SS | SS | SS | SS | SS | SS |
| 1 | SS | SS | SS | SS | SS | SS | SS |
| 2 | SS | SS | SS | SS | SS | SS | II |
| 3 | SS | SS | SS | II** | SS | II | II |
| 4 | II | SS | II | II | II | II | II |
| 5 | SS | SS | SS | SS | SS | SS | SS |
| 10 | SS | SS | SS | SS | SS | SS | SS |
| 18 | SS | SS | SS | SS | SS | SS | SS |
| 19 | SS | SS | SS | SS | SS | SS | SS |
| 20 | SS | SS | SS | SS | SS | SS | SS |

(continued)

| Compd. No. | Acetate buffer pH 4 | Acetate buffer pH 5 | Histidine buffer pH 6 | Histidine buffer pH 7 | Phosphate buffer pH 6 | Phosphate buffer pH 7 | Phosphate buffer pH 7.5 |
|---|---|---|---|---|---|---|---|
| 21 | SS | SS | SS | SS | SS | SS | SS |
| 22 | SS | SS | SS | SS | SS | SS | SS |
| 24 | SS | SS | SS | SS | SS | SS | SS |
| 25 | SS | SS | SS | SS | SS | SS | SS |
| 26 | SS | SS | SS | SS | SS | SS | SS |
| 28 | SS | SS | SS | SS | SS | SS | SS |
| 29 | SS | SS | SS | SS | SS | SS | SS |
| 32 | SS | SS | SS | SS | SS | SS | SS |
| 33 | SS | SS | SS | SS | SS | SS | SS |
| 36 | SS | SS | SS | SS | SS | SS | SS |
| 37 | SS | SS | SS | SS | SS | SS | SS |
| 38 | II | II | II | II | II | II | II |
| 39 | SS | SS | SS | SS | SS | SS | SS |
| 40 | SS | SS | SS | SS | SS | SS | SS |
| 41 | SS | SS | SS | SS | SS | SS | SS |
| 42 | SS | SS | SS | SS | SS | SS | SS |
| 44 | SS | II | II | SS | SS | SS | SS |
| 45 | II | II | II | II | II | II | II |
| 46 | II | II | II | II | II | II | II |
| 47 | SS | SS | SS | SS | SS | SS | SS |
| 48 | SS | SS | SS | SS | SS | SS | SS |
| 49 | II | II | II | II | II | II | II |
| 50 | SS | SS | SS | SS | SS | SS | SS |
| 51 | II | II | II | II | II | II | II |
| 52 | SS | SS | SS | SS | SS | SS | SS |
| 53 | SS | SS | SS | SS | SS | SS | SS |
| 54 | SS | SS | SS | SS | SS | SS | SS |
| 55 | II | II | II | II | II | II | II |
| 56 | SS | II | II | II | II | II | II |
| 57 | SS | SS | SS | SS | SS | SS | SS |
| 58 | SS | SS | SS | SS | SS | II | SS |
| 59 | SS | SS | SS | SS | SS | II | SS |
| 60 | SS | SS | SS | SS | SS | II | SS |
| 61 | SS | SS | II | II | II | II | II |
| 62 | SS | II | II | II | II | II | II |
| 63 | SS | II | SS | SS | SS | SS | SS |

(continued)

| Compd. No. | Acetate buffer pH 4 | Acetate buffer pH 5 | Histidine buffer pH 6 | Histidine buffer pH 7 | Phosphate buffer pH 6 | Phosphate buffer pH 7 | Phosphate buffer pH 7.5 |
|---|---|---|---|---|---|---|---|
| 64 | SS | II | SS | SS | SS | SS | SS |
| 65 | SS | SS | SS | SS | SS | SS | SS |
| 66 | SS | SS | SS | SS | SS | SS | SS |
| 67 | SS | SS | SS | SS | SS | SS | SS |
| 68 | SS | SS | SS | SS | SS | SS | SS |
| 69 | SS | SS | SS | SS | SS | SS | SS |
| 70 | SS | SS | SS | SS | SS | SS | SS |
| 71 | SS | SS | SS | SS | SS | SS | SS |
| 72 | SS | SS | SS | SS | SS | SS | SS |
| 73 | SS | II | SS | SS | SS | SS | SS |
| 74 | SS | SS | SS | SS | SS | SS | SS |
| 75 | SS | II | II | SS | SS | SS | SS |
| 76 | SS | II | SS | SS | SS | SS | SS |
| 77 | SS | II | II | SS | II | SS | SS |
| 78 | SS | SS | SS | SS | SS | SS | SS |
| 79 | SS | SS | SS | SS | SS | SS | SS |
| 80 | SS | SS | II | SS | SS | SS | SS |
| 81 | SS | SS | SS | SS | SS | SS | SS |
| 82 | SS | SS | SS | SS | SS | SS | SS |
| 83 | SS | SS | SS | SS | SS | SS | SS |
| 84 | SS | SS | SS | SS | SS | SS | SS |
| 85 | SS | SS | SS | SS | SS | SS | SS |
| 86 | SS | SS | SS | SS | SS | SS | SS |
| 87 | SS | SS | SS | SS | SS | SS | SS |
| 88 | SS | SS | SS | SS | SS | SS | SS |
| 89 | SS | SS | SS | SS | SS | SS | SS |
| 90 | SS | SS | SS | SS | SS | SS | SS |
| 91 | SS | SS | SS | SS | SS | SS | SS |
| 92 | SS | SS | SS | SS | SS | SS | SS |
| 93 | SS | SS | SS | SS | SS | SS | SS |
| 94 | SS | SS | SS | SS | SS | SS | SS |
| 95 | SS | SS | SS | SS | SS | SS | SS |
| 96 | SS | SS | SS | SS | SS | SS | SS |
| 97 | SS | SS | SS | SS | SS | SS | SS |
| 98 | SS | SS | SS | SS | II | SS | SS |
| 99 | SS | SS | SS | SS | II | SS | SS |

(continued)

| Compd. No. | Acetate buffer pH 4 | Acetate buffer pH 5 | Histidine buffer pH 6 | Histidine buffer pH 7 | Phosphate buffer pH 6 | Phosphate buffer pH 7 | Phosphate buffer pH 7.5 |
|---|---|---|---|---|---|---|---|
| 100 | SS | SS | SS | SS | II | SS | SS |
| 101 | SS | SS | SS | SS | II | SS | SS |
| 102 | SS | II | SS | SS | II | SS | SS |
| 103 | SS | SS | SS | SS | II | SS | SS |
| 104 | SS | SS | SS | SS | II | SS | SS |
| 105 | SS | SS | SS | SS | SS | SS | SS |
| 107 | SS | SS | SS | SS | SS | SS | SS |
| 108 | SS | SS | SS | SS | SS | SS | SS |
| 109 | SS | SS | SS | SS | II | SS | SS |
| 110 | SS | SS | SS | SS | II | SS | SS |
| 111 | SS | SS | SS | SS | SS | SS | SS |
| 112 | SS | SS | SS | II | II | SS | II |
| 114 | SS | SS | SS | II | II | II | II |
| 115 | SS | SS | SS | II | II | SS | II |
| 119 | SS | SS | SS | SS | SS | SS | SS |
| 120 | SS | SS | SS | SS | SS | SS | SS |
| 121 | SS | SS | II | SS | SS | II | SS |
| 122 | SS | SS | SS | SS | SS | SS | SS |
| 123 | SS | SS | SS | SS | SS | SS | SS |
| 124 | SS | SS | SS | SS | SS | SS | SS |
| 125 | SS | SS | SS | SS | II | SS | SS |
| 126 | SS | SS | SS | SS | SS | SS | SS |
| 127 | SS | SS | SS | SS | SS | SS | SS |
| 129 | SS | SS | SS | SS | SS | SS | SS |
| 130 | SS | SS | SS | SS | SS | SS | SS |
| 131 | SS | SS | SS | SS | SS | SS | SS |
| 132 | SS | SS | SS | SS | SS | SS | SS |
| 133 | SS | SS | SS | SS | SS | SS | SS |
| 134 | SS | SS | SS | SS | SS | SS | SS |
| 135 | SS | SS | SS | SS | SS | SS | SS |
| 136 | SS | SS | SS | SS | SS | SS | SS |
| 137 | SS | SS | SS | SS | SS | SS | SS |
| 138 | SS | SS | SS | SS | SS | SS | SS |
| 139 | SS | SS | SS | SS | SS | SS | SS |
| 141 | SS | SS | SS | SS | SS | SS | SS |
| 143 | SS | SS | SS | SS | SS | SS | SS |

(continued)

| Compd. No. | Acetate buffer pH 4 | Acetate buffer pH 5 | Histidine buffer pH 6 | Histidine buffer pH 7 | Phosphate buffer pH 6 | Phosphate buffer pH 7 | Phosphate buffer pH 7.5 |
|---|---|---|---|---|---|---|---|
| 144 | SS | SS | SS | SS | SS | SS | SS |
| 145 | SS | SS | SS | SS | SS | SS | SS |
| 146 | SS | SS | SS | SS | SS | SS | SS |
| 147 | SS | II | SS | SS | SS | SS | SS |
| 148 | SS | SS | SS | SS | SS | SS | SS |
| 149 | SS | SS | SS | SS | SS | SS | SS |
| 150 | SS | SS | SS | SS | SS | SS | SS |
| 151 | SS | SS | SS | SS | SS | SS | SS |
| 152 | SS | SS | SS | SS | SS | SS | SS |
| 153 | SS | SS | II | SS | SS | SS | SS |
| 154 | SS | SS | SS | II | II | II | II |
| 155 | SS | SS | II | II | II | II | II |
| 156 | SS | SS | SS | II | SS | II | II |
| 157 | II | SS | SS | SS | SS | SS | SS |
| 158 | SS | SS | SS | SS | SS | SS | SS |
| 159 | SS | SS | SS | SS | SS | SS | SS |
| 160 | SS | SS | SS | SS | SS | SS | SS |
| 161 | II | SS | SS | SS | SS | SS | SS |
| 162 | SS | SS | SS | SS | SS | SS | SS |
| 163 | SS | SS | SS | SS | SS | II | SS |
| 165 | SS | SS | SS | SS | SS | II | SS |
| 166 | SS | SS | SS | SS | SS | SS | SS |
| 167 | SS | SS | SS | SS | SS | SS | SS |
| 168 | SS | II | SS | SS | SS | SS | SS |
| 172 | SS | SS | SS | SS | SS | SS | SS |
| 174 | SS | SS | SS | SS | SS | SS | II |
| 175 | SS | SS | SS | SS | SS | SS | II |
| 176 | SS | SS | SS | SS | SS | SS | SS |
| *SS indicates solubility ≥ 1mg/ml<br>**II indicates solubility ≤ 1 mg/ml | | | | | | | |

Example 5

Assessment of physical stability

Aggregation in the form of fibril formation was detected using the amyloid-specific dye Thioflavin T (ThT), which is frequently employed to demonstrate the presence of fibrils in solution (see, e.g., Groenning, M., J. Chem. Biol. 3(1) (2010), pp. 1-18; Groenning et al., J. Struct. Biol. 158 (2007) pp. 358-369; and Levine, H., III, Protein Sci. 2 (1993) pp. 404-410) Test peptides (2 mg/ml) were dissolved in demineralized water adjusted to pH 2.5 with HCl, at ambient temperature (typically 25°C). Solutions containing (i) 1 mg/ml of test peptide, 40 μM ThT and 50 mM phosphate (Ph) buffer

(pH 7.0), (ii) 1 mg/ml of test peptide, 40 μM ThT and 50 mM histidine (His) buffer (pH 7.0), and (iii) 1 mg/ml of test peptide, 40 μM ThT and 50 mM acetate (Ac) buffer (pH 4.0), were loaded in a 96-well black fluorescence plate (clear bottom) in triplicate. Data were collected at fixed intervals of 10 min, each preceded by 300 s of automixing (agitation), over a period of 96 hours at 40° C. Physical stability, expressed as lag-time of fibril formation (in hours), was defined as the intersection between two linear regressions representing the initial stable phase and the growth phase. Table 2 below shows the fibrillation results for tested peptides, for pramlintide, and for a comparison pramlintide derivative disclosed in the literature {N-α-[(S)-4-carboxy-4-(19-carboxynonadecanoylamino)butyryl]-[Arg1,Glu14,His17,Pro37]-pramlintide; disclosed as Example 96 in WO 2012/168430 A2; denoted NN96 herein, and having the amino acid sequence: RC()NTATC()ATQRLAEFLHHSSNNFGPILPPTNVGSNTP (SEQ ID NO: 171)}

Example 6

Assessment of chemical stability

Samples of each test peptide were dissolved in MilliQ™ water, and the pH of the solution was adjusted to pH 4, pH 7.5 or pH 9, respectively, using either HCl or NaOH. The final peptide concentration was 0.2 mg/ml. Samples were placed in glass vials and incubated at 40 °C. The samples were analyzed by RP-HPLC on a C8 column with gradient elution using an ammonium formate/water eluent system, or on a C18 column with gradient elution using a trifluoroacetic acid/water eluent system. The area-percentage (area-%) of the main peak after incubation time T = t (relative to time T = 0) was determined by UV spectroscopy at 220 nm.

The purity was first determined as follows:

$$\text{Purity (area-\%)} = (\text{area of main peak/total area of all peaks}) \times 100.$$

The purity was then normalized between time points by setting purity at time 0 (T=0) to 100 for each pH value for a given peptide, as follows:

$$\text{Normalised area-\% at time t (T=t)} = [\text{area-\% (T=t)/area-\% (T=0)}] \times 100.$$

The chemical stability assessment results (in the form of normalized purity values) are summarized in Table 2 (below). The normalized purity values in Table 2 were determined after 7 days of incubation.

Table 2. $EC_{50}$, chemical stability and fibrillation data for compounds of the invention

| | | | | Chem. stability; 7 days, 40°C | | | Fibrillation lag time (hours) | | |
|---|---|---|---|---|---|---|---|---|---|
| SEQ ID NO. | Compd . No. | hCTR2 $EC_{50}$ (nM) | hAMY R3 $EC_{50}$ (nM) | pH 4 | pH 7.5 | pH 9 | pH 4.0 (Ac) | pH 7.0 (Ph) | pH 7.0 (His) |
| 3 | 1 | 0.160 | 0.210 | 89 | 64 | 56 | FND§§ | FND | FND |
| 4 | 2 | 0.160 | 0.260 | | | | FND&& | FND&& | |
| 5 | 3 | 1.300 | | | | | FND | FND | FND |
| 6 | 4 | 1.300 | | | | | | | |
| 7 | 5 | 0.750 | | | | | | | |
| 8 | 6 | 7.800 | | | | | | | |
| 9 | 7 | 1.900 | | | | | | | |
| 10 | 8 | 2.600 | | | | | | | |
| 11 | 9 | 1.100 | | | | | | | |
| 12 | 10 | 0.120 | | | | | | | |
| 13 | 11 | 2.300 | | | | | | | |
| 14 | 12 | 0.088 | | | | | | | |

(continued)

| SEQ ID NO. | Compd . No. | hCTR2 $EC_{50}$ (nM) | hAMY R3 $EC_{50}$ (nM) | pH 4 | pH 7.5 | pH 9 | pH 4.0 (Ac) | pH 7.0 (Ph) | pH 7.0 (His) |
|---|---|---|---|---|---|---|---|---|---|
| 15 | 13 | 0.067 | | | | | | | |
| 16 | 14 | 0.210 | | | | | | | |
| 17 | 15 | 0.160 | | | | | | | |
| 18 | 16 | 0.076 | | | | | | | |
| 19 | 17 | 0.093 | | | | | | | |
| 20 | 18 | 0.060 | 0.17 | | | | 20 | 33 | 33 |
| 21 | 19 | 0.071 | | | | | | | |
| 22 | 20 | 0.094 | | | | | | | |
| 23 | 21 | 0.380 | | | | | | | |
| 24 | 22 | 0.310 | | | | | | | |
| 25 | 23 | 0.074 | | | | | | | |
| 26 | 24 | 0.130 | | | | | | | |
| 27 | 25 | 0.100 | | | | | | | |
| 28 | 26 | 0.110 | | | | | | | |
| 29 | 27 | 0.140 | | | | | | | |
| 30 | 28 | 0.160 | | | | | | | |
| 31 | 29 | 0.420 | | 100 | 69 | 53 | | | |
| 32 | 30 | 0.560 | | | | | | | |
| 33 | 31 | 0.130 | | 80 | 66 | | | | |
| 34 | 32 | 0.120 | | | | | | | |
| 35 | 33 | 0.110 | | | | | | | |
| 36 | 34 | 0.260 | | | | | | | |
| 37 | 35 | 0.160 | | | | | | | |
| 38 | 36 | 0.170 | | | | | | | |
| 39 | 37 | 0.390 | | | | | | | |
| 40 | 38 | 0.160 | | | | | | | |
| 41 | 39 | 0.190 | | | | | | | |
| 42 | 40 | 0.380 | | | | | | | |
| 43 | 41 | 0.071 | | | | | | | |
| 44 | 42 | 0.120 | | | | | | | |
| 45 | 43 | 0.100 | | | | | | | |
| 46 | 44 | 0.150 | | | | | | | |
| 47 | 45 | 0.140 | | | | | | | |
| 48 | 46 | 0.150 | | | | | | | |
| 49 | 47 | 0.060 | | | | | | | |
| 50 | 48 | 0.120 | | | | | | | |
| 51 | 49 | 0.140 | | | | | | | |

(continued)

| SEQ ID NO. | Compd . No. | hCTR2 $EC_{50}$ (nM) | hAMY R3 $EC_{50}$ (nM) | pH 4 | pH 7.5 | pH 9 | pH 4.0 (Ac) | pH 7.0 (Ph) | pH 7.0 (His) |
|---|---|---|---|---|---|---|---|---|---|
| 52 | 50 | 0.140 | | | | | | | |
| 53 | 51 | 0.093 | | | | | | | |
| 54 | 52 | 0.076 | | 95 | 92 | 84 | | | |
| 55 | 53 | 0.120 | | | | | | | |
| 56 | 54 | 0.091 | | | | | | | |
| 57 | 55 | 0.076 | | | | | | | |
| 58 | 56 | 0.140 | | | | | | | |
| 59 | 57 | 0.130 | | | | | | | |
| 60 | 58 | 0.110 | | | | | | | |
| 61 | 59 | 0.140 | | | | | | | |
| 62 | 60 | 0.120 | | | | | | | |
| 63 | 61 | 0.300 | | | | | FND | 3 | 3 |
| 64 | 62 | 0.370 | | | | | | | |
| 65 | 63 | 0.200 | | | | | | | |
| 66 | 64 | 0.400 | | | | | | | |
| 67 | 65 | 0.140 | | | | | | | |
| 68 | 66 | 0.110 | | | | | | | |
| 69 | 67 | 0.190 | | | | | | | |
| 70 | 68 | 0.096 | | | | | | | |
| 71 | 69 | 0.240 | | | | | | | |
| 72 | 70 | 0.110 | | | | | | | |
| 73 | 71 | 0.068 | | | | | | | |
| 74 | 72 | 0.071 | 0.098 | 90 | 81 | 80 | FND | FND | FND |
| 75 | 73 | 0.072 | 0.140 | 95 | 85 | 88 | FND | FND | FND |
| 76 | 74 | 0.097 | | | | | | | |
| 77 | 75 | 0.110 | | | | | | | |
| 78 | 76 | 0.190 | 0.240 | 101 | 85 | 86 | FND | FND | FND |
| 79 | 77 | 0.170 | 0.240 | | | | | | |
| 80 | 78 | 0.070 | | | | | | | |
| 81 | 79 | 0.100 | | | | | | | |
| 82 | 80 | 0.062 | | | | | | | |
| 83 | 81 | 0.057 | | | | | | | |
| 84 | 82 | 0.070 | 0.120 | 99 | 97 | 96 | FND | FND | FND |
| 85 | 83 | 0.076 | 0.095 | | | | | | |
| 86 | 84 | 0.110 | 0.100 | | | | | | |
| 87 | 85 | 0.083 | | | | | | | |
| 88 | 86 | 0.065 | | | | | | | |

(continued)

| SEQ ID NO. | Compd . No. | hCTR2 $EC_{50}$ (nM) | hAMY R3 $EC_{50}$ (nM) | pH 4 | pH 7.5 | pH 9 | pH 4.0 (Ac) | pH 7.0 (Ph) | pH 7.0 (His) |
|---|---|---|---|---|---|---|---|---|---|
| 89 | 87 | 0.046 | | 63 | 39 | 40 | 78‡ | 50‡‡ | FND |
| 90 | 88 | 0.069 | | | | | | | |
| 90 | 89 | 0.054 | | | | | | | |
| 90 | 90 | 0.066 | | | | | | | |
| 90 | 91 | 0.047 | | | | | | | |
| 90 | 92 | 0.054 | | | | | | | |
| 90 | 93 | 0.064 | | | | | | | |
| 90 | 94 | 0.078 | | | | | | | |
| 91 | 95 | 0.082 | | 97 | 90 | 90 | | | |
| 92 | 96 | 0.340 | 5.600 | | | | | | |
| 93 | 97 | 0.330 | 0.680 | | | | | | |
| 94 | 98 | 0.350 | 1.700 | | | | | | |
| 95 | 99 | 0.270 | 2.200 | | | | | | |
| 96 | 100 | 0.370 | 6.300 | | | | | | |
| 97 | 101 | 0.600 | 2.700 | | | | | | |
| 98 | 102 | 0.420 | 1.000 | | | | | | |
| 99 | 103 | 0.650 | 5.100 | | | | | | |
| 100 | 104 | 0.700 | 4.800 | | | | | | |
| 101 | 105 | 0.083 | 0.092 | 57 | 57 | 42 | FND | FND | FND |
| 102 | 106 | 0.120 | 0.140 | | | | | | |
| 103 | 107 | 0.180 | 0.520 | | | | FND | 16 | FND |
| 104 | 108 | 0.320 | 0.670 | | | | | | |
| 105 | 109 | 0.300 | 0.520 | | | | | | |
| 106 | 110 | 0.160 | 0.087 | 74 | 66 | 59 | FND | FND | FND |
| 107 | 111 | 0.140 | 0.210 | | | | | | |
| 108 | 112 | 0.160 | 0.150 | | | | | | |
| 109 | 113 | 0.170 | 0.190 | | | | | | |
| 110 | 114 | 0.210 | 0.180 | | | | | | |
| 111 | 115 | 0.470 | 0.370 | | | | | | |
| 112 | 116 | 0.930 | 1.600 | | | | | | |
| 113 | 117 | 0.470 | 8.200 | | | | | | |
| 114 | 118 | 0.062 | 0.120 | 85 | 79 | 80 | FND | FND | FND |
| 115 | 119 | 0.170 | 0.300 | 90 | 61 | 76 | | | |
| 116 | 120 | 0.043 | 0.150 | 95 | 65 | 77 | FND | FND | FND |
| 117 | 121 | 0.150 | 0.300 | | | | | | |
| 118 | 122 | 0.110 | 0.200 | | | | | | |
| 119 | 123 | 0.280 | 0.290 | | | | | | |

(continued)

| SEQ ID NO. | Compd . No. | hCTR2 $EC_{50}$ (nM) | hAMY R3 $EC_{50}$ (nM) | pH 4 | pH 7.5 | pH 9 | pH 4.0 (Ac) | pH 7.0 (Ph) | pH 7.0 (His) |
|---|---|---|---|---|---|---|---|---|---|
| 120 | 124 | 0.140 | 0.170 | | | | | | |
| 121 | 125 | 0.061 | 0.260 | 77 | 70 | 68 | | | |
| 122 | 126 | 0.210 | 0.280 | | | | | | |
| 123 | 127 | 0.190 | 0.580 | 94 | 79 | 83 | | | |
| 124 | 128 | 0.280 | 0.450 | | | | | | |
| 125 | 129 | 0.150 | 0.150 | 86 | 51 | 64 | | | |
| 126 | 130 | 0.110 | 0.150 | 90 | 77 | 86 | | | |
| 127 | 131 | 0.100 | 0.180 | | | | | | |
| 128 | 132 | 0.220 | 0.350 | | | | | | |
| 129 | 133 | 0.300 | 0.360 | | | | | | |
| 130 | 134 | 0.220 | 0.650 | 94 | 46 | 62 | FND | FND | FND |
| 131 | 135 | 0.760 | 0.610 | | | | | | |
| 132 | 136 | 0.120 | 0.290 | 74 | 32 | 33 | | | |
| 133 | 137 | 0.240 | 0.860 | 93 | 60 | 73 | | | |
| 134 | 138 | 0.230 | 0.340 | | | | | | |
| 135 | 139 | 0.150 | 0.350 | | | | | | |
| 136 | 140 | 0.098 | 0.180 | | | | | | |
| 137 | 141 | 0.110 | 0.130 | | | | | | |
| 138 | 142 | 0.140 | 0.290 | | | | | | |
| 139 | 143 | 0.260 | 0.440 | | | | | | |
| 140 | 144 | 0.110 | 0.240 | | | | | | |
| 141 | 145 | 0.092 | 0.220 | | | | | | |
| 142 | 146 | 0.092 | 0.150 | | | | | | |
| 143 | 147 | 0.160 | 0.160 | | | | | | |
| 144 | 148 | 0.140 | 0.170 | | | | | | |
| 145 | 149 | 0.160 | 0.260 | | | | | | |
| 146 | 150 | 0.160 | 0.260 | | | | | | |
| 147 | 151 | 0.066 | 0.110 | | | | | | |
| 147 | 152 | 0.950 | 5.200 | | | | | | |
| 148 | 153 | 0.730 | 6.100 | | | | | | |
| 149 | 154 | 0.550 | 6.300 | | | | | | |
| 150 | 155 | 0.670 | 5.300 | | | | | | |
| 150 | 156 | 0.540 | 0.250 | | | | | | |
| 151 | 157 | 0.072 | 0.160 | 53 | 62 | 67 | | | |
| 152 | 158 | 0.048 | 0.110 | 58 | 51 | 72 | | | |
| 153 | 159 | 0.100 | 0.140 | | | | | | |
| 154 | 160 | 0.130 | 0.140 | | | | | | |

(continued)

| SEQ ID NO. | Compd . No. | hCTR2 $EC_{50}$ (nM) | hAMY R3 $EC_{50}$ (nM) | pH 4 | pH 7.5 | pH 9 | pH 4.0 (Ac) | pH 7.0 (Ph) | pH 7.0 (His) |
|---|---|---|---|---|---|---|---|---|---|
| 155 | 161 | 0.084 | 0.190 | | 90 | 78 | FND | FND | FND |
| 156 | 162 | 0.049 | 0.140 | 100 | 96 | 88 | FND | FND | FND |
| 157 | 163 | 0.420 | 0.740 | | | | | | |
| 158 | 164 | 0.100 | 0.270 | | | | | | |
| 159 | 165 | 0.110 | 0.270 | | | | | | |
| 160 | 166 | 0.074 | 0.110 | 95 | 91 | 92 | FND | FND | FND |
| 161 | 167 | 0.078 | 0.110 | | | | | | |
| 162 | 168 | 0.570 | 0.700 | | | | | | |
| 163 | 169 | 3.500 | 0.640 | | | | | | |
| 164 | 170 | 0.650 | 0.570 | | | | | | |
| 165 | 171 | 0.840 | 0.540 | | | | | | |
| 166 | 172 | 0.045 | 0.095 | 94 | 97 | 95 | FND | FND | FND |
| 167 | 173 | 0.062 | 0.140 | | | | | | |
| 168 | 174 | 0.180 | 0.180 | 92 | 31 | 39 | FND | FND | FND |
| 169 | 175 | 0.200 | 0.200 | | | | FND | FND | |
| 170 | 176 | 0.120 | 0.170 | | | | | | |
| 2 | pramlin tide | 1.40 | 0.230 | | | | 48 | 2 | |
| 171 | NN96 | 0.089 | 0.150 | | | | FND | 60 | 25 |

#Average of a minimum of 3 determinations. [**]Blanks in Table 2 indicate: not determined. [§§]FND = Fibrillation not detected. [&&]Purity of tested compound sample <90%. [‡]Lag-time determination based on one sample; FND in two out of three samples. [‡‡]Lag-time determination based on two samples; FND in one out of three samples.

Example 7

Pharmacokinetic (PK) profiling of compounds of the invention in rats

Sprague Dawley male rats were given a single subcutaneous (sc) bolus of each peptide to be tested at doses as specified below..

Study A: Following the administration of a 100 nmol/kg dose of a test peptide of the invention (Compound 18) and a comparison pramlintide analogue NN96 (see Example 5, above), respectively, blood samples were drawn from the tail vein at 0.5, 3, 8, 15, 36, 60 and 81 hours post-dosing. 4 rats were used (2 rats per compound). The rats were euthanized immediately after the last blood sampling by overdose of $O_2/CO_2$.

Study B: 20 nmol/kg doses of Compound 72 and Compound 76, respectively, of the invention were administered. Blood samples were drawn by sublingual bleeding prior to dosing and at 2, 6, 12, 24, 48, 72 and 96 hours after dosing. 4 rats were used (2 rats per compound). The rats were euthanized immediately after the last blood sampling by overdose of $O_2/CO_2$.

Study C: 30 nmol/kg doses of Compound 73, Compound 82 and Compound 118, respectively, of the invention were administered. Blood samples were drawn from the tail vein prior to dosing and at 24, 48, 72 and 96 hours after dosing. Blood samples were taken from two rats at each time point, and only 2 blood samples were drawn from each rat, i.e. 8 rats were used for each compound. The rats were euthanized immediately after the last blood sampling by concussion and cervical dislocation.

Study D: 30 nmol/kg doses of Compound 162, Compound 166 and Compound 172 were administered. Blood samples were drawn from the tail vein prior to dosing and at 24, 48, 72, 96 and 168 hours after dosing. Blood samples were taken from two rats at each time point, and only 2 blood samples were drawn from each rat, i.e. 10 rats were used

for each compound. The rats were euthanized immediately after the last blood sampling by concussion and cervical dislocation.

The dosing vehicle used for each test peptide was a mannitol-containing histidine buffer (pH 7.0). Plasma samples were analyzed after precipitation with ethanol by liquid chromatography mass spectrometry (LC-MS/MS). Mean plasma concentrations were used for calculation of the pharmacokinetic parameters using the non-compartmental approach in Phoenix WinNonlin 6.3.

Plasma terminal elimination half-life ($t_{½}$) was determined as ln(2)/λz, where λz is the magnitude of the slope of the log linear regression of the log concentration versus time profile during the terminal phase. Apparent clearance (CL/F) was determined as dose/$AUC_{inf}$ (sc), where $AUC_{inf}$ is the area under the plasma concentration vs. time curve extrapolated to infinity ($AUC_{inf} = AUC_{last} + C_{last}/\lambda z$, where $C_{last}$ is the last observed plasma concentration).

The observed PK data are summarized in Table 3 below, and the dose-normalized PK profiles are shown in Figure 1.

Table 3. Terminal elimination half-life (hours) and apparent clearance (liter/hour/kg) in rats following sc administration of Compounds 18, 72, 76, 73, 82, 118, 162, 166 and 172, and comparison compound NN96.

| Compound | $t_{½}$ (hours) | CL/F (liter/hour/kg) |
|---|---|---|
| 18 | 37.6 | 0.00348 |
| 72 | 30.9 | 0.0027 |
| 76 | 37.3 | 0.00353 |
| 73 | 55.4 | 0.00306 |
| 82 | 44.2 | 0.00401 |
| 118 | 56.4 | 0.00351 |
| 162 | 29.8 | 0.00592 |
| 166 | 27.9 | 0.00379 |
| 172 | 24.4 | 0.00916 |
| NN96 | 34.7 | 0.00473 |

Example 8

Effect of compounds of the invention on acute food intake and body weight in normal Sprague Dawley rats

Sprague Dawley (SD) rats were obtained from Taconic A/S, Denmark. The animals arrived at least 7 days before the study start to allow acclimatization to experimental conditions. From arrival and throughout the study, the rats were housed in groups of 2 (n = 2) in a light-, temperature- and humidity-controlled room (reversed 12/12 h light/dark cycle: lights turned off during day-time and on during night-time; temperature 20-22°C; relative humidity 50-80%). Animals had access ad libitum to food (Altromin™ 1324, Brogaarden A/S, Gentofte, Denmark) and water (domestic quality tap water with citric acid added to pH ~ 3.6) during the entire study. Stratification of rats was based on body weight (BW) on a cage-by-cage basis; mean BWs per cage were used as the basis for pairing four cages together in a group (n = 8 per group). A vehicle group and positive control group were included in each set of tests. Rats were dosed subcutaneously (sc) once in the morning immediately before turning off the lights, using a body weight-corrected dose of peptide (30 nmol/kg). Dosing volume was 5 ml/kg. Food intake was recorded manually at t = 0 (pre-dose) and at t = 4, 7, 10, 24, 48, 72 and 96 hours after dosing. Body weight was measured daily.

Statistical analyses on body weight were performed using GraphPad™ Prism version 5. The measured parameters were compared using unpaired t tests. Differences were considered statistically significant at $p < 0.05$. Data are means ± SEM; n =8.

Table 4 below summarizes data (food intake inhibition after 48 hours, and body weight loss after 96 hours) obtained for tested compounds of the invention and for a comparison pramlintide analogue reported in the literature (denoted NN96; see Example 5 above).

Table 4. Vehicle-corrected data for acute food intake inhibition (%, after 48 hours) and for body weight loss (mean ± SEM; %, after 96 hours) following sc administration (dose 30 nmol/kg sc) of Compounds 1, 18, 61, 72, 73, 76, 82, 87, 105, 107, 110, 118, 120, 134, 161, 162, 166, 172 and 174, and comparison compound NN96.

| Compound | Food intake inhibition (%) after 48 hours | Body weight loss (%) after 96 hours |
|---|---|---|
| 18 | 98 | 13 ± 0.8 |
| 1 | 98 | 9 ± 1.8 |
| 61 | 24 | 1 ± 1.6# |
| 72 | 99 | 12 ± 1.2 |
| 76 | 100 | 11 ± 1.2 |
| 87 | 98 | 10 ± 1.2 |
| 107 | 83 | 6 ± 1.5 |
| 110 | 98 | 11 ± 1.1 |
| 105 | ND§ | 7 ± 2.4 |
| 120 | ND§ | 12 ± 1.8 |
| 134 | 27 | 4 ± 3.1# |
| 73 | 93 | 11 ± 1.8 |
| 82 | 93 | 13 ± 1.0 |
| 118 | 93 | 13 ± 1.8 |
| 162 | 99 | 13 ± 2.2 |
| 166 | 99 | 12 ± 1.6 |
| 172 | 99 | 13 ± 1.1 |
| 161 | 99 | 10 ± 2.7 |
| 174 | 99 | 8 ± 1.4 |
| NN96 | 100 | 14 ± 1.8 |
| §ND = not determined. #Non-significant vs. vehicle | | |

Results

24 hours after dosing, the majority of the tested compounds had induced almost complete anorexia that persisted at 48 h after the dosing. The pronounced reduction in food intake was reflected by a significant decrease in body weight observed on day 4 post-dosing (after 96 h) in the treated rats. The effect on food inhibition was transient, i.e. was reversed following cessation of dosing of test compound.

Example 9

Efficacy testing in relation to development of obesity in rats fed on a high-fat diet (HFD)

Male Sprague Dawley rats obtained from Taconic A/S, Denmark, were used in this study. The animals arrived at least 7 days before the study start to allow acclimatization to experimental conditions. From arrival and throughout the study, the rats were housed in groups of 2 (n = 2) in a light-, temperature- and humidity-controlled room (reversed 12/12 h light/dark cycle: lights turned off during day-time and on during night-time; temperature 20-22°C; relative humidity 50-80%) and maintained on a HFD (60% of total energy from fat, D12492, Research Diet Inc., New Brunswick, USA) for 2 weeks. Animals had access ad libitum to water (domestic quality tap water with citric acid added to pH ~ 3.6) during the entire study.The rats were stratified according to body weight (primary) and % body weight gain from the beginning of feeding with HFD (secondary), and treated for 4 weeks with Compound 18 [1, 5 and 30 nmol/kg sc, once weekly

(QW)] and Compound 18 (5 nmol/kg sc, every 4$^{th}$ day), and with comparison/reference compounds NN96 (a pramlintide analogue, see Example 5; 30 nmol/kg sc, QW) and the marketed human GLP-1 receptor agonist liraglutide [50 nmol/kg sc, twice daily (bid)], respectively.

Statistical analyses were performed using GraphPad™ Prism version 5.The measured parameters were compared using one-way ANOVA, or Kruskal-Wallis test in cases where variances were significantly different. Post-hoc Dunnett's multiple comparison tests were conducted. When appropriate, the two-way ANOVA test was used followed by Bonferroni post tests; $p < 0.05$ was in all cases considered statistically significant.

The preventive effects of Compound 18 of the present invention on the development of obesity were investigated by measurement of body weight, food intake, water intake, blood glucose levels, plasma insulin levels, liver fat mass and fat depots. The data are summarized in Figs. 2-5 herein.

Results

It is apparent from Figs. 2-5 that Compound 18 had a significant, dose-dependent preventive effect on the development of obesity in HFD-fed rats. The anti-obesity effects were characterized by: significantly decreased food intake; a 26 % (high-dose group) reduction in body weight gain at the end of the study; a decrease in terminal liver fat mass (5 and 30 nmol/kg, QW); as well as a decrease in mass of fat depots. In addition, significant differences in terminal blood glucose levels (5 and 30 nmol/kg, QW) and plasma insulin levels (5 and 30 nmol/kg, QW) were observed in animals treated with Compound 18 as compared to vehicle-treated rats, indicating a reduction in the development of insulin resistance in HFD-fed rats.

Administration of Compound 18 every 4$^{th}$ day resulted in effects similar to those of Compound 18 dosed QW.

It thus appears that treatment with the amylin analogue of the invention in question, Compound 18, prevents the development of obesity in rats on HFD. The observed effects included a decrease in food intake, reduction in body weight gain, and reduction in adiposity. In addition, beneficial effects were observed on circulating levels of glucose and insulin.

Example 10

Efficacy testing in relation to food preference (FP) and development of obesity in rats fed on a high-fat diet (HFD)

Male Sprague Dawley rats obtained from Taconic A/S, Denmark, were used in this study. The animals arrived at least 7 days before the study start to allow acclimatization to experimental conditions. From arrival and throughout the study, the rats were housed in groups of 2 (n = 2) in a light-, temperature- and humidity-controlled room (reversed 12/12 h light/dark cycle: lights turned off during day-time and on during night-time; temperature 20-22°C; relative humidity 50-80%) and maintained on a HFD (60% of total energy from fat, D12492, Research Diet Inc., New Brunswick, USA) for 2 weeks. Animals had access ad libitum to water (domestic quality tap water with citric acid added to pH ~ 3.6) during the entire study. The rats were stratified according to body weight (primary) and % body weight gain from the beginning of feeding with HFD (secondary). Three days before the initiation of treatment the rats were allowed to self-select between a low-fat diet (LFD, standard chow, 11 % of total energy from fat, Atromin™ 1324, Brogaarden A/S, Gentofte, Denmark) and a high-fat diet, and they remained on the self-selected regimen for the duration of the study. Rats were treated for four weeks with Compound 18 [30 nmol/kg sc, once weekly (QW)] or vehicle.

Statistical analyses were performed using GraphPad™ Prism version 5. The comparison between the vehicle control group and treatment group was done either by two-way ANOVA test followed by Bonferroni post tests, or by unpaired t-test or Mann Whitney test in cases where variances were significantly different; $p < 0.05$ was considered statistically significant.

The efficacy of Compound 18 in relation to change in food preference and in relation to the development of obesity were investigated by measurement of food preference, body weight, food intake, water intake, blood glucose levels, plasma insulin levels, mass of liver fat and mass of fat depots. The data are summarized in Figs. 6-10 herein.

Results

The food preference study conducted with Compound 18 demonstrated a decrease in the total caloric intake combined from both diets in rats treated with Compound 18, and that there was a a significant increase in caloric intake deriving from LFD, suggesting shifted preference for the low-fat chow. The significant decrease in food intake and calorie consumption resulted in reduction of body weight gain in rats treated with Compound 18. The overall anti-obesity effects were comparable to the effects observed in rats treated with an equimolar dose of Compound 18 and maintained strictly on HFD (see Example 9, above) except that terminal blood glucose levels were unaffected (Fig. 10). The lack of effect on terminal blood glucose levels may be due to the fact that blood glucose levels were lower in both vehicle and Compound

18 groups, compared to the baseline levels.

Example 11

Efficacy testing in relation to glycemic control in Zucker diabetic fatty rats

84 male Zucker diabetic fatty (ZDF) rats (9-10 weeks of age) were used in this study. The animals were housed in groups of n = 2. Stratification of the animals was based on non-fasting blood glucose (primary), changes in non-fasting blood glucose measured from the arrival of the animals until one week before the initiation of the study (secondary) and body weight (tertiary). The animals were further divided into two "teams" with a pair-fed group (group 2) allocated to team 2. Animals from group 2 were pair-fed in order to compare between the contribution made by reduced food intake and by drug-induced effects on glycemic control. The amount of food eaten by the animals treated with Compound 82 (30 nmol/kg, team 1) each day was determined, and the same amount was given to a vehicle-treated pair-fed group of animals the following day.

Rats were treated for 4 weeks with Compound 82 (3, 30 and 100 nmol/kg, sc, every (ev.) $5^{th}$ day), Compound 18 (30 nmol/kg sc, every $5^{th}$ day), or the GLP-1 analogue liraglutide (40 nmol/kg sc, twice daily (bid)).

The effects of the compounds on glycemic control were investigated by measurement of non-fasting and fasting blood glucose levels and HbA1c levels.

Statistical analyses were performed using GraphPad™ Prism version 5. The measured parameters were compared using one- way ANOVA followed by Dunnett's multiple comparison test. Comparison between the pair-fed vehicle-treated group and the Compound 82 (30 nmol/kg) group was done using Student's two-tailed and unpaired t-test. Differences were considered statistically significant at $p < 0.05$.

Results

In all treatment groups, non-fasting and fasting blood glucose levels at the start of treatment were not different from those in the vehicle-treated rats (see Fig.11 A and D). Rats treated with Compound 82 or Compound 18 had significantly lower non-fasting blood glucose levels on day 13 and on day 27 compared to the vehicle treated rats ($P < 0.001$, Fig. 11 B, C). Also, fasting blood glucose levels were significantly decreased in rats treated with Compound 82 (3, 30, and 100 nmol/kg, $P < 0.001$, Fig. 11 E) or with Compound 18 (30 nmol/kg, $P < 0.001$, Fig. 11 E). In addition, both non-fasting and fasting blood glucose levels were significantly different at the end of the study in rats treated with Compound 82 (30 nmol/kg, $P < 0.05$, Fig. 11 C and E) compared to the levels in rats from the pair-fed group. The reference compound liraglutide also exhibited a significant blood glucose lowering effect ($P < 0.001$, Fig. 11 B, C, and E). Beneficial effects on blood glucose levels were reflected in terminal glycated hemoglobin (HbA1c) (%) levels. Thus, treatment with Compound 82 caused a significant and dose-dependent reduction in terminal HbA1c levels compared to the vehicle group [$5.6 \pm 0.2$ (Compound 82, 3 nmol/kg); $5.4 \pm 0.1$ (Compound 82, 30 nmol/kg); $5.4 \pm 0.1$ (Compound 82, 100 nmol/kg), $P < 0.001$; Fig. 12 A]. Significantly lowered HbA1c levels were also observed in rats treated with Compound 18 ($5.4 \pm 0.1$, $P < 0.001$) or with liraglutide ($5.8 \pm 0.2$, $P < 0.001$) and in the pair-fed group ($6.7 \pm 0.4$, $P < 0.001$) compared to levels in the vehicle-treated rats ($8.7 \pm 0.2$). Moreover, significant reductions in HbA1c levels were observed in rats treated with Compound 82 (30 nmol/kg, $P < 0.05$, Fig. 12 A) compared to the pair-fed group. Fig. 12 B shows changes in HbA1c levels from the baseline to the termination of the study.

The tested compounds of the invention thus improved glycemic control in ZDF rats by decreasing non-fasting and fasting blood glucose levels and lowering terminal Hb1Ac levels.

Example 12

Pharmacokinetics of test compounds in mice

NMRI mice (supplier: Taconic; females with a body weight of approximately 25 g) were given either a single subcutaneous (sc) bolus or a single intravenous (iv) bolus of each peptide to be tested.

Following sc administration of test peptide (Compound 176, 1 or 18) (100 nmol/kg), blood samples were drawn 0.5, 2, 4, 8, 18, 40 and 56 hours post-dosing by eye bleeding. Following iv administration of Compound 176, 1 or 18 (100 nmol/kg), blood samples were drawn 0.08, 0.17, 0.5, 2, 8, 18 and 30 hours post-dosing by eye bleeding. The dosing vehicle used for each test peptide was a mannitol-containing histidine buffer (pH 7.0).

Pramlintide (400 nmol/kg) was also administered as a single sc bolus or as a single iv bolus to mice. Following sc administration, blood samples were drawn 5, 15, 20, 30, 60, 120, 180 and 240 minutes post-dosing by sublingual bleeding. Following iv administration, blood samples were drawn 5, 10, 15, 30, 60, 120, 180, and 240 minutes post-dosing by sublingual bleeding. The dosing vehicle used for Pramlintide was a mannitol-containing acetate buffer (pH 4.0).

Blood samples were taken from two mice at each time point, i.e. 14-16 mice were included for each compound. The mice were euthanized immediately after blood sampling by cervical dislocation. Plasma samples were analyzed after solid-phase extraction (SPE) by liquid chromatography mass spectrometry (LC-MS/MS). Mean plasma concentrations were used for calculation of the pharmacokinetic parameters using the non-compartmental approach in Phoenix WinNonlin 6.3.

Plasma terminal elimination half-life ($t_{½}$) was determined as ln(2)/λz, where λz is the magnitude of the slope of the log linear regression of the log concentration versus time profile during the terminal phase.

Bioavailability was determined as $AUC_{inf}$ (sc) / $AUC_{inf}$ (iv) x 100, where $AUC_{inf}$ is the area under the plasma concentration - time curve extrapolated to infinity ($AUC_{inf} = AUC_{last} + C_{last}/\lambda z$, where $C_{last}$ is the last observed plasma concentration).

Data are summarized in Table 5, below.

Table 5. Terminal elimination half-life (h) and bioavailability in mice following sc and iv administration of Compounds 176, 1 and 18, and Pramlintide

| **Compound** | **t½ (hours)** | | **Bioavailability** |
|---|---|---|---|
| | iv | sc | sc |
| Pramlintide | 0.1 | 0.3 | 46% |
| 176 | 24.4 | 23.8 | 81% |
| 1 | 32.9 | 34.6 | 77% |
| 18 | 20.8 | 22.8 | 79% |

Example 13

Effect of test compounds on acute food intake in normal mice

The mice were kept in an automated food- and water-intake monitoring system (HM-2 system, MBRose, Denmark) which allows automated on-line measurements of accumulated food-intake and other parameters. Normal chow-fed C57BL/6J male mice were used. The mice were kept 6 per cage, and each dosing group consisted of 7-8 animals. Prior to the experiment, the animals were stratified into groups based on their body weight. The animals were dosed once subcutaneously with compounds (50 or 200 nmol/kg) or vehicle (50 mM histidine buffer containing 200 mM mannitol, pH 7), just prior to the dark period (lights off between 11.00 and 23.00 hr) in which they are active and eat. The animals were monitored by the HM-2 system up to 24 h after dosing.

The results are presented in Figure 13 (50 nmol/kg dosing) and Figure 14 (200 nmol/kg dosing). Data are mean ± SEM. *, $p < 0.05$ vs. vehicle. Two-way ANOVA followed by Bonferroni post-tests was used for the statistical analyses.

## Claims

1. A compound having the formula I:

$R^1$-Z-$R^2$ (I)

wherein

$R^1$ is hydrogen, $C_{1-4}$ acyl, benzoyl or $C_{1-4}$ alkyl, or a half-life extending moiety M, wherein M is optionally linked to Z via a linker moiety L;
$R^2$ is OH or $NHR^3$, wherein $R^3$ is hydrogen or $C_{1-3}$-alkyl; and
Z is an amino acid sequence of formula Id:
X1-Cys()-X3-Thr-Ala-Thr-Cys()-Ala-Thr-X10-Arg-Leu-Ala-X14-Phe-X16-X17-X18-X19-X20-X21-X22-X23-Gly(Me)-X25-Ile(Me)-X27-X28-X29-X30-X31-Val-Gly-Ser-X35-Thr-X37 (Id);
wherein

X1 is selected from the group consisting of Trp, Lys and Arg;
X3 is selected from the group consisting of Gly, Asn, Glu and Pro;

X10 is selected from the group consisting of Gln, Asp and Glu;
X14 is selected from the group consisting of Asp and Glu;
X16 is Leu or is absent;
X17 is His or is absent;
X18 is selected from the group consisting of His and Arg, or is absent;
X19 is selected from the group consisting of Ser and D-Ser, or is absent;
X20 is Ser or is absent;
X21 is selected from the group consisting of Asn and Ser, or is absent;
X22 is selected from the group consisting of Asn and Ser, or is absent;
X23 is selected from the group consisting of Phe and D-Phe, or is absent;
X25 is Ala or is absent;
X27 is Leu or is absent;
X28 is selected from the group consisting of Ser, Ser(Me), D-Ser and D-Ser(Me), or is absent;
X29 is selected from the group consisting of Ser and Ser(Me) or is absent;
X30 is Thr or is absent;
X31 is selected from the group consisting of Asp, Glu and Asn;
X35 is selected from the group consisting of Asp, Glu and Asn; and
X37 is selected from the group consisting of Tyr, Tyr(Me), Pro, Hyp, Apr, Aze, Pip, Php and Bep;

wherein the parentheses () associated with the two cysteine (C, Cys) residues at sequence positions 2 and 7 indicate the presence of an intramolecular disulfide bridge between the two Cys residues in question;
wherein no more than three positions may be absent, with the proviso that:

(i) if two positions are absent, they are adjacent to one another or one of the absent positions is X25; and
(ii) if three positions are absent, two are adjacent to one another and the other is X25; and wherein the compound has improved potency compared to human amylin at one or more of the receptors hCTR2, hAMRY1, hAMRY2 or hAMRY3;

or a pharmaceutically acceptable salt or solvate thereof.

2. A compound or pharmaceutically acceptable salt or solvate thereof according to claim 1 wherein Z is an amino acid sequence of formula Ie:

X1-Cys()-X3-Thr-Ala-Thr-Cys()-Ala-Thr-X10-Arg-Leu-Ala-Glu-Phe-Leu-His-X18-Ser-Ser-X21-X22-X23-Gly(Me)-Ala-Ile(Me)-Leu-X28-Ser-Thr-X31-Val-Gly-Ser-X35-Thr-X37 (Ie);

wherein

X1 is selected from the group consisting of Trp, Lys and Arg;
X3 is selected from the group consisting of Gly, Asn and Pro;
X10 is selected from the group consisting of Gln, Asp and Glu;
X18 is selected from the group consisting of His and Arg;
X21 is selected from the group consisting of Asn and Ser, or is absent;
X22 is selected from the group consisting of Asn and Ser, or is absent;
X23 is selected from the group consisting of Phe and D-Phe;
X28 is selected from the group consisting of Ser and D-Ser;
X31 is selected from the group consisting of Asp, Glu and Asn;
X35 is selected from the group consisting of Asp, Glu and Asn; and
X37 is selected from the group consisting of Pro and Hyp.

3. A compound or pharmaceutically acceptable salt or solvate thereof according to any one of the preceding claims wherein:

(i) X3 is selected from Asn, Gly and Pro, e.g. Asn; and/or
(ii) X10 is Gln.

4. A compound or pharmaceutically acceptable salt or solvate thereof according to claim 1 or claim 3 when dependent thereon, wherein X14 is Glu.

5. A compound or pharmaceutically acceptable salt or solvate thereof according to any one of the preceding claims wherein:

(i) X23 is D-Phe and/or X28 is D-Ser;
(ii) X21 and/or X22 are absent;
(iii) X21 and/or X22 are Asn; and/or
(iv) X21 and/or X22 are Ser.

6. A compound or pharmaceutically acceptable salt or solvate thereof according to claim 1 or any of claims 3 to 5 when dependent thereon wherein:

(i) X19 and/or X20 are absent; or
(ii) X19 and/or X20 are Ser.

7. A compound or pharmaceutically acceptable salt or solvate thereof according to claim 1 or any of claims 3 to 6 when dependent thereon wherein X25 is absent.

8. A compound or pharmaceutically acceptable salt or solvate thereof according to any one of the preceding claims wherein:

(i) X31 and/or X35 are Glu;
(ii) X31 and/or X35 are Asp; or
(iii) X31 and/or X35 are Asn.

9. A compound or pharmaceutically acceptable salt or solvate thereof according to any one of the preceding claims wherein Z has a sequence selected from the group consisting of:

| | |
|---|---|
| RC()NTATC()ATQRLAEFLHHSSNNF-Gly(Me)-A-Ile(Me)-LSSTNVGSNTP | SEQ ID NO: 3 |
| RC()NTATC()ATQRLAEFLHHSSNNF-Gly(Me)-Ile(Me)-LSSTNVGSNTP | SEQ ID NO: 4 |
| RC()NTATC()ATQRLAEFLHHSSNN-DPhe-Gly(Me)-A-Ile(Me)-LSSTNVGSNTP | SEQ ID NO: 15 |
| RC()NTATC()ATQRLAEFLHRSSNNF-Gly(Me)-A-Ile(Me)-LSSTNVGSNTP | SEQ ID NO: 20 |
| KC()NTATC()ATQRLAEFLHHSSNNF-Gly(Me)-A-Ile(Me)-LSSTNVGSNTP | SEQ ID NO: 21 |
| KC()NTATC()ATQRLAEFLHHSSNNF-Gly(Me)-Ile(Me)-LSSTNVGSNTP | SEQ ID NO: 22 |
| RC()NTATC()ATQRLAEFLHHSSNNF-Gly(Me)-A-Ile(Me)-L-DSer-STNVGSNTP | SEQ ID NO: 25 |
| RC()NTATC()ATQRLAEFLHHSSNNF-Gly(Me)-Ile(Me)-L-DSer-STNVGSNTP | SEQ ID NO: 26 |
| RC()NTATC()ATQRLAEFLHHSSNNF-Gly(Me)-A-Ile(Me)-LSTNVGSNTP | SEQ ID NO: 27 |
| RC()NTATC()ATQRLAEFLHHSSNNF-Gly(Me)-Ile(Me)-LSTNVGSNTP SEQ | ID NO: 28 |
| RC()NTATC()ATQRLAEFLHH-DSer-SNNF-Gly(Me)-A-Ile(Me)-LSSTNVGSNTP | SEQ ID NO: 31 |
| RC()NTATC()ATQRLAEFLHH-DSer-SNNF-Gly(Me)-Ile(Me)-LSSTNVGSNTP | SEQ ID NO: 32 |
| RC()NTATC()ATQRLAEFLHHSSNNF-Gly(Me)-A-Ile(Me)-LSSNVGSNTP | SEQ ID NO: 39 |
| RC()NTATC()ATQRLAEFLHHSSNF-Gly(Me)-Ile(Me)-LSSTNVGSNTP | SEQ ID NO: 40 |
| RC()NTATC()ATQRLAEFLHSSNNF-Gly(Me)-A-Ile(Me)-LSSTNVGSNTP | SEQ ID NO: 41 |
| RC()NTATC()ATQRLAEFLHSSNNF-Gly(Me)-Ile(Me)-LSSTNVGSNTP | SEQ ID NO: 42 |
| RC()NTATC()ATQRLAEFLSSNNF-Gly(Me)-A-Ile(Me)-LSSTNVGSNTP | SEQ ID NO: 43 |
| RC()NTATC()ATQRLAEFLSSNNF-Gly(Me)-Ile(Me)-LSSTNVGSNTP | SEQ ID NO: 44 |
| RC()NTATC()ATQRLAEFLHHSSNF-Gly(Me)-A-Ile(Me)-LSSTNVGSNTP | SEQ ID NO: 46 |
| RC()NTATC()ATQRLAEFLHHSNNF-Gly(Me)-A-Ile(Me)-LSSTNVGSNTP | SEQ ID NO: 47 |
| RC()NTATC()ATQRLAEFLHHSNNF-Gly(Me)-Ile(Me)-LSSTNVGSNTP | SEQ ID NO: 48 |
| RC()NTATC()ATQRLAEFLHHNNF-Gly(Me)-A-Ile(Me)-LSSTNVGSNTP | SEQ ID NO: 49 |
| RC()NTATC()ATQRLAEFLHHNNF-Gly(Me)-Ile(Me)-LSSTNVGSNTP | SEQ ID NO: 50 |
| RC()NTATC()ATQRLAEFLHHSSF-Gly(Me)-A-Ile(Me)-LSSTNVGSNTP | SEQ ID NO: 54 |
| RC()NTATC()ATQRLAEFLHHSSF-Gly(Me)-Ile(Me)-LSSTNVGSNTP | SEQ ID NO: 55 |
| RC()NTATC()ATQRLAEFLHHSSNNF-Gly(Me)-A-Ile(Me)-SSTNVGSNTP | SEQ ID NO: 56 |
| RC()NTATC()ATQRLAEFLHHSSNNF-Gly(Me)-Ile(Me)-SSTNVGSNTP | SEQ ID NO: 57 |
| KC()NTATC()ATQRLAEFLHRSSNNF-Gly(Me)-A-Ile(Me)-LSSTNVGSNTP | SEQ ID NO: 59 |

KC()NTATC()ATQRLAEFLHRSSNNF-Gly(Me)-Ile(Me)-LSSTNVGSNTP SEQ ID NO: 60
RC()NTATC()ATQRLAEFLHHSSNN-DPhe-Gly(Me)-A-Ile(Me)-L-DSer-STNVGSNTP SEQ ID NO: 61
RC()NTATC()ATQRLAEFLHHSSNN-DPhe-Gly(Me)-Ile(Me)-L-DSer-STNVGSNTP SEQ ID NO: 62
RC()NTATC()ATQRLAEFLHHSSNNF-Gly(Me)-A-Ile(Me)-LSSTNVGSNT-Tyr(Me) SEQ ID NO: 63
RC()NTATC()ATQRLAEFLHHSSNNF-Gly(Me)-Ile(Me)-LSSTNVGSNT-Tyr(Me) SEQ ID NO: 64
KC()NTATC()ATQRLAEFLHRSSNNF-Gly(Me)-A-Ile(Me)-L-DSer-STNVGSNTP SEQ ID NO: 70
KC()NTATC()ATQRLAEFLHRSSNNF-Gly(Me)-Ile(Me)-L-DSer-STNVGSNTP SEQ ID NO: 72
KC()NTATC()ATQRLAEFLHRSSNN-DPhe-Gly(Me)-A-Ile(Me)-LSSTNVGSNTP SEQ ID NO: 73
KC()NTATC()ATQRLAEFLHRSSNN-DPhe-Gly(Me)-A-Ile(Me)-L-DSer-STNVGSNTP SEQ ID NO: 74
RC()NTATC()ATQRLAEFLHRSSSSF-Gly(Me)-A-Ile(Me)-LSSTDVGSDTP SEQ ID NO: 75
RC()NTATC()ATQRLAEFLHRSSSSF-Gly(Me)-A-Ile(Me)-LSSTDVGSETP SEQ ID NO: 76
RC()NTATC()ATQRLAEFLHRSSSSF-Gly(Me)-Ile(Me)-LSSTDVGSETP SEQ ID NO: 77
RC()NTATC()ATQRLAEFLHRSSSSF-Gly(Me)-A-Ile(Me)-LSSTEVGSETP SEQ ID NO: 78
RC()NTATC()ATQRLAEFLHRSSSSF-Gly(Me)-Ile(Me)-LSSTEVGSETP SEQ ID NO: 79
RC()NTATC()ATQRLAEFLHRSSF-Gly(Me)-A-Ile(Me)-LSSTEVGSDTP SEQ ID NO: 80
RC()NTATC()ATQRLAEFLHRSSF-Gly(Me)-Ile(Me)-LSSTEVGSDTP SEQ ID NO: 81
RC()NTATC()ATQRLAEFLHRSSF-Gly(Me)-A-Ile(Me)-LSSTDVGSETP SEQ ID NO: 82
RC()NTATC()ATQRLAEFLHRSSF-Gly(Me)-A-Ile(Me)-LSSTDVGSDTP SEQ ID NO: 83
RC()NTATC()ATQRLAEFLHRSSF-Gly(Me)-A-Ile(Me)-LSSTEVGSETP SEQ ID NO: 84
RC()NTATC()ATQRLAEFLHRSSF-Gly(Me)-Ile(Me)-LSSTEVGSETP SEQ ID NO: 91
RC()NTATC()ATQRLAEFLHRSSNNF-Gly(Me)-A-Ile(Me)-LSSTNVGSNT-DPro SEQ ID NO: 100
RC()NTATC()ATQRLAEFLHRSSNNF-Gly(Me)-A-Ile(Me)-LSSTNVGSNT-Hyp SEQ ID NO: 101
RC()NTATC()ATQRLAEFLHRSSNNF-Gly(Me)-Ile(Me)-LSSTNVGSNT-Hyp SEQ ID NO: 102
RC()NTATC()ATQRLAEFLHRSSNNF-Gly(Me)-A-Ile(Me)-LSSTNVGSNT-Aze SEQ ID NO: 103
RC()NTATC()ATQRLAEFLHRSSNNF-Gly(Me)-Ile(Me)-LSSTNVGSNT-Aze SEQ ID NO: 104
RC()NTATC()ATQRLAEFLHRSSNNF-Gly(Me)-A-Ile(Me)-LSSTNVGSNT-Pip SEQ ID NO: 105
RC()NTATC()ATQRLAEFLHRSSNNF-Gly(Me)-A-Ile(Me)-LSSTNVGSNT-Apr SEQ ID NO: 106
RC()NTATC()ATQRLAEFLHRSSNNF-Gly(Me)-Ile(Me)-LSSTNVGSNT-Apr SEQ ID NO: 107
RC()NTATC()ATQRLAEFLHRSSNNF-Gly(Me)-A-Ile(Me)-LSSTNVGSNT-Php SEQ ID NO: 108
RC()NTATC()ATQRLAEFLHRSSNNF-Gly(Me)-Ile(Me)-LSSTNVGSNT-Php SEQ ID NO: 109
RC()NTATC()ATQRLAEFLHRSSNNF-Gly(Me)-A-Ile(Me)-LSSTNVGSNT-Bep SEQ ID NO: 110
RC()NTATC()ATERLAEFLHRSSSSF-Gly(Me)-A-Ile(Me)-LSSTEVGSETP SEQ ID NO: 115
RC()NTATC()ATDRLAEFLHRSSSSF-Gly(Me)-A-Ile(Me)-LSSTEVGSETP SEQ ID NO: 116
WC()NTATC()ATQRLAEFLHRSSNNF-Gly(Me)-A-Ile(Me)-LSSTNVGSNTP SEQ ID NO: 121
RC()ETATC()ATQRLAEFLHRSSSSF-Gly(Me)-A-Ile(Me)-LSSTEVGSETP SEQ ID NO: 124
RC()PTATC()ATQRLAEFLHRSSSSF-Gly(Me)-A-Ile(Me)-LSSTEVGSETP SEQ ID NO: 125
RC()GTATC()ATQRLAEFLHRSSSSF-Gly(Me)-A-Ile(Me)-LSSTEVGSETP SEQ ID NO: 126
RC()ETATC()ATERLAEFLHRSSSSF-Gly(Me)-A-Ile(Me)-LSSTEVGSETP SEQ ID NO: 130
RC()PTATC()ATERLAEFLHRSSSSF-Gly(Me)-A-Ile(Me)-LSSTEVGSETP SEQ ID NO: 132
RC()ETATC()ATDRLAEFLHRSSSSF-Gly(Me)-A-Ile(Me)-LSSTEVGSETP SEQ ID NO: 146
RC()PTATC()ATDRLAEFLHRSSSSF-Gly(Me)-A-Ile(Me)-LSSTEVGSETP SEQ ID NO: 147
RC()NTATC()ATQRLAEFLHRSSNNF-Gly(Me)-Ile(Me)-LSSTNVGSNTP SEQ ID NO: 148
RC()NTATC()ATQRLAEFLHRSSNNF-Gly(Me)-A-Ile(Me)-LSSTNVGSNTY SEQ ID NO: 149
RC()NTATC()ATQRLAEFLHRSSNNF-Gly(Me)-Ile(Me)-LSSTNVGSNTY SEQ ID NO: 150
WC()GTATC()ATDRLAEFLHRSSF-Gly(Me)-A-Ile(Me)-LSSTDVGSDTP SEQ ID NO: 151
RC()GTATC()ATDRLAEFLHRSSF-Gly(Me)-A-Ile(Me)-LSSTDVGSDTP SEQ ID NO: 152
RC()NTATC()ATERLAEFLHRSSSSF-Gly(Me)-A-Ile(Me)-LSSTDVGSDTP SEQ ID NO: 154
WC()GTATC()ATERLAEFLHRSSF-Gly(Me)-A-Ile(Me)-LSSTEVGSETP SEQ ID NO: 155
RC()GTATC()ATERLAEFLHRSSF-Gly(Me)-A-lle(Me)-LSSTEVGSETP SEQ ID NO: 156
WC()GTATC()ATERLAEFLHRSSF-Gly(Me)-A-Ile(Me)-LSSTEVGSET-Hyp SEQ ID NO: 158
WC()GTATC()ATERLAEFLHRSSF-Gly(Me)-Ile(Me)-LSSTEVGSET-Hyp SEQ ID NO: 159
RC()GTATC()ATERLAEFLHRSSF-Gly(Me)-A-Ile(Me)-LSSTEVGSET-Hyp SEQ ID NO: 160
RC()GTATC()ATERLAEFLHRSSF-Gly(Me)-Ile(Me)-LSSTEVGSET-Hyp SEQ ID NO: 161
RC()ETATC()ATERLAEFLHRSSSSF-Gly(Me)-A-Ile(Me)-LSSTEVGSET-Hyp SEQ ID NO: 164
RC()ETATC()ATERLAEFLHRSSSSF-Gly(Me)-Ile(Me)-LSSTEVGSET-Hyp SEQ ID NO: 165
RC()PTATC()ATDRLAEFLHRSSF-Gly(Me)-A-Ile(Me)-LSSTDVGSDTP SEQ ID NO: 166
RC()PTATC()ATERLAEFLHRSSSSF-Gly(Me)-A-Ile(Me)-LSSTDVGSDTP SEQ ID NO: 167 and
RC()NTATC()ATQRLAEFLHHSSNNF-Gly(Me)-Ile(Me)-LPPTNVGSNTP (SEQ ID NO: 170)

**10.** A compound or pharmaceutically acceptable salt or solvate thereof according to any one of the preceding claims, wherein the half-life extending moiety M is an alkanoyl group, e.g. wherein the half-life extending moiety M is selected from 15-carboxy-pentadecanoyl, 17-carboxy-heptadecanoyl and 19-carboxy-nonadecanoyl.

**11.** A compound or pharmaceutically acceptable salt or solvate thereof according to any one of the preceding claims, comprising a linker moiety L, e.g. wherein the linker moiety L is a residue of an amino acid selected among Gly, Pro, Ala, Val, Leu, Ile, Met, Cys, Phe, Tyr, Trp, His, Lys, Arg, Gln, Asn, α-Glu, γ-Glu, ε-Lys, Asp, Ser, Thr, Gaba, Aib, β-Ala, 4-aminobutanoyl, 5-aminopentanoyl, 6-aminohexanoyl, 7-aminoheptanoyl, 8-aminooctanoyl, 9-aminononanoyl, 10-aminodecanoyl and 8Ado, for example wherein the linker moiety L is a γ-Glu residue.

**12.** A compound or pharmaceutically acceptable salt or solvate thereof according to any one of the preceding claims which is selected from the group consisting of:

| | |
|---|---|
| [19CD]-isoGlu-RC()NTATC()ATQRLAEFLHHSSNNF-Gly(Me)-A-Ile(Me)-LSSTNVGSNTP-$NH_2$ | Compd. 1 |
| [19CD]-isoGlu-RC()NTATC()ATQRLAEFLHHSSNNF-Gly(Me)-Ile(Me)-LSSTNVGSNTP-$NH_2$ | Compd. 2 |
| [19CD]-isoGlu-RC()NTATC()ATQRLAEFLHHSSNN-DPhe-Gly(Me)-A-Ile(Me)-LSSTNVGSNTP-$NH_2$ | Compd. 13 |
| [19CD]-isoGlu-RC()NTATC()ATQRLAEFLHRSSNNF-Gly(Me)-A-Ile(Me)-LSSTNVGSNTP-$NH_2$ | Compd. 18 |
| [19CD]-isoGlu-KC()NTATC()ATQRLAEFLHHSSNNF-Gly(Me)-A-Ile(Me)-LSSTNVGSNTP-$NH_2$ | Compd. 19 |
| [19CD]-isoGlu-KC()NTATC()ATQRLAEFLHHSSNNF-Gly(Me)-Ile(Me)-LSSTNVGSNTP-$NH_2$ | Compd. 20 |
| [19CD]-isoGlu-RC()NTATC()ATQRLAEFLHHSSNNF-Gly(Me)-A-Ile(Me)-L-DSer-STNVGSNTP-$NH_2$ | Compd. 23 |
| [19CD]-isoGlu-RC()NTATC()ATQRLAEFLHHSSNNF-Gly(Me)-Ile(Me)-L-DSer-STNVGSNTP-$NH_2$ | Compd. 24 |
| [19CD]-isoGlu-RC()NTATC()ATQRLAEFLHHSSNNF-Gly(Me)-A-Ile(Me)-LSTNVGSNTP-$NH_2$ | Compd. 25 |
| [19CD]-isoGlu-RC()NTATC()ATQRLAEFLHHSSNNF-Gly(Me)-Ile(Me)-LSTNVGSNTP-$NH_2$ | Compd. 26 |
| [19CD]-isoGlu-RC()NTATC()ATQRLAEFLHHSSNNF-Gly(Me)-ALSSTNVGSNTP-$NH_2$ | Compd. 27 |
| [19CD]-isoGlu-RC()NTATC()ATQRLAEFLHH-DSer-SNNF-Gly(Me)-A-Ile(Me)-LSSTNVGSNTP-$NH_2$ | Compd. 29 |
| [19CD]-isoGlu-RC()NTATC()ATQRLAEFLHH-DSer-SNNF-Gly(Me)-Ile(Me)-LSSTNVGSNTP-$NH_2$ | Compd. 30 |
| [19CD]-isoGlu-RC()NTATC()ATQRLAEFLHHSSNNF-Gly(Me)-A-Ile(Me)-LSSNVGSNTP-$NH_2$ | Compd. 37 |
| [19CD]-isoGlu-RC()NTATC()ATQRLAEFLHHSSNF-Gly(Me)-Ile(Me)-LSSTNVGSNTP-$NH_2$ | Compd. 38 |
| [19CD]-isoGlu-RC()NTATC()ATQRLAEFLHSSNNF-Gly(Me)-A-Ile(Me)-LSSTNVGSNTP-$NH_2$ | Compd. 39 |
| [19CD]-isoGlu-RC()NTATC()ATQRLAEFLHSSNNF-Gly(Me)-Ile(Me)-LSSTNVGSNTP-$NH_2$ | Compd. 40 |
| [19CD]-isoGlu-RC()NTATC()ATQRLAEFLSSNNF-Gly(Me)-A-Ile(Me)-LSSTNVGSNTP-$NH_2$ | Compd. 41 |
| [19CD]-isoGlu-RC()NTATC()ATQRLAEFLSSNNF-Gly(Me)-Ile(Me)-LSSTNVGSNTP-$NH_2$ | Compd. 42 |
| [19CD]-isoGlu-RC()NTATC()ATQRLAEFLHHSSNF-Gly(Me)-A-Ile(Me)-LSSTNVGSNTP-$NH_2$ | Compd. 44 |
| [19CD]-isoGlu-RC()NTATC()ATQRLAEFLHHSNNF-Gly(Me)-A-Ile(Me)-LSSTNVGSNTP-$NH_2$ | Compd. 45 |
| [19CD]-isoGlu-RC()NTATC()ATQRLAEFLHHSNNF-Gly(Me)-Ile(Me)-LSSTNVGSNTP-$NH_2$ | Compd. 46 |
| [19CD]-isoGlu-RC()NTATC()ATQRLAEFLHHNNF-Gly(Me)-A-Ile(Me)-LSSTNVGSNTP-$NH_2$ | Compd. 47 |
| [19CD]-isoGlu-RC()NTATC()ATQRLAEFLHHNNF-Gly(Me)-Ile(Me)-LSSTNVGSNTP-$NH_2$ | Compd. 48 |
| [19CD]-isoGlu-RC()NTATC()ATQRLAEFLHHSSNNFA-Ile(Me)-LSSTNVGSNTP-$NH_2$ | Compd. 49 |
| [19CD]-isoGlu-RC()NTATC()ATQRLAEFLHHSSF-Gly(Me)-A-Ile(Me)-LSSTNVGSNTP-$NH_2$ | Compd. 52 |
| [19CD]-isoGlu-RC()NTATC()ATQRLAEFLHHSSF-Gly(Me)-Ile(Me)-LSSTNVGSNTP-$NH_2$ | Compd. 53 |
| [19CD]-isoGlu-RC()NTATC()ATQRLAEFLHHSSNNF-Gly(Me)-A-Ile(Me)-SSTNVGSNTP-$NH_2$ | Compd. 54 |
| [19CD]-isoGlu-RC()NTATC()ATQRLAEFLHHSSNNF-Gly(Me)-Ile(Me)-SSTNVGSNTP-$NH_2$ | Compd. 55 |
| [19CD]-isoGlu-KC()NTATC()ATQRLAEFLHRSSNNF-Gly(Me)-A-Ile(Me)-LSSTNVGSNTP-$NH_2$ | Compd. 57 |
| [19CD]-isoGlu-KC()NTATC()ATQRLAEFLHRSSNNF-Gly(Me)-Ile(Me)-LSSTNVGSNTP-$NH_2$ | Compd. 58 |
| [19CD]-isoGlu-RC()NTATC()ATQRLAEFLHHSSNN-DPhe-Gly(Me)-A-Ile(Me)-L-DSer-STNVGSNTP-$NH_2$ | Compd. 59 |
| [19CD]-isoGlu-RC()NTATC()ATQRLAEFLHHSSNN-DPhe-Gly(Me)-Ile(Me)-L-DSer-STNVGSNTP-$NH_2$ | Compd. 60 |
| [19CD]-isoGlu-RC()NTATC()ATQRLAEFLHHSSNNF-Gly(Me)-A-Ile(Me)-LSSTNVGSNT-Tyr (Me)-$NH_2$ | Compd. 61 |

(continued)

| | |
|---|---|
| [19CD]-isoGlu-RC()NTATC()ATQRLAEFLHHSSNNF-Gly(Me)-Ile(Me)-LSSTNVGSNT-Tyr (Me)-$NH_2$ | Compd. 62 |
| [19CD]-isoGlu-KC()NTATC()ATQRLAEFLHRSSNNF-Gly(Me)-A-Ile(Me)-L-DSer-STNVGSNTP-$NH_2$ | Compd. 68 |
| [19CD]-isoGlu-KC()NTATC()ATQRLAEFLHRSSNNF-Gly(Me)-Ile(Me)-L-DSer-STNVGSNTP-$NH_2$ | Compd. 70 |
| [19CD]-isoGlu-KC()NTATC()ATQRLAEFLHRSSNN-DPhe-Gly(Me)-A-Ile(Me)-LSSTNVGSNTP-$NH_2$ | Compd. 71 |
| [19CD]-isoGlu-KC()NTATC()ATQRLAEFLHRSSNN-DPhe-Gly(Me)-A-Ile(Me)-L-DSer-STNVGSNTP-$NH_2$ | Compd. 72 |
| [19CD]-isoGlu-RC()NTATC()ATQRLAEFLHRSSSSF-Gly(Me)-A-Ile(Me)-LSSTDVGSDTP-$NH_2$ | Compd. 73 |
| [19CD]-isoGlu-RC()NTATC()ATQRLAEFLHRSSSSF-Gly(Me)-A-Ile(Me)-LSSTDVGSETP-$NH_2$ | Compd. 74 |
| [19CD]-isoGlu-RC()NTATC()ATQRLAEFLHRSSSSF-Gly(Me)-Ile(Me)-LSSTDVGSETP-$NH_2$ | Compd. 75 |
| [19CD]-isoGlu-RC()NTATC()ATQRLAEFLHRSSSSF-Gly(Me)-A-Ile(Me)-LSSTEVGSETP-$NH_2$ | Compd. 76 |
| [19CD]-isoGlu-RC()NTATC()ATQRLAEFLHRSSSSF-Gly(Me)-Ile(Me)-LSSTEVGSETP-$NH_2$ | Compd. 77 |
| [19CD]-isoGlu-RC()NTATC()ATQRLAEFLHRSSF-Gly(Me)-A-Ile(Me)-LSSTEVGSDTP-$NH_2$ | Compd. 78 |
| [19CD]-isoGlu-RC()NTATC()ATQRLAEFLHRSSF-Gly(Me)-Ile(Me)-LSSTEVGSDTP-$NH_2$ | Compd. 79 |
| [19CD]-isoGlu-RC()NTATC()ATQRLAEFLHRSSF-Gly(Me)-A-Ile(Me)-LSSTDVGSETP-$NH_2$ | Compd. 80 |
| [19CD]-isoGlu-RC()NTATC()ATQRLAEFLHRSSF-Gly(Me)-A-Ile(Me)-LSSTDVGSDTP-$NH_2$ | Compd. 81 |
| [19CD]-isoGlu-RC()NTATC()ATQRLAEFLHRSSF-Gly(Me)-A-Ile(Me)-LSSTEVGSETP-$NH_2$ | Compd. 82 |
| [17CD]-isoGlu-RC()NTATC()ATQRLAEFLHRSSNNF-Gly(Me)-A-Ile(Me)-LSSTNVGSNTP-$NH_2$ | Compd. 89 |
| [15CD]-isoGlu-RC()NTATC()ATQRLAEFLHRSSNNF-Gly(Me)-A-Ile(Me)-LSSTNVGSNTP-$NH_2$ | Compd. 90 |
| [Tetradecanoyl]-isoGlu-RC()NTATC()ATQRLAEFLHRSSNNF-Gly(Me)-A-Ile(Me)-LSSTNVGSNTP-$NH_2$ | Compd. 91 |
| [Hexadecanoyl]-isoGlu-RC()NTATC()ATQRLAEFLHRSSNNF-Gly(Me)-A-Ile(Me)-LSSTNVGSNTP-$NH_2$ | Compd. 92 |
| [Octadecanoyl]-isoGlu-RC()NTATC()ATQRLAEFLHRSSNNF-Gly(Me)-A-Ile(Me)-LSSTNVGSNTP-$NH_2$ | Compd. 93 |
| [Eicosanoyl]-isoGlu-RC()NTATC()ATQRLAEFLHRSSNNF-Gly(Me)-A-Ile(Me)-LSSTNVGSNTP-$NH_2$ | Compd. 94 |
| [19CD]-isoGlu-RC()NTATC()ATQRLAEFLHRSSF-Gly(Me)-Ile(Me)-LSSTEVGSETP-$NH_2$ | Compd. 95 |
| [19CD]-isoGlu-RC()NTATC()ATQRLAEFLHRSSNNF-Gly(Me)-A-Ile(Me)-LSSTNVGSNT-Hyp-$NH_2$ | Compd. 105 |
| [19CD]-isoGlu-RC()NTATC()ATQRLAEFLHRSSNNF-Gly(Me)-Ile(Me)-LSSTNVGSNT-Hyp-$NH_2$ | Compd. 106 |
| [19CD]-isoGlu-RC()NTATC()ATQRLAEFLHRSSNNF-Gly(Me)-A-Ile(Me)-LSSTNVGSNT-Aze-$NH_2$ | Compd. 107 |
| [19CD]-isoGlu-RC()NTATC()ATQRLAEFLHRSSNNF-Gly(Me)-Ile(Me)-LSSTNVGSNT-Aze-$NH_2$ | Compd. 108 |
| [19CD]-isoGlu-RC()NTATC()ATQRLAEFLHRSSNNF-Gly(Me)-A-Ile(Me)-LSSTNVGSNT-Pip-$NH_2$ | Compd. 109 |
| [19CD]-isoGlu-RC()NTATC()ATQRLAEFLHRSSNNF-Gly(Me)-A-Ile(Me)-LSSTNVGSNT-Apr-$NH_2$ | Compd. 110 |
| [19CD]-isoGlu-RC()NTATC()ATQRLAEFLHRSSNNF-Gly(Me)-Ile(Me)-LSSTNVGSNT-Apr-$NH_2$ | Compd. 111 |
| [19CD]-isoGlu-RC()NTATC()ATQRLAEFLHRSSNNF-Gly(Me)-A-Ile(Me)-LSSTNVGSNT-Php-$NH_2$ | Compd. 112 |
| [19CD]-isoGlu-RC()NTATC()ATQRLAEFLHRSSNNF-Gly(Me)-Ile(Me)-LSSTNVGSNT-Php-$NH_2$ | Compd. 113 |
| [19CD]-isoGlu-RC()NTATC()ATQRLAEFLHRSSNNF-Gly(Me)-A-Ile(Me)-LSSTNVGSNT-Bep-$NH_2$ | Compd. 114 |
| [19CD]-isoGlu-RC()NTATC()ATERLAEFLHRSSSSF-Gly(Me)-A-Ile(Me)-LSSTEVGSETP-$NH_2$ | Compd. 119 |
| [19CD]-isoGlu-RC()NTATC()ATDRLAEFLHRSSSSF-Gly(Me)-A-Ile(Me)-LSSTEVGSETP-$NH_2$ | Compd. 120 |
| [19CD]-isoGlu-WC()NTATC()ATQRLAEFLHRSSNNF-Gly(Me)-A-Ile(Me)-LSSTNVGSNTP-$NH_2$ | Compd. 125 |
| [19CD]-isoGlu-RC()ETATC()ATQRLAEFLHRSSSSF-Gly(Me)-A-Ile(Me)-LSSTEVGSETP-$NH_2$ | Compd. 128 |
| [19CD]-isoGlu-RC()PTATC()ATQRLAEFLHRSSSSF-Gly(Me)-A-Ile(Me)-LSSTEVGSETP-$NH_2$ | Compd. 129 |
| [19CD]-isoGlu-RC()GTATC()ATQRLAEFLHRSSSSF-Gly(Me)-A-Ile(Me)-LSSTEVGSETP-$NH_2$ | Compd. 130 |
| [19CD]-isoGlu-RC()ETATC()ATERLAEFLHRSSSSF-Gly(Me)-A-Ile(Me)-LSSTEVGSETP-$NH_2$ | Compd. 134 |
| [19CD]-isoGlu-RC()PTATC()ATERLAEFLHRSSSSF-Gly(Me)-A-Ile(Me)-LSSTEVGSETP-$NH_2$ | Compd. 136 |
| [19CD]-isoGlu-RC()ETATC()ATDRLAEFLHRSSSSF-Gly(Me)-A-Ile(Me)-LSSTEVGSETP-$NH_2$ | Compd. 150 |
| [19CD]-isoGlu-RC()PTATC()ATDRLAEFLHRSSSSF-Gly(Me)-A-Ile(Me)-LSSTEVGSETP-$NH_2$ | Compd. 151 |
| [19CD]-isoGlu-RC()NTATC()ATQRLAEFLHRSSNNF-Gly(Me)-A-Ile(Me)-LSSTNVGSNTP-NHMe | Compd. 152 |

(continued)

| | |
|---|---|
| [19CD]-isoGlu-RC()NTATC()ATQRLAEFLHRSSNNF-Gly(Me)-Ile(Me)-LSSTNVGSNTP-NHMe | Compd. 153 |
| [19CD]-isoGlu-RC()NTATC()ATQRLAEFLHRSSNNF-Gly(Me)-A-Ile(Me)-LSSTNVGSNTY-NHMe | Compd. 154 |
| [19CD]-isoGlu-RC()NTATC()ATQRLAEFLHRSSNNF-Gly(Me)-Ile(Me)-LSSTNVGSNTY-NHMe | Compd. 155 |
| [19CD]-isoGlu-RC()NTATC()ATQRLAEFLHRSSNNF-Gly(Me)-A-Ile(Me)-LSSTNVGSNTY-$NH_2$ | Compd. 156 |
| [19CD]-isoGlu-WC()GTATC()ATDRLAEFLHRSSF-Gly(Me)-A-Ile(Me)-LSSTDVGSDTP-$NH_2$ | Compd. 157 |
| [19CD]-isoGlu-RC()GTATC()ATDRLAEFLHRSSF-Gly(Me)-A-Ile(Me)-LSSTDVGSDTP-$NH_2$ | Compd. 158 |
| [19CD]-isoGlu-RC()NTATC()ATERLAEFLHRSSSSF-Gly(Me)-A-Ile(Me)-LSSTDVGSDTP-$NH_2$ | Compd. 160 |
| [19CD]-isoGlu-WC()GTATC()ATERLAEFLHRSSF-Gly(Me)-A-Ile(Me)-LSSTEVGSETP-$NH_2$ | Compd. 161 |
| [19CD]-isoGlu-RC()GTATC()ATERLAEFLHRSSF-Gly(Me)-A-Ile(Me)-LSSTEVGSETP-$NH_2$ | Compd. 162 |
| [19CD]-isoGlu-WC()GTATC()ATERLAEFLHRSSF-Gly(Me)-A-Ile(Me)-LSSTEVGSET-Hyp-$NH_2$ | Compd. 164 |
| [19CD]-isoGlu-WC()GTATC()ATERLAEFLHRSSF-Gly(Me)-Ile(Me)-LSSTEVGSET-Hyp-$NH_2$ | Compd. 165 |
| [19CD]-isoGlu-RC()GTATC()ATERLAEFLHRSSF-Gly(Me)-A-Ile(Me)-LSSTEVGSET-Hyp-$NH_2$ | Compd. 166 |
| [19CD]-isoGlu-RC()GTATC()ATERLAEFLHRSSF-Gly(Me)-Ile(Me)-LSSTEVGSET-Hyp-$NH_2$ | Compd. 167 |
| [19CD]-isoGlu-RC()ETATC()ATERLAEFLHRSSSSF-Gly(Me)-A-Ile(Me)-LSSTEVGSET-Hyp-$NH_2$ | Compd. 170 |
| [19CD]-isoGlu-RC()ETATC()ATERLAEFLHRSSSSF-Gly(Me)-Ile(Me)-LSSTEVGSET-Hyp-$NH_2$ | Compd. 171 |
| [19CD]-isoGlu-RC()PTATC()ATDRLAEFLHRSSF-Gly(Me)-A-Ile(Me)-LSSTDVGSDTP-$NH_2$ | Compd. 172 |
| [19CD]-isoGlu-RC()PTATC()ATERLAEFLHRSSSSF-Gly(Me)-A-Ile(Me)-LSSTDVGSDTP-$NH_2$ and | Compd. 173 |
| [19CD]-isoGlu-RC()NTATC()ATQRLAEFLHHSSNNF-Gly(Me)-Ile(Me)-LPPTNVGSNTP-$NH_2$ | Compd. 176 |

**13.** A method for the synthesis of a compound or pharmaceutically acceptable salt or solvate according to any one of the preceding claims comprising synthesizing the peptide by solid-phase or liquid-phase methodology, optionally isolating and purifying the final product, and further optionally comprising the step of forming a disulfide bond between the thiol groups of the cysteine side chains at positions 2 and 7.

**14.** A compound or pharmaceutically acceptable salt or solvate thereof according to any one of claims 1 to 12 for use in a method of medical treatment of a disease, condition or disorder selected from Alzheimer's disease, diabetes, type 1 diabetes, type 2 diabetes, pre-diabetes, insulin resistance syndrome, impaired glucose tolerance (IGT), diabetic heart (including diabetic cardiomyopathy and heart failure), metabolic syndrome, non-alcoholic steatohepatitis, hepatic steatosis, obesity, morbid obesity, morbid obesity prior to surgery, obesity-linked inflammation, obesity-linked gall bladder disease, obesity-induced sleep apnea and respiratory problems, degeneration of cartilage and osteoarthritis, and reproductive health complications such as infertility, wherein said compound or pharmaceutically acceptable salt or solvate is optionally administered in combination with an anti-diabetic agent, anti-obesity agent, anti-hypertension agent, anti-dyslipidemia agent, proton pump inhibitor or anti-inflammatory agent, e.g. wherein said anti-diabetic agent is metformin, a sulfonylurea, a glinide, a DPP-IV inhibitor, a glitazone, a GLP-1 or a GLP-1 analogue, an exendin-4 or an exendin-4 analogue, a glucagon-GLP-1 dual agonist or any other GLP-1 receptor agonist, an SGLT2 inhibitor, a GPR40 agonist, an insulin or an insulin analogue, optionally wherein the subject to be treated is a human.

**15.** A pharmaceutical composition comprising a compound or pharmaceutically acceptable salt or solvate thereof according to any one of claims 1 to 12, and a pharmaceutically acceptable carrier, excipient or vehicle.

## Patentansprüche

**1.** Verbindung der Formel I:

$$R^1\text{-}Z\text{-}R^2 \qquad (I)$$

worin

$R^1$ Wasserstoff, $C_{1-4}$-Acyl, Benzoyl oder $C_{1-4}$-Alkyl oder eine die Halbwertszeit verlängernde Gruppierung M ist, wobei M gegebenenfalls mit Z über eine Linkergruppe L verbunden ist;
$R^2$ OH oder $NHR^3$ ist, wobei $R^3$ Wasserstoff oder $C_{1-8}$-Alkyl ist; und
Z eine Aminosäuresequenz der Formel Id ist:

X1-Cys()-X3-Thr-Ala-Thr-Cys()-Ala-Thr-X10-Arg-Leu-Ala-X14-Phe-X16-X17-X18-X19-X20-X21-X22-X23-Gly(Me)-X25-Ile(Me)-X27-X28-X29-X30-X31-Val-Gly-Ser-X35-Thr-X37 (Id);

worin

X1 aus der aus Trp, Lys und Arg bestehenden Gruppe ausgewählt ist;
X3 aus der aus Gly, Asn, Glu und Pro bestehenden Gruppe ausgewählt ist;
X10 aus der aus Gln, Asp und Glu bestehenden Gruppe ausgewählt ist;
X14 aus der aus Asp und Glu bestehenden Gruppe ausgewählt ist;
X16 Leu ist oder fehlt;
X17 His ist oder fehlt;
X18 aus der aus His und Arg bestehenden Gruppe ausgewählt ist oder fehlt;
X19 aus der aus Ser und D-Ser bestehenden Gruppe ausgewählt ist oder fehlt;
X20 Ser ist oder fehlt;
X21 aus der aus Asn und Ser bestehenden Gruppe ausgewählt ist oder fehlt;
X22 aus der aus Asn und Ser bestehenden Gruppe ausgewählt ist oder fehlt;
X23 aus der aus Phe und D-Phe bestehenden Gruppe ausgewählt ist oder fehlt;
X25 Ala ist oder fehlt;
X27 Leu ist oder fehlt;
X28 aus der aus Ser, Ser(Me), D-Ser und D-Ser(Me) bestehenden Gruppe ausgewählt ist oder fehlt;
X29 aus der aus Ser und Ser(Me) bestehenden Gruppe ausgewählt ist oder fehlt;
X30 Thr ist oder fehlt;
X31 aus der aus Asp, Glu und Asn bestehenden Gruppe ausgewählt ist;
X35 aus der aus Asp, Glu und Asn bestehenden Gruppe ausgewählt ist;
X37 aus der aus Tyr, Tyr(Me), Pro, Hyp, Apr, Aze, Pip, Php und Bep bestehenden Gruppe ausgewählt ist;

wobei die Klammern () bei den beiden Cysteinresten (C, Cys) bei Sequenzposition 2 und 7 die Gegenwart einer intramolekularen Disulfidbrücke zwischen den beiden betreffenden Cys-Resten anzeigen;
wobei nicht mehr als drei Positionen fehlen, mit der Maßgabe, dass:

(i) wenn zwei Positionen fehlen, diese nebeneinander liegen oder eine der fehlenden Position X25 ist; und
(ii) wenn drei Positionen fehlen, zwei nebeneinander liegen und die andere X25 ist;

und wobei die Verbindung im Vergleich zu menschlichem Amylin an einem oder mehreren der Rezeptoren hCTR2, hAMRY1, hAMRY2 oder hAMRY3 verbesserte Wirksamkeit aufweist;
oder ein pharmazeutisch annehmbares Salz oder Solvat davon.

**2.** Verbindung oder pharmazeutisch annehmbares Salz oder Solvat nach Anspruch 1, wobei Z eine Aminosäuresequenz der Formel Ie ist:
X1-Cys()-X3-Thr-Ala-Thr-Cys()-Ala-Thr-X10-Arg-Leu-Ala-Glu-Phe-Leu-His-X18-Ser-Ser-X21-X22-X23-Gly(Me)-Ala-Ile(Me)-Leu-X28-Ser-Thr-X31-Val-Gly-Ser-X35-Thr-X37 (Ie); worin

X1 aus der aus Trp, Lys und Arg bestehenden Gruppe ausgewählt ist;
X3 aus der aus Gly, Asn und Pro bestehenden Gruppe ausgewählt ist;
X10 aus der aus Gln, Asp und Glu bestehenden Gruppe ausgewählt ist;
X18 aus der aus His und Arg bestehenden Gruppe ausgewählt ist;
X21 aus der aus Asn und Ser bestehenden Gruppe ausgewählt ist oder fehlt;
X22 aus der aus Asn und Ser bestehenden Gruppe ausgewählt ist oder fehlt;
X23 aus der aus Phe und D-Phe bestehenden Gruppe ausgewählt ist;
X28 aus der aus Ser und D-Ser bestehenden Gruppe ausgewählt ist;
X31 aus der aus Asp, Glu und Asn bestehenden Gruppe ausgewählt ist;
X35 aus der aus Asp, Glu und Asn bestehenden Gruppe ausgewählt ist; und
X37 aus der aus Pro und Hyp bestehenden Gruppe ausgewählt ist.

**3.** Verbindung oder pharmazeutisch annehmbares Salz oder Solvat davon nach einem der vorangegangenen Ansprüche, wobei:

(i) X3 aus Asn, Glu und Pro, z. B. Asn, ausgewählt ist; und/oder
(ii) X10 Gln ist.

**4.** Verbindung oder pharmazeutisch annehmbares Salz oder Solvat davon nach Anspruch 1 oder Anspruch 3, wenn er von diesem abhängt, wobei X14 Glu ist.

**5.** Verbindung oder pharmazeutisch annehmbares Salz oder Solvat davon nach einem der vorangegangenen Ansprüche, wobei:

(i) X23 D-Phe ist und/oder X28 D-Ser ist;
(ii) X21 und/oder X22 fehlen;
(ii) X21 und/oder X22 Asn sind; und/oder
(iii) X21 und/oder X22 Ser sind.

**6.** Verbindung oder pharmazeutisch annehmbares Salz oder Solvat davon nach Anspruch 1 oder nach einem der Ansprüche 3 bis 5, wenn sie von diesem abhängen, wobei:

(i) X19 und/oder X20 fehlen; oder
(ii) X19 und/oder X20 Ser sind.

**7.** Verbindung oder pharmazeutisch annehmbares Salz oder Solvat davon nach Anspruch 1 oder nach einem der Ansprüche 3 bis 6, wenn sie von diesem abhängen, wobei X25 fehlt.

**8.** Verbindung oder pharmazeutisch annehmbares Salz oder Solvat davon nach einem der vorangegangenen Ansprüche, wobei:

(i) X31 und/oder X35 Glu sind;
(ii) X31 und/oder X35 Asp sind; oder
(iii) X31 und/oder X35 Asn sind.

**9.** Verbindung oder pharmazeutisch annehmbares Salz oder Solvat davon nach einem der vorangegangenen Ansprüche, wobei Z eine aus folgender Gruppe ausgewählte Sequenz aufweist:

| | |
|---|---|
| RC()NTATC()ATQRLAEFLHHSSNNF-Gly(Me)-A-Ile(Me)-LSSTNVGSNTP | SEQ ID NO: 3 |
| RC()NTATC()ATQRLAEFLHHSSNNF-Gly(Me)-Ile(Me)-LSSTNVGSNTP | SEQ ID NO: 4 |
| RC()NTATC()ATQRLAEFLHHSSNN-DPhe-Gly(Me)-A-Ile(Me)-LSSTNVGSNTP | SEQ ID NO: 15 |
| RC()NTATC()ATQRLAEFLHRSSNNF-Gly(Me)-A-Ile(Me)-LSSTNVGSNTP | SEQ ID NO: 20 |
| KC()NTATC()ATQRLAEFLHHSSNNF-Gly(Me)-A-Ile(Me)-LSSTNVGSNTP | SEQ ID NO: 21 |
| KC()NTATC()ATQRLAEFLHHSSNNF-Gly(Me)-Ile(Me)-LSSTNVGSNTP | SEQ ID NO: 22 |
| RC()NTATC()ATQRLAEFLHHSSNNF-Gly(Me)-A-Ile(Me)-L-DSer-STNVGSNTP | SEQ ID NO: 25 |
| RC()NTATC()ATQRLAEFLHHSSNNF-Gly(Me)-Ile(Me)-L-DSer-STNVGSNTP | SEQ ID NO: 26 |
| RC()NTATC()ATQRLAEFLHHSSNNF-Gly(Me)-A-Ile(Me)-LSTNVGSNTP | SEQ ID NO: 27 |
| RC()NTATC()ATQRLAEFLHHS5NNF-Gly(Me)-Ile(Me)-LSTNVGSNTP | SEQ ID NO: 28 |
| RC()NTATC()ATQRLAEFLHH-DSer-SNNF-Gly(Me)-A-Ile(Me)-LSSTNVGSNTP | SEQ ID NO: 31 |
| RC()NTATC()ATQRLAEFLHH-DSer-SNNF-Gly(Me)-Ile(Me)-LSSTNVGSNTP | SEQ ID NO: 32 |
| RC()NTATC()ATQRLAEFLHHSSNNF-Gly(Me)-A-Ile(Me)-LSSNVGSNTP | SEQ ID NO: 39 |
| RC()NTATC()ATQRLAEFLHHSSNF-Gly(Me)-Ile(Me)-LSSTNVGSNTP | SEQ ID NO: 40 |
| RC()NTATC()ATQRLAEFLHSSNNF-Gly(Me)-A-Ile(Me)-LSSTNVGSNTP | SEQ ID NO: 41 |
| RC()NTATC()ATQRLAEFLHSSNNF-Gly(Me)-Ile(Me)-LSSTNVGSNTP | SEQ ID NO: 42 |
| RC()NTATC()ATQRLAEFLSSNNF-Gly(Me)-A-Ile(Me)-LSSTNVGSNTP | SEQ ID NO: 43 |
| RC()NTATC()ATQRLAEFLSSNNF-Gly(Me)-Ile(Me)-LSSTNVGSNTP | SEQ ID NO: 44 |
| RC()NTATC()ATQRLAEFLHHSSNF-Gly(Me)-A-Ile(Me)-LSSTNVGSNTP | SEQ ID NO: 46 |
| RC()NTATC()ATQRLAEFLHHSNNF-Gly(Me)-A-Ile(Me)-LSSTNVGSNTP | SEQ ID NO: 47 |
| RC()NTATC()ATQRLAEFLHHSNNF-Gly(Me)-Ile(Me)-LSSTNVGSNTP | SEQ ID NO: 48 |
| RC()NTATC()ATQRLAEFLHHNNF-Gly(Me)-A-Ile(Me)-LSSTNVGSNTP | SEQ ID NO: 49 |
| RC()NTATC()ATQRLAEFLHHNNF-Gly(Me)-Ile(Me)-LSSTNVGSNTP | SEQ ID NO: 50 |
| RC()NTATC()ATQRLAEFLHHSSF-Gly(Me)-A-Ile(Me)-LSSTNVGSNTP | SEQ ID NO: 54 |
| RC()NTATC()ATQRLAEFLHHSSF-Gly(Me)-Ile(Me)-LSSTNVGSNTP | SEQ ID NO: 55 |

RC()NTATC()ATQRLAEFLHHSSNNF-Gly(Me)-A-Ile(Me)-SSTNVGSNTP SEQ ID NO: 56
RC()NTATC()ATQRLAEFLHHSSNNF-Gly(Me)-Ile(Me)-SSTNVGSNTP SEQ ID NO: 57
KC()NTATC()ATQRLAEFLHRSSNNF-Gly(Me)-A-Ile(Me)-LSSTNVGSNTP SEQ ID NO: 59
KC()NTATC()ATQRLAEFLHRSSNNF-Gly(Me)-Ile(Me)-LSSTNVGSNTP SEQ ID NO: 60
RC()NTATC()ATQRLAEFLHHSSNN-DPhe-Gly(Me)-A-Ile(Me)-L-DSer-STNVGSNTP SEQ ID NO: 61
RC()NTATC()ATQRLAEFLHHSSNN-DPhe-Gly(Me)-Ile(Me)-L-DSer-STNVGSNTP SEQ ID NO: 62
RC()NTATC()ATQRLAEFLHHSSNNF-Gly(Me)-A-Ile(Me)-LSSTNVGSNT-Tyr(Me) SEQ ID NO: 63
RC()NTATC()ATQRLAEFLHHSSNNF-Gly(Me)-Ile(Me)-LSSTNVGSNT-Tyr(Me) SEQ ID NO: 64
KC()NTATC()ATQRLAEFLHRSSNNF-Gly(Me)-A-Ile(Me)-L-DSer-STNVGSNTP SEQ ID NO: 70
KC()NTATC()ATQRLAEFLHRSSNNF-Gly(Me)-Ile(Me)-L-DSer-STNVGSNTP SEQ ID NO: 72
KC()NTATC()ATQRLAEFLHRSSNN-DPhe-Gly(Me)-A-Ile(Me)-LSSTNVGSNTP SEQ ID NO: 73
KC()NTATC()ATQRLAEFLHRSSNN-DPhe-Gly(Me)-A-Ile(Me)-L-DSer-STNVGSNTP SEQ ID NO: 74
RC()NTATC()ATQRLAEFLHRSSSSF-Gly(Me)-A-Ile(Me)-LSSTDVGSDTP SEQ ID NO: 75
RC()NTATC()ATQRLAEFLHRSSSSF-Gly(Me)-A-Ile(Me)-LSSTDVGSETP SEQ ID NO: 76
RC()NTATC()ATQRLAEFLHRSSSSF-Gly(Me)-Ile(Me)-LSSTDVGSETP SEQ ID NO: 77
RC()NTATC()ATQRLAEFLHRSSSSF-Gly(Me)-A-Ile(Me)-LSSTEVGSETP SEQ ID NO: 78
RC()NTATC()ATQRLAEFLHRSSSSF-Gly(Me)-Ile(Me)-LSSTEVGSETP SEQ ID NO: 79
RC()NTATC()ATQRLAEFLHRSSF-Gly(Me)-A-Ile(Me)-LSSTEVGSDTP SEQ ID NO: 80
RC()NTATC()ATQRLAEFLHRSSF-Gly(Me)-Ile(Me)-LSSTEVGSDTP SEQ ID NO: 81
RC()NTATC()ATQRLAEFLHRSSF-Gly(Me)-A-Ile(Me)-LSSTDVGSETP SEQ ID NO: 82
RC()NTATC()ATQRLAEFLHRSSF-Gly(Me)-A-Ile(Me)-LSSTDVGSDTP SEQ ID NO: 83
RC()NTATC()ATQRLAEFLHRSSF-Gly(Me)-A-Ile(Me)-LSSTEVGSETP SEQ ID NO: 84
RC()NTATC()ATQRLAEFLHRSSF-Gly(Me)-Ile(Me)-LSSTEVGSETP SEQ ID NO: 91
RC()NTATC()ATQRLAEFLHRSSNNF-Gly(Me)-A-Ile(Me)-LSSTNVGSNT-DPro SEQ ID NO: 100
RC()NTATC()ATQRLAEFLHRSSNNF-Gly(Me)-A-Ile(Me)-LSSTNVGSNT-Hyp SEQ ID NO: 101
RC()NTATC()ATQRLAEFLHRSSNNF-Gly(Me)-Ile(Me)-LSSTNVGSNT-Hyp SEQ ID NO: 102
RC()NTATC()ATQRLAEFLHRSSNNF-Gly(Me)-A-Ile(Me)-LSSTNVGSNT-Aze SEQ ID NO: 103
RC()NTATC()ATQRLAEFLHRSSNNF-Gly(Me)-Ile(Me)-LSSTNVGSNT-Aze SEQ ID NO: 104
RC()NTATC()ATQRLAEFLHRSSNNF-Gly(Me)-A-Ile(Me)-LSSTNVGSNT-Pip SEQ ID NO: 105
RC()NTATC()ATQRLAEFLHRSSNNF-Gly(Me)-A-Ile(Me)-LSSTNVGSNT-Apr SEQ ID NO: 106
RC()NTATC()ATQRLAEFLHRSSNNF-Gly(Me)-Ile(Me)-LSSTNVGSNT-Apr SEQ ID NO: 107
RC()NTATC()ATQRLAEFLHRSSNNF-Gly(Me)-A-Ile(Me)-LSSTNVGSNT-Php SEQ ID NO: 108
RC()NTATC()ATQRLAEFLHRSSNNF-Gly(Me)-Ile(Me)-LSSTNVGSNT-Php SEQ ID NO: 109
RC()NTATC()ATQRLAEFLHRSSNNF-Gly(Me)-A-Ile(Me)-LSSTNVGSNT-Bep SEQ ID NO: 110
RC()NTATC()ATERLAEFLHRSSSSF-Gly(Me)-A-Ile(Me)-LSSTEVGSETP SEQ ID NO: 115
RC()NTATC()ATDRLAEFLHRSSSSF-Gly(Me)-A-Ile(Me)-LSSTEVGSETP SEQ ID NO: 116
WC()NTATC()ATQRLAEFLHRSSNNF-Gly(Me)-A-Ile(Me)-LSSTNVGSNTP SEQ ID NO: 121
RC()ETATC()ATQRLAEFLHRSSSSF-Gly(Me)-A-Ile(Me)-LSSTEVGSETP SEQ ID NO: 124
RC()PTATC()ATQRLAEFLHRSSSSF-Gly(Me)-A-Ile(Me)-LSSTEVGSETP SEQ ID NO: 125
RC()GTATC()ATQRLAEFLHRSSSSF-Gly(Me)-A-Ile(Me)-LSSTEVGSETP SEQ ID NO: 126
RC()ETATC()ATERLAEFLHRSSSSF-Gly(Me)-A-Ile(Me)-LSSTEVGSETP SEQ ID NO: 130
RC()PTATC()ATERLAEFLHRSSSSF-Gly(Me)-A-Ile(Me)-LSSTEVGSETP SEQ ID NO: 132
RC()ETATC()ATDRLAEFLHRSSSSF-Gly(Me)-A-Ile(Me)-LSSTEVGSETP SEQ ID NO: 146
RC()PTATC()ATDRLAEFLHRSSSSF-Gly(Me)-A-Ile(Me)-LSSTEVGSETP SEQ ID NO: 147
RC()NTATC()ATQRLAEFLHRSSNNF-Gly(Me)-Ile(Me)-LSSTNVGSNTP SEQ ID NO: 148
RC()NTATC()ATQRLAEFLHRSSNNF-Gly(Me)-A-Ile(Me)-LSSTNVGSNTY SEQ ID NO: 149
RC()NTATC()ATQRLAEFLHRSSNNF-Gly(Me)-Ile(Me)-LSSTNVGSNTY SEQ ID NO: 150
WC()GTATC()ATDRLAEFLHRSSF-Gly(Me)-A-Ile(Me)-LSSTDVGSDTP SEQ ID NO: 151
RC()GTATC()ATDRLAEFLHRSSF-Gly(Me)-A-Ile(Me)-LSSTDVGSDTP SEQ ID NO: 152
RC()NTATC()ATERLAEFLHRSSSSF-Gly(Me)-A-Ile(Me)-LSSTDVGSDTP SEQ ID NO: 154
WC()GTATC()ATERLAEFLHRSSF-Gly(Me)-A-Ile(Me)-LSSTEVGSETP SEQ ID NO: 155
RC()GTATC()ATERLAEFLHRSSF-Gly(Me)-A-Ile(Me)-LSSTEVGSETP SEQ ID NO: 156
WC()GTATC()ATERLAEFLHRSSF-Gly(Me)-A-Ile(Me)-LSSTEVGSET-Hyp SEQ ID NO: 158
WC()GTATC()ATERLAEFLHRSSF-Gly(Me)-Ile(Me)-LSSTEVGSET-Hyp SEQ ID NO: 159
RC()GTATC()ATERLAEFLHRSSF-Gly(Me)-A-Ile(Me)-LSSTEVGSET-Hyp SEQ ID NO: 160
RC()GTATC()ATERLAEFLHRSSF-Gly(Me)-Ile(Me)-LSSTEVGSET-Hyp SEQ ID NO: 161
RC()ETATC()ATERLAEFLHRSSSSF-Gly(Me)-A-Ile(Me)-LSSTEVGSET-Hyp SEQ ID NO: 164
RC()ETATC()ATERLAEFLHRSSSSF-Gly(Me)-Ile(Me)-LSSTEVGSET-Hyp SEQ ID NO: 165

RC()PTATC()ATDRLAEFLHRSSF-Gly(Me)-A-Ile(Me)-LSSTDVGSDTP SEQ ID NO: 166
RC()PTATC()ATERLAEFLHRSSSSF-Gly(Me)-A-Ile(Me)-LSSTDVGSDTP SEQ ID NO: 167 und
RC()NTATC()ATQRLAEFLHHSSNNF-Gly(Me)-Ile(Me)-LPPTNVGSNTP (SEQ ID NO: 170)

**10.** Verbindung oder pharmazeutisch annehmbares Salz oder Solvat davon nach einem der vorangegangenen Ansprüche, wobei die die Halbwertszeit verlängernde Gruppierung M eine Alkanoylgruppe ist, wobei die die Halbwertszeit verlängernde Gruppierung M beispielsweise aus 15-Carboxypentadecanoyl, 17-Carboxyheptadecanoyl und 19-Carboxynonadecanoyl ausgewählt ist.

**11.** Verbindung oder pharmazeutisch annehmbares Salz oder Solvat davon nach einem der vorangegangenen Ansprüche, die eine Linkergruppe L umfasst, wobei die Linkergruppe L z. B. ein Rest einer Aminosäure ist, die aus Gly, Pro, Ala, Val, Leu, Ile, Met, Cys, Phe, Tyr, Trp, His, Lys, Arg, Gln, Asn, α-Glu, γ-Glu, ε-Lys, Asp, Ser, Thr, Gaba, Aib, β-Ala, 4-Aminobutanoyl, 5-Aminopentanoyl, 6-Aminohexanoyl, 7-Aminoheptanoyl, 8-Aminooctanoyl, 9-Aminononanoyl, 10-Aminodecanoyl und 8Ado ausgewählt ist, wobei die Linkergruppe L beispielsweise ein γ-Glu-Rest ist.

**12.** Verbindung oder pharmazeutisch annehmbares Salz oder Solvat davon nach einem der vorangegangenen Ansprüche, ausgewählt aus folgender Gruppe:

| | |
|---|---|
| [19CD]-isoGlu-RC()NTATC()ATQRLAEFLHHSSNNF-Gly(Me)-A-Ile(Me)-LSSTNVGSNTP-$NH_2$ | Verbind. 1 |
| [19CD]-isoGlu-RC()NTATC()ATQRLAEFLHHSSNNF-Gly(Me)-Ile(Me)-LSSTNVGSNTP-$NH_2$ | Verbind. 2 |
| [19CD]-isoGlu-RC()NTATC()ATQRLAEFLHHSSNN-DPhe-Gly(Me)-A-Ile(Me)-LSSTNVGSNTP-$NH_2$ | Verbind. 13 |
| [19CD]-isoGlu-RC()NTATC()ATQRLAEFLHRSSNNF-Gly(Me)-A-Ile(Me)-LSSTNVGSNTP-$NH_2$ | Verbind. 18 |
| [19CD]-isoGlu-KC()NTATC()ATG7RLAEFLHHSSNNF-Gly(Me)-A-Ile(Me)-LSSTNVGSNTP-$NH_2$ | Verbind. 19 |
| [19CD]-isoGlu-KC()NTATC()ATQRLAEFLHHSSNNF-Gly(Me)-Ile(Me)-LSSTNVGSNTP-$NH_2$ | Verbind. 20 |
| [19CD]-isoGlu-RC()NTATC()ATQRLAEFLHHSSNNF-Gly(Me)-A-Ile(Me)-L-DSer-STNVGSNTP-$NH_2$ | Verbind. 23 |
| [19CD]-isoGlu-RC()NTATC()ATQRLAEFLHHSSNNF-Gly(Me)-Ile(Me)-L-DSer-STNVGSNTP-$NH_2$ | Verbind. 24 |
| [19CD]-isoGlu-RC()NTATC()ATQRLAEFLHHSSNNF-Gly(Me)-A-Ile(Me)-LSTNVGSNTP-$NH_2$ | Verbind. 25 |
| [19CD]-isoGlu-RC()NTATC()ATQRLAEFLHHSSNNF-Gly(Me)-Ile(Me)-LSTNVGSNTP-$NH_2$ | Verbind. 26 |
| [19CD]-isoGlu-RC()NTATC()ATQRLAEFLHHSSNNF-Gly(Me)-ALSSTNVGSNTP-$NH_2$ | Verbind. 27 |
| [19CD]-isoGlu-RC()NTATC()ATQRLAEFLHH-DSer-SNNF-Gly(Me)-A-Ile(Me)-LSSTNVGSNTP-$NH_2$ | Verbind. 29 |
| [19CD]-isoGlu-RC()NTATC()ATQRLAEFLHH-DSer-SNNF-Gly(Me)-Ile(Me)-LSSTNVGSNTP-$NH_2$ | Verbind. 30 |
| [19CD]-isoGlu-RC()NTATC()ATQRLAEFLHHSSNNF-Gly(Me)-A-Ile(Me)-LSSNVGSNTP-$NH_2$ | Verbind. 37 |
| [19CD]-isoGlu-RC()NTATC()ATQRLAEFLHHSSNF-Gly(Me)-Ile(Me)-LSSTNVGSNTP-$NH_2$ | Verbind. 38 |
| [19CD]-isoGlu-RC()NTATC()ATQRLAEFLHSSNNF-Gly(Me)-A-Ile(Me)-LSSTNVGSNTP-$NH_2$ | Verbind. 39 |
| [19CD]-isoGlu-RC()NTATC()ATQRLAEFLHSSNNF-Gly(Me)-Ile(Me)-LSSTNVGSNTP-$NH_2$ | Verbind. 40 |
| [19CD]-isoGlu-RC()NTATC()ATQRLAEFLSSNNF-Gly(Me)-A-Ile(Me)-LSSTNVGSNTP-$NH_2$ | Verbind. 41 |
| [19CD]-isoGlu-RC()NTATC()ATQRLAEFLSSNNF-Gly(Me)-Ile(Me)-LSSTNVGSNTP-$NH_2$ | Verbind. 42 |
| [19CD]-isoGlu-RC()NTATC()ATQRLAEFLHHSSNF-Gly(Me)-A-Ile(Me)-LSSTNVGSNTP-$NH_2$ | Verbind. 44 |
| [19CD]-isoGlu-RC()NTATC()ATQRLAEFLHHSNNF-Gly(Me)-A-Ile(Me)-LSSTNVGSNTP-$NH_2$ | Verbind. 45 |
| [19CD]-isoGlu-RC()NTATC()ATQRLAEFLHHSNNF-Gly(Me)-Ile(Me)-LSSTNVGSNTP-$NH_2$ | Verbind. 46 |
| [19CD]-isoGlu-RC()NTATC()ATQRLAEFLHHNNF-Gly(Me)-A-Ile(Me)-LSSTNVGSNTP-$NH_2$ | Verbind. 47 |
| [19CD]-isoGlu-RC()NTATC()ATQRLAEFLHHNNF-Gly(Me)-Ile(Me)-LSSTNVGSNTP-$NH_2$ | Verbind. 48 |
| [19CD]-isoGlu-RC()NTATC()ATQRLAEFLHHSSNNFA-Ile(Me)-LSSTNVGSNTP-$NH_2$ | Verbind. 49 |
| [19CD]-isoGlu-RC()NTATC()ATQRLAEFLHHSSF-Gly(Me)-A-Ile(Me)-LSSTNVGSNTP-$NH_2$ | Verbind. 52 |
| [19CD]-isoGlu-RC()NTATC()ATQRLAEFLHHSSF-Gly(Me)-Ile(Me)-LSSTNVGSNTP-$NH_2$ | Verbind. 53 |
| [19CD]-isoGlu-RC()NTATC()ATQRLAEFLHHSSNNF-Gly(Me)-A-Ile(Me)-SSTNVGSNTP-$NH_2$ | Verbind. 54 |
| [19CD]-isoGlu-RC()NTATC()ATQRLAEFLHHSSNNF-Gly(Me)-Ile(Me)-SSTNVGSNTP-$NH_2$ | Verbind. 55 |
| [19CD]-isoGlu-KC()NTATC()ATQRLAEFLHRSSNNF-Gly(Me)-A-Ile(Me)-LSSTNVGSNTP-$NH_2$ | Verbind. 57 |
| [19CD]-isoGlu-KC()NTATC()ATQRLAEFLHRSSNNF-Gly(Me)-Ile(Me)-LSSTNVGSNTP-$NH_2$ | Verbind. 58 |

(fortgesetzt)

| | |
|---|---|
| [19CD]-isoGlu-RC()NTATC()ATQRLAEFLHHSSNN-DPhe-Gly(Me)-A-Ile(Me)-L-DSer-STNVGSNTP-$NH_2$ | Verbind. 59 |
| [19CD]-isoGlu-RC()NTATC()ATQRLAEFLHHSSNN-DPhe-Gly(Me)-Ile(Me)-L-DSer-STNVGSNTP-$NH_2$ | Verbind. 60 |
| [19CD]-isoGlu-RC()NTATC()ATQRLAEFLHHSSNNF-Gly(Me)-A-Ile(Me)-LSSTNVGSNT-Tyr (Me)-$NH_2$ | Verbind. 61 |
| [19CD]-isoGlu-RC()NTATC()ATQRLAEFLHHSSNNF-Gly(Me)-Ile(Me)-LSSTNVGSNT-Tyr (Me)-$NH_2$ | Verbind. 62 |
| [19CD]-isoGlu-KC()NTATC()ATQRLAEFLHRSSNNF-Gly(Me)-A-Ile(Me)-L-DSer-STNVGSNTP-$NH_2$ | Verbind. 68 |
| [19CD]-isoGlu-KC()NTATC()ATQRLAEFLHRSSNNF-Gly(Me)-Ile(Me)-L-DSer-STNVGSNTP-$NH_2$ | Verbind. 70 |
| [19CD]-isoGlu-KC()NTATC()ATQRLAEFLHRSSNN-DPhe-Gly(Me)-A-Ile(Me)-LSSTNVGSNTP-$NH_2$ | Verbind.71 |
| [19CD]-isoGlu-KC()NTATC()ATQRLAEFLHRSSNN-DPhe-Gly(Me)-A-Ile(Me)-L-DSer-STNVGSNTP-$NH_2$ | Verbind.72 |
| [19CD]-isoGlu-RC()NTATC()ATQRLAEFLHRSSSSF-Gly(Me)-A-Ile(Me)-LSSTDVGSDTP-$NH_2$ | Verbind. 73 |
| [19CD]-isoGlu-RC()NTATC()ATQRLAEFLHRSSSSF-Gly(Me)-A-Ile(Me)-LSSTDVGSETP-$NH_2$ | Verbind. 74 |
| [19CD]-isoGlu-RC()NTATC()ATQRLAEFLHRSSSSF-Gly(Me)-Ile(Me)-LSSTDVGSETP-$NH_2$ | Verbind. 75 |
| [19CD]-isoGlu-RC()NTATC()ATQRLAEFLHRSSSSF-Gly(Me)-A-Ile(Me)-LSSTEVGSETP-$NH_2$ | Verbind. 76 |
| [19CD]-isoGlu-RC()NTATC()ATQRLAEFLHRSSSSF-Gly(Me)-Ile(Me)-LSSTEVGSETP-$NH_2$ | Verbind. 77 |
| [19CD]-isoGlu-RC()NTATC()ATQRLAEFLHRSSF-Gly(Me)-A-Ile(Me)-LSSTEVGSDTP-$NH_2$ | Verbind. 78 |
| [19CD]-isoGlu-RC()NTATC()ATQRLAEFLHRSSF-Gly(Me)-Ile(Me)-LSSTEVGSDTP-$NH_2$ | Verbind. 79 |
| [19CD]-isoGlu-RC()NTATC()ATQRLAEFLHRSSF-Gly(Me)-A-Ile(Me)-LSSTDVGSETP-$NH_2$ | Verbind. 80 |
| [19CD]-isoGlu-RC()NTATC()ATQRLAEFLHRSSF-Gly(Me)-A-Ile(Me)-LSSTDVGSDTP-$NH_2$ | Verbind. 81 |
| [19CD]-isoGlu-RC()NTATC()ATQRLAEFLHRSSF-Gly(Me)-A-Ile(Me)-LSSTEVGSETP-$NH_2$ | Verbind. 82 |
| [17CD]-isoGlu-RC()NTATC()ATQRLAEFLHRSSNNF-Gly(Me)-A-Ile(Me)-LSSTNVGSNTP-$NH_2$ | Verbind. 89 |
| [15CD]-isoGlu-RC()NTATC()ATQRLAEFLHRSSNNF-Gly(Me)-A-Ile(Me)-LSSTNVGSNTP-$NH_2$ | Verbind. 90 |
| [Tetradecanoyl]-isoGlu-RC()NTATC()ATQRLAEFLHRSSNNF-Gly(Me)-A-Ile (Me)-LSSTNVGSNTP-$NH_2$ | Verbind. 91 |
| [Hexadecanoyl]-isoGlu-RC()NTATC()ATQRLAEFLHRSSNNF-Gly(Me)-A-Ile (Me)-LSSTNVGSNTP-$NH_2$ | Verbind. 92 |
| [Octadecanoyl]-isoGlu-RC()NTATC()ATQRLAEFLHRSSNNF-Gly(Me)-A-Ile(Me)-LSSTNVGSNTP-$NH_2$ | Verbind. 93 |
| [Eicosanoyl]-isoGlu-RC()NTATC()ATQRLAEFLHRSSNNF-Gly(Me)-A-Ile(Me)-LSSTNVGSNTP-$NH_2$ | Verbind. 94 |
| [19CD]-isoGlu-RC()NTATC()ATQRLAEFLHRSSF-Gly(Me)-Ile(Me)-LSSTEVGSETP-$NH_2$ | Verbind. 95 |
| [19CD]-isoGlu-RC()NTATC()ATQRLAEFLHRSSNNF-Gly(Me)-A-Ile(Me)-LSSTNVGSNT-Hyp-$NH_2$ | Verbind. 105 |
| [19CD]-isoGlu-RC()NTATC()ATQRLAEFLHRSSNNF-Gly(Me)-Ile(Me)-LSSTNVGSNT-Hyp-$NH_2$ | Verbind. 106 |
| [19CD]-isoGlu-RC()NTATC()ATQRLAEFLHRSSNNF-Gly(Me)-A-Ile(Me)-LSSTNVGSNT-Aze-$NH_2$ | Verbind. 107 |
| [19CD]-isoGlu-RC()NTATC()ATQRLAEFLHRSSNNF-Gly(Me)-Ile(Me)-LSSTNVGSNT-Aze-$NH_2$ | Verbind. 108 |
| [19CD]-isoGlu-RC()NTATC()ATQRLAEFLHRSSNNF-Gly(Me)-A-Ile(Me)-LSSTNVGSNT-Pip-$NH_2$ | Verbind. 109 |
| [19CD]-isoGlu-RC()NTATC()ATQRLAEFLHRSSNNF-Gly(Me)-A-Ile(Me)-LSSTNVGSNT-Apr-$NH_2$ | Verbind. 110 |
| [19CD]-isoGlu-RC()NTATC()ATQRLAEFLHRSSNNF-Gly(Me)-Ile(Me)-LSSTNVGSNT-Apr-$NH_2$ | Verbind. 111 |
| [19CD]-isoGlu-RC()NTATC()ATQRLAEFLHRSSNNF-Gly(Me)-A-Ile(Me)-LSSTNVGSNT-Php-$NH_2$ | Verbind. 112 |
| [19CD]-isoGlu-RC()NTATC()ATQRLAEFLHRSSNNF-Gly(Me)-Ile(Me)-LSSTNVGSNT-Php-$NH_2$ | Verbind. 113 |
| [19CD]-isoGlu-RC()NTATC()ATQRLAEFLHRSSNNF-Gly(Me)-A-Ile(Me)-LSSTNVGSNT-Bep-$NH_2$ | Verbind. 114 |
| [19CD]-isoGlu-RC()NTATC()ATERLAEFLHRSSSSF-Gly(Me)-A-Ile(Me)-LSSTEVGSETP-$NH_2$ | Verbind. 119 |
| [19CD]-isoGlu-RC()NTATC()ATDRLAEFLHRSSSSF-Gly(Me)-A-Ile(Me)-LSSTEVGSETP-$NH_2$ | Verbind. 120 |
| [19CD]-isoGlu-WC()NTATC()ATQRLAEFLHRSSNNF-Gly(Me)-A-Ile(Me)-LSSTNVGSNTP-$NH_2$ | Verbind. 125 |
| [19CD]-isoGlu-RC()ETATC()ATQRLAEFLHRSSSSF-Gly(Me)-A-Ile(Me)-LSSTEVGSETP-$NH_2$ | Verbind. 128 |
| [19CD]-isoGlu-RC()PTATC()ATQRLAEFLHRSSSSF-Gly(Me)-A-Ile(Me)-LSSTEVGSETP-$NH_2$ | Verbind. 129 |
| [19CD]-isoGlu-RC()GTATC()ATQRLAEFLHRSSSSF-Gly(Me)-A-Ile(Me)-LSSTEVGSETP-$NH_2$ | Verbind. 130 |

(fortgesetzt)

| | |
|---|---|
| [19CD]-isoGlu-RC()ETATC()ATERLAEFLHRSSSSF-Gly(Me)-A-Ile(Me)-LSSTEVGSETP-$NH_2$ | Verbind. 134 |
| [19CD]-isoGlu-RC()PTATC()ATERLAEFLHRSSSSF-Gly(Me)-A-Ile(Me)-LSSTEVGSETP-$NH_2$ | Verbind. 136 |
| [19CD]-isoGlu-RC()ETATC()ATDRLAEFLHRSSSSF-Gly(Me)-A-Ile(Me)-LSSTEVGSETP-$NH_2$ | Verbind. 150 |
| [19CD]-isoGlu-RC()PTATC()ATDRLAEFLHRSSSSF-Gly(Me)-A-Ile(Me)-LSSTEVGSETP-$NH_2$ | Verbind. 151 |
| [19CD]-isoGlu-RC()NTATC()ATQRLAEFLHRSSNNF-Gly(Me)-A-Ile(Me)-LSSTNVGSNTP-NHMe | Verbind. 152 |
| [19CD]-isoGlu-RC()NTATC()ATQRLAEFLHRSSNNF-Gly(Me)-Ile(Me)-LSSTNVGSNTP-NHMe | Verbind. 153 |
| [19cD]-isoGlu-RC()NTATC()ATQRLAEFLHRSSNNF-Gly(Me)-A-Ile(Me)-LSSTNVGSNTY-NHMe | Verbind. 154 |
| [19CD]-isoGlu-RC()NTATC()ATQRLAEFLHRSSNNF-Gly(Me)-Ile(Me)-LSSTNVGSNTY-NHMe | Verbind. 155 |
| [19CD]-isoGlu-RC()NTATC()ATQRLAEFLHRSSNNF-Gly(Me)-A-Ile(Me)-LSSTNVGSNTY-$NH_2$ | Verbind. 156 |
| [19CD]-isoGlu-WC()GTATC()ATDRLAEFLHRSSF-Gly(Me)-A-Ile(Me)-LSSTDVGSDTP-$NH_2$ | Verbind. 157 |
| [19CD]-isoGlu-RC()GTATC()ATDRLAEFLHRSSF-Gly(Me)-A-Ile(Me)-LSSTDVGSDTP-$NH_2$ | Verbind. 158 |
| [19CD]-isoGlu-RC()NTATC()ATERLAEFLHRSSSSF-Gly(Me)-A-Ile(Me)-LSSTDVGSDTP-$NH_2$ | Verbind. 160 |
| [19CD]-isoGlu-WC()GTATC()ATERLAEFLHRSSF-Gly(Me)-A-Ile(Me)-LSSTEVGSETP-$NH_2$ | Verbind. 161 |
| [19CD]-isoGlu-RC()GTATC()ATERLAEFLHRSSF-Gly(Me)-A-Ile(Me)-LSSTEVGSETP-$NH_2$ | Verbind. 162 |
| [19CD]-isoGlu-WC()GTATC()ATERLAEFLHRSSF-Gly(Me)-A-Ile(Me)-LSSTEVGSET-Hyp-$NH_2$ | Verbind. 164 |
| [19CD]-isoGlu-WC()GTATC()ATERLAEFLHRSSF-Gly(Me)-Ile(Me)-LSSTEVGSET-Hyp-$NH_2$ | Verbind. 165 |
| [19CD]-isoGlu-RC()GTATC()ATERLAEFLHRSSF-Gly(Me)-A-Ile(Me)-LSSTEVGSET-Hyp-$NH_2$ | Verbind. 166 |
| [19CD]-isoGlu-RC()GTATC()ATERLAEFLHRSSF-Gly(Me)-Ile(Me)-LSSTEVGSET-Hyp-$NH_2$ | Verbind. 167 |
| [19CD]-isoGlu-RC()ETATC()ATERLAEFLHRSSSSF-Gly(Me)-A-Ile(Me)-LSSTEVGSET-Hyp-$NH_2$ | Verbind. 170 |
| [19CD]-isoGlu-RC()ETATC()ATERLAEFLHRSSSSF-Gly(Me)-Ile(Me)-LSSTEVGSET-Hyp-$NH_2$ | Verbind. 171 |
| [19CD]-isoGlu-RC()PTATC()ATDRLAEFLHRSSF-Gly(Me)-A-Ile(Me)-LSSTDVGSDTP-$NH_2$ | Verbind. 172 |
| [19CD]-isoGlu-RC()PTATC()ATERLAEFLHRSSSSF-Gly(Me)-A-Ile(Me)-LSSTDVGSDTP-$NH_2$ | Verbind. 173 |
| und | |
| [19CD]-isoGlu-RC()NTATC()ATQRLAEFLHHSSNNF-Gly(Me)-Ile(Me)-LPPTNVGSNTP-$NH_2$ | Verbind. 176 |

**13.** Verfahren zur Synthese einer Verbindung oder eines pharmazeutisch annehmbaren Salzes oder Solvats nach einem der vorangegangenen Ansprüche, das die Synthese des Peptids durch eine Festphasen- oder Flüssigphasenmethode, gegebenenfalls das Isolieren und Reinigen des Endprodukts und weiters gegebenenfalls den Schritt des Bildens einer Disulfidbindung zwischen den Thiolgruppen der Cysteinseitenketten an Position 2 und 7 umfasst.

**14.** Verbindung oder pharmazeutisch annehmbares Salz oder Solvat davon nach einem der Ansprüche 1 bis 12 zur Verwendung in einem medizinischen Behandlungsverfahren für eine Erkrankung, ein Leiden oder eine Störung, die/das aus Alzheimer-Krankheit, Diabetes, Diabetes Typ 1, Diabetes Typ 2, Prädiabetes, Insulinresistenzsyndrom, gestörter Glucosetoleranz (IGT), diabetischem Herz (einschließlich diabetischer Kardiomyopathie und Herzversagen), metabolischem Syndrom, nichtalkoholischer Steatohepatitis, hepatischer Steatose, Adipositas, morbider Adipositas, morbider Adipositas vor einer Operation, mit Adipositas zusammenhängender Entzündung, mit Adipositas zusammenhängender Gallenblasenerkrankung, durch Adipositas induzierter Schlafapnoe und Atemproblemen, Knorpeldegeneration und Osteoarthritis sowie reproduktiven Gesundheitskomplikationen wie Unfruchtbarkeit ausgewählt ist, wobei die Verbindung oder das pharmazeutisch annehmbare Salz oder Solvat davon gegebenenfalls in Kombination mit einem Antidiabetesmittel, Antiadipositasmittel, Antihypertoniemittel, Antidyslipidämiemittel, Protonenpumpeninhibitor oder Antiphlogistikum verabreicht wird, wobei das Antidiabetesmittel z. B. Metformin, ein Sulfonylharnstoff, ein Glinid, ein DPP-IV-Inhibitor, ein Glitazon, ein GLP-1 oder ein GLP-1-Analogon, ein Exendin-4 oder ein Exendin-4-Analogon, ein Glucagon-GLP-1-Dualagonist oder ein beliebiger anderer GLP-1-Rezeptoragonist, ein SGLT2-Inhibitor, ein GPR40-Agonist, Insulin oder ein Insulinanalogon ist, wobei das zu behandelnde Individuum gegebenenfalls ein Mensch ist.

**15.** Pharmazeutische Zusammensetzung, die eine Verbindung oder ein pharmazeutisch annehmbares Salz oder Solvat davon nach einem der Ansprüche 1 bis 12 und einen/ein pharmazeutisch annehmbaren/s Träger, Exzipienten oder Vehikel umfasst.

## Revendications

**1.** Composé de formule I :

$R^1$-Z-$R^2$ (I)

dans laquelle

$R^1$ est un hydrogène, acyle en $C_1$ à $C_4$, benzoyle ou alkyle en $C_1$ à $C_4$, ou un fragment M prolongeant la demi-vie, lequel M est éventuellement lié à Z via un fragment lieur L ;
$R^2$ est OH ou $NHR^3$ où $R^3$ est un hydrogène ou alkyle en $C_1$ à $C_3$ ; et
Z est une séquence d'acides aminés de formule Id :

X1-Cys()-X3-Thr-Ala-Thr-Cys()-Ala-Thr-X10-Arg-Leu-Ala-X14-Phe-X16-X17-X18-X19-X20-X21-X22-X23-Gly(Me)-X25-Ile(Me)-X27-X28-X29-X30-X31-Val-Gly-Ser-X35-Thr-X37 (Id)

dans laquelle

X1 est choisi dans le groupe constitué par Trp, Lys et Arg ;
X3 est choisi dans le groupe constitué par Gly, Asn, Glu et Pro ;
X10 est choisi dans le groupe constitué par Gln, Asp et Glu ;
X14 est choisi dans le groupe constitué par Asp et Glu ;
X16 est Leu ou est absent ;
X17 est His ou est absent ;
X18 est choisi dans le groupe constitué par His et Arg, ou est absent ;
X19 est choisi dans le groupe constitué par Ser et D-Ser, ou est absent ;
X20 est Ser ou est absent ;
X21 est choisi dans le groupe constitué par Asn et Ser, ou est absent ;
X22 est choisi dans le groupe constitué par Asn et Ser, ou est absent ;
X23 est choisi dans le groupe constitué par Phe et D-Phe, ou est absent ;
X25 est Ala ou est absent ;
X27 est Leu ou est absent ;
X28 est choisi dans le groupe constitué par Ser, Ser(Me), D-Ser et D-Ser(Me), ou est absent ;
X29 est choisi dans le groupe constitué par Ser et Ser(Me) ou est absent ;
X30 est Thr ou est absent ;
X31 est choisi dans le groupe constitué par Asp, Glu et Asn ;
X35 est choisi dans le groupe constitué par Asp, Glu et Asn ; et
X37 est choisi dans le groupe constitué par Tyr, Tyr(Me), Pro, Hyp, Apr, Aze, Pip, Php et Bep ;

et dans laquelle les parenthèses associées aux deux résidus de cystéine (C, Cys) aux positions de séquence 2 et 7 indiquent la présence d'un pont disulfure intramoléculaire entre les deux résidus Cys en question ;
dans lequel au plus trois positions peuvent être absentes, avec les conditions suivantes :

(i) si deux positions sont absentes, elles sont adjacentes l'une de l'autre ou bien l'une des positions absente est X25 ; et
(ii) si trois positions sont absentes, deux sont adjacentes l'une à l'autre et la dernière est X25 ;

et lequel composé a une puissance améliorée par rapport à l'amyline humaine au niveau d'un ou plusieurs des récepteurs hCTR2, hAMRY1, hAMRY2 et hAMRY3 ;
ou un sel ou solvate pharmaceutiquement acceptable de celui-ci.

**2.** Composé ou sel ou solvate pharmaceutiquement acceptable de celui-ci selon la revendication 1, dans lequel Z est une séquence d'acides aminés de formule Ie :

X1-Cys()-X3-Thr-Ala-Thr-Cys()-Ala-Thr-X10-Arg-Leu-Ala-Glu-Phe-Leu-His-X18-Ser-Ser-X21-X22-X23-Gly(Me)-Ala-Ile(Me)-Leu-X28-Ser-Thr-X31-Val-Gly-Ser-X35-Thr-X37 (Ie)

dans laquelle

X1 est choisi dans le groupe constitué par Trp, Lys et Arg ;
X3 est choisi dans le groupe constitué par Gly, Asn et Pro ;
X10 est choisi dans le groupe constitué par Gln, Asp et Glu ;
X18 est choisi dans le groupe constitué par His et Arg ;
X21 est choisi dans le groupe constitué par Asn et Ser, ou est absent ;
X22 est choisi dans le groupe constitué par Asn et Ser, ou est absent ;
X23 est choisi dans le groupe constitué par Phe et D-Phe ;
X28 est choisi dans le groupe constitué par Ser et D-Ser ;
X31 est choisi dans le groupe constitué par Asp, Glu et Asn ;
X35 est choisi dans le groupe constitué par Asp, Glu et Asn ; et
X37 est choisi dans le groupe constitué par Pro et Hyp.

**3.** Composé ou sel ou solvate pharmaceutiquement acceptable de celui-ci selon l'une quelconque des revendications précédentes, dans lequel :

(i) X3 est choisi parmi Asn, Gly et Pro, par exemple Asn ; et/ou
(ii) X10 est Gln.

**4.** Composé ou sel ou solvate pharmaceutiquement acceptable de celui-ci selon la revendication 1 ou la revendication 3 lorsqu'elle en dépend, dans lequel X14 est Glu.

**5.** Composé ou sel ou solvate pharmaceutiquement acceptable de celui-ci selon l'une quelconque des revendications précédentes, dans lequel :

(i) X23 est D-Phe et/ou X28 est D-Ser ;
(ii) X21 et/ou X22 sont absents ;
(iii) X21 et/ou X22 sont Asn ; et/ou
(iv) X21 et/ou X22 sont Ser.

**6.** Composé ou sel ou solvate pharmaceutiquement acceptable de celui-ci selon la revendication 1 ou l'une quelconque des revendications 3 à 5 lorsqu'elles en dépendent, dans lequel :

(i) X19 et/ou X20 sont absents ; ou
(ii) X19 et/ou X20 sont Ser.

**7.** Composé ou sel ou solvate pharmaceutiquement acceptable de celui-ci selon la revendication 1 ou l'une quelconque des revendications 3 à 6 lorsqu'elles en dépendent, dans lequel X25 est absent.

**8.** Composé ou sel ou solvate pharmaceutiquement acceptable de celui-ci selon l'une quelconque des revendications précédentes, dans lequel :

(i) X31 et/ou X35 sont Glu ;
(ii) X31 et/ou X35 sont Asp ; ou
(iii) X31 et/ou X35 sont Asn.

**9.** Composé ou sel ou solvate pharmaceutiquement acceptable de celui-ci selon l'une quelconque des revendications précédentes, dans lequel Z a une séquence choisie dans le groupe constitué par :

RC()NTATC()ATQRLAEFLHHSSNNF-Gly(Me)-A-Ile(Me)-LSSTNVGSNTP SEQ ID NO: 3
RC()NTATC()ATQRLAEFLHHSSNNF-Gly(Me)-Ile(Me)-LSSTNVGSNTP SEQ ID NO: 4

RC()NTATC()ATQRLAEFLHHSSNN-DPhe-Gly(Me)-A-Ile(Me)-LSSTNVGSNTP SEQ ID NO: 15
RC()NTATC()ATQRLAEFLHRSSNNF-Gly(Me)-A-Ile(Me)-LSSTNVGSNTP SEQ ID NO: 20
KC()NTATC()ATQRLAEFLHHSSNNF-Gly(Me)-A-Ile(Me)-LSSTNVGSNTP SEQ ID NO: 21
KC()NTATC()ATQRLAEFLHHSSNNF-Gly(Me)-Ile(Me)-LSSTNVGSNTP SEQ ID NO: 22
RC()NTATC()ATQRLAEFLHHSSNNF-Gly(Me)-A-Ile(Me)-L-DSer-STNVGSNTP SEQ ID NO: 25
RC()NTATC()ATQRLAEFLHHSSNNF-Gly(Me)-Ile(Me)-L-DSer-STNVGSNTP SEQ ID NO: 26
RC()NTATC()ATQRLAEFLHHSSNNF-Gly(Me)-A-Ile(Me)-LSTNVGSNTP SEQ ID NO: 27
RC()NTATC()ATQRLAEFLHHSSNNF-Gly(Me)-Ile(Me)-LSTNVGSNTP SEQ ID NO: 28
RC()NTATC()ATQRLAEFLHH-DSer-SNNF-Gly(Me)-A-Ile(Me)-LSSTNVGSNTP SEQ ID NO: 31
RC()NTATC()ATQRLAEFLHH-DSer-SNNF-Gly(Me)-Ile(Me)-LSSTNVGSNTP SEQ ID NO: 32
RC()NTATC()ATQRLAEFLHHSSNNF-Gly(Me)-A-Ile(Me)-LSSNVGSNTP SEQ ID NO: 39
RC()NTATC()ATQRLAEFLHHSSNF-Gly(Me)-Ile(Me)-LSSTNVGSNTP SEQ ID NO: 40
RC()NTATC()ATQRLAEFLHSSNNF-Gly(Me)-A-Ile(Me)-LSSTNVGSNTP SEQ ID NO: 41
RC()NTATC()ATQRLAEFLHSSNNF-Gly(Me)-Ile(Me)-LSSTNVGSNTP SEQ ID NO: 42
RC()NTATC()ATQRLAEFLSSNNF-Gly(Me)-A-Ile(Me)-LSSTNVGSNTP SEQ ID NO: 43
RC()NTATC()ATQRLAEFLSSNNF-Gly(Me)-Ile(Me)-LSSTNVGSNTP SEQ ID NO: 44
RC()NTATC()ATQRLAEFLHHSSNF-Gly(Me)-A-Ile(Me)-LSSTNVGSNTP SEQ ID NO: 46
RC()NTATC()ATQRLAEFLHHSNNF-Gly(Me)-A-Ile(Me)-LSSTNVGSNTP SEQ ID NO: 47
RC()NTATC()ATQRLAEFLHHSNNF-Gly(Me)-Ile(Me)-LSSTNVGSNTP SEQ ID NO: 48
RC()NTATC()ATQRLAEFLHHNNF-Gly(Me)-A-Ile(Me)-LSSTNVGSNTP SEQ ID NO: 49
RC()NTATC()ATQRLAEFLHHNNF-Gly(Me)-Ile(Me)-LSSTNVGSNTP SEQ ID NO: 50
RC()NTATC()ATQRLAEFLHHSSF-Gly(Me)-A-Ile(Me)-LSSTNVGSNTP SEQ ID NO: 54
RC()NTATC()ATQRLAEFLHHSSF-Gly(Me)-Ile(Me)-LSSTNVGSNTP SEQ ID NO: 55
RC()NTATC()ATQRLAEFLHHSSNNF-Gly(Me)-A-Ile(Me)-SSTNVGSNTP SEQ ID NO: 56
RC()NTATC()ATQRLAEFLHHSSNNF-Gly(Me)-Ile(Me)-SSTNVGSNTP SEQ ID NO: 57
KC()NTATC()ATQRLAEFLHRSSNNF-Gly(Me)-A-Ile(Me)-LSSTNVGSNTP SEQ ID NO: 59
KC()NTATC()ATQRLAEFLHRSSNNF-Gly(Me)-Ile(Me)-LSSTNVGSNTP SEQ ID NO: 60
RC()NTATC()ATQRLAEFLHHSSNN-DPhe-Gly(Me)-A-Ile(Me)-L-DSer-STNVGSNTP SEQ ID NO: 61
RC()NTATC()ATQRLAEFLHHSSNN-DPhe-Gly(Me)-Ile(Me)-L-DSer-STNVGSNTP SEQ ID NO: 62
RC()NTATC()ATQRLAEFLHHSSNNF-Gly(Me)-A-Ile(Me)-LSSTNVGSNT-Tyr(Me) SEQ ID NO: 63
RC()NTATC()ATQRLAEFLHHSSNNF-Gly(Me)-Ile(Me)-LSSTNVGSNT-Tyr(Me) SEQ ID NO: 64
KC()NTATC()ATQRLAEFLHRSSNNF-Gly(Me)-A-Ile(Me)-L-DSer-STNVGSNTP SEQ ID NO: 70
KC()NTATC()ATQRLAEFLHRSSNNF-Gly(Me)-Ile(Me)-L-DSer-STNVGSNTP SEQ ID NO: 72
KC()NTATC()ATQRLAEFLHRSSNN-DPhe-Gly(Me)-A-Ile(Me)-LSSTNVGSNTP SEQ ID NO: 73
KC()NTATC()ATQRLAEFLHRSSNN-DPhe-Gly(Me)-A-Ile(Me)-L-DSer-STNVGSNTP SEQ ID NO: 74
RC()NTATC()ATQRLAEFLHRSSSSF-Gly(Me)-A-Ile(Me)-LSSTDVGSDTP SEQ ID NO: 75
RC()NTATC()ATQRLAEFLHRSSSSF-Gly(Me)-A-Ile(Me)-LSSTDVGSETP SEQ ID NO: 76
RC()NTATC()ATQRLAEFLHRSSSSF-Gly(Me)-Ile(Me)-LSSTDVGSETP SEQ ID NO: 77
RC()NTATC()ATQRLAEFLHRSSSSF-Gly(Me)-A-Ile(Me)-LSSTEVGSETP SEQ ID NO: 78
RC()NTATC()ATQRLAEFLHRSSSSF-Gly(Me)-Ile(Me)-LSSTEVGSETP SEQ ID NO: 79
RC()NTATC()ATQRLAEFLHRSSF-Gly(Me)-A-Ile(Me)-LSSTEVGSDTP SEQ ID NO: 80
RC()NTATC()ATQRLAEFLHRSSF-Gly(Me)-Ile(Me)-LSSTEVGSDTP SEQ ID NO: 81
RC()NTATC()ATQRLAEFLHRSSF-Gly(Me)-A-Ile(Me)-LSSTDVGSETP SEQ ID NO: 82
RC()NTATC()ATQRLAEFLHRSSF-Gly(Me)-A-Ile(Me)-LSSTDVGSDTP SEQ ID NO: 83
RC()NTATC()ATQRLAEFLHRSSF-Gly(Me)-A-Ile(Me)-LSSTEVGSETP SEQ ID NO: 84
RC()NTATC()ATQRLAEFLHRSSF-Gly(Me)-Ile(Me)-LSSTEVGSETP SEQ ID NO: 91
RC()NTATC()ATQRLAEFLHRSSNNF-Gly(Me)-A-Ile(Me)-LSSTNVGSNT-DPro SEQ ID NO: 100
RC()NTATC()ATQRLAEFLHRSSNNF-Gly(Me)-A-Ile(Me)-LSSTNVGSNT-Hyp SEQ ID NO: 101
RC()NTATC()ATQRLAEFLHRSSNNF-Gly(Me)-Ile(Me)-LSSTNVGSNT-Hyp SEQ ID NO: 102
RC()NTATC()ATQRLAEFLHRSSNNF-Gly(Me)-A-Ile(Me)-LSSTNVGSNT-Aze SEQ ID NO: 103
RC()NTATC()ATQRLAEFLHRSSNNF-Gly(Me)-Ile(Me)-LSSTNVGSNT-Aze SEQ ID NO: 104
RC()NTATC()ATQRLAEFLHRSSNNF-Gly(Me)-A-Ile(Me)-LSSTNVGSNT-Pip SEQ ID NO: 105
RC()NTATC()ATQRLAEFLHRSSNNF-Gly(Me)-A-Ile(Me)-LSSTNVGSNT-Apr SEQ ID NO: 106
RC()NTATC()ATQRLAEFLHRSSNNF-Gly(Me)-Ile(Me)-LSSTNVGSNT-Apr SEQ ID NO: 107
RC()NTATC()ATQRLAEFLHRSSNNF-Gly(Me)-A-Ile(Me)-LSSTNVGSNT-Php SEQ ID NO: 108
RC()NTATCOATQRLAEFLHRSSNNF-Gly(Me)-Ile(Me)-LSSTNVGSNT-Php SEQ ID NO: 109
RC()NTATC()ATGRIAEFLHRSSNNF-Gly(Me)-A-Ile(Me)-LSSTNVGSNT-Bep SEQ ID NO: 110
RC()NTATC()ATERLAEFLHRSSSSF-Gly(Me)-A-Ile(Me)-LSSTEVGSETP SEQ ID NO: 115

RC()NTATC()ATDRLAEFLHRSSSSF-Gly(Me)-A-Ile(Me)-LSSTEVGSETP SEQ ID NO: 116
WG()NTATC()ATQRLAEFLHRSSNNF-Gly(Me)-A-Ile(Me)-LSSTNVGSNTP SEQ ID NO: 121
RC()ETATC()ATQRLAEFLHRSSSSF-Gly(Me)-A-Ile(Me)-LSSTEVGSETP SEQ ID NO: 124
RC()PTATC()ATQRLAEFLHRSSSSF-Gly(Me)-A-Ile(Me)-LSSTEVGSETP SEQ ID NO: 125
RC()GTATC()ATQRLAEFLHRSSSSF-Gly(Me)-A-Ile(Me)-LSSTEVGSETP SEQ ID NO: 126
RC()ETATC()ATERLAEFLHRSSSSF-Gly(Me)-A-Ile(Me)-LSSTEVGSETP SEQ ID NO: 130
RC()PTATC()ATERLAEFLHRSSSSF-Gly(Me)-A-Ile(Me)-LSSTEVGSETP SEQ ID NO: 132
RC()ETATC()ATDRLAEFLHRSSSSF-Gly(Me)-A-Ile(Me)-LSSTEVGSETP SEQ ID NO: 146
RC()PTATC()ATDRLAEFLHRSSSSF-Gly(Me)-A-Ile(Me)-LSSTEVGSETP SEQ ID NO: 147
RC()NTATC()ATQRLAEFLHRSSNNF-Gly(Me)-Ile(Me)-LSSTNVGSNTP SEQ ID NO: 148
RC()NTATC()ATQRLAEFLHRSSNNF-Gly(Me)-A-Ile(Me)-LSSTNVGSNTY SEQ ID NO: 149
RC()NTATC()ATQRLAEFLHRSSNNF-Gly(Me)-Ile(Me)-LSSTNVGSNTV SEQ ID NO: 150
WC()GTATC()ATDRLAEFLHRSSF-Gly(Me)-A-Ile(Me)-LSSTDVGSDTP SEQ ID NO: 151
RC()GTATC()ATDRLAEFLHRSSF-Gly(Me)-A-Ile(Me)-LSSTDVGSDTP SEQ ID NO: 152
RC()NTATC()ATERLAEFLHRSSSSF-Gly(Me)-A-Ile(Me)-LSSTDVGSDTP SEQ ID NO: 154
WC()GTATC()ATERLAEFLHRSSF-Gly(Me)-A-Ile(Me)-LSSTEVGSETP SEQ ID NO: 155
RC()GTATC()ATERLAEFLHRSSF-Gly(Me)-A-Ile(Me)-LSSTEVGSETP SEQ ID NO: 156
WC()GTATC()ATERLAEFLHRSSF-Gly(Me)-A-Ile(Me)-LSSTEVGSET-Hyp SEQ ID NO: 158
WC()GTATC()ATERLAEFLHRSSF-Gly(Me)-Ile(Me)-LSSTEVGSET-Hyp SEQ ID NO: 159
RC()GTATC()ATERLAEFLHRSSF-Gly(Me)-A-Ile(Me)-LSSTEVGSET-Hyp SEQ ID NO: 160
RC()GTATC()ATERLAEFLHRSSF-Gly(Me)-Ile(Me)-LSSTEVGSET-Hyp SEQ ID NO: 161
RC()ETATC()ATERU\EFLHRSSSSF-Gly(Me)-A-Ile(Me)-LSSTEVGSET-Hyp SEQ ID NO: 164
RC()ETATC()ATERLAEFLHRSSSSF-Gly(Me)-Ile(Me)-LSSTEVGSET-Hyp SEQ ID NO: 165
RC()PTATC()ATDRLAEFLHRSSF-Gly(Me)-A-Ile(Me)-LSSTDVGSDTP SEQ ID NO: 166
RC()PTATC()ATERLAEFLHRSSSSF-Gly(Me)-A-Ile(Me)-LSSTDVGSDTP SEQ ID NO: 167 et
RC()NTATC()ATQRLAEFLHHSSNNF-Gly(Me)-Ile(Me)-LPPTNVGSNTP (SEQ ID NO: 170)

**10.** Composé ou sel ou solvate pharmaceutiquement acceptable de celui-ci selon l'une quelconque des revendications précédentes, dans lequel le fragment prolongeant la demi-vie M est un groupe alcanoyle, par exemple dans lequel le fragment prolongeant la demi-vie M est choisi parmi 15-carboxy-pentadécanoyle, 17-carboxy-heptadécanoyle et 19-carboxy-nonadécanoyle.

**11.** Composé ou sel ou solvate pharmaceutiquement acceptable de celui-ci selon l'une quelconque des revendications précédentes, comprenant un fragment lieur L, par exemple dans lequel le fragment lieur L est un résidu d'un acide aminé choisi parmi Gly, Pro, Ala, Val, Leu, Ile, Met, Cys, Phe, Tyr, Trp, His, Lys, Arg, Gln, Asm, α-Glu, γ-Glu, ε-Lys, Asp, Ser, Thr, Gaba, Aib, β-Ala, 4-aminobutanoyle, 5-aminopentanoyle, 6-aminohexanoyle, 7-aminoheptanoyle, 8-aminooctanoyle, 9-aminononanoyle, 10-aminodécanoyle et 8Ado, par exemple dans lequel le fragment lieur L est un résidu de γ-Glu.

**12.** Composé ou sel ou solvate pharmaceutiquement acceptable de celui-ci selon l'une quelconque des revendications précédentes, qui est choisi dans le groupe constitué par :

| | |
|---|---|
| [19CD]-isoGlu-RC()NTATC()ATQRLAEFLHHSSNNF-Gly(Me)-A-Ile(Me)-LSSTNVGSNTP-$NH_2$ | Compd. 1 |
| [19CD]-isoGlu-RC()NTATC()ATQRLAEFLHHSSNNF-Gly(Me)-Ile(Me)-LSSTNVGSNTP-$NH_2$ | Compd. 2 |
| [19CD]-isoGlu-RC()NTATC()ATQRLAEFLHHSSNN-DPhe-Gly(Me)-A-Ile(Me)-LSSTNVGSNTP-$NH_2$ | Compd. 13 |
| [19CD]-isoGlu-RC()NTATC()ATQRLAEFLHRSSNNF-Gly(Me)-A-Ile(Me)-LSSTNVGSNTP-$NH_2$ | Compd. 18 |
| [19CD]-isoGlu-KCQNTATC()ATQRLAEFLHHSSNNF-Gly(Me)-A-Ile(Me)-LSSTNVGSNTP-$NH_2$ | Compd. 19 |
| [19CD]-isoGlu-KC()NTATC()ATQRLAEFLHHSSNNF-Gly(Me)-Ile(Me)-LSSTNVGSNTP-$NH_2$ | Compd. 20 |
| [19CD]-isoGlu-RC()NTATC()ATQRLAEFLHHSSNNF-Gly(Me)-A-Ile(Me)-L-DSer-STNVGSNTP-$NH_2$ | Compd. 23 |
| [19CD]-isoGlu-RCQNTATC()ATQRLAEFLHHSSNNF-Gly(Me)-Ile(Me)-L-DSer-STNVGSNTP-$NH_2$ | Compd. 24 |
| [19CD]-isoGlu-RC()NTATC()ATQRLAEFLHHSSNNF-Gly(Me)-A-Ile(Me)-LSTNVGSNTP-$NH_2$ | Compd. 25 |
| [19CD]-isoGlu-RC()NTATC()ATQRLAEFLHHSSNNF-Gly(Me)-Ile(Me)-LSTNVGSNTP-$NH_2$ | Compd. 26 |
| [19CD]-isoGlu-RC()NTATC()ATQRLAEFLHHSSNNF-Gly(Me)-ALSSTNVGSNTP-$NH_2$ | Compd. 27 |

(suite)

| | |
|---|---|
| [19CD]-isoGlu-RC()NTATC()ATQRLAEFLHH-DSer-SNNF-Gly(Me)-A-Ile(Me)-LSSTNVGSNTP-$NH_2$ | Compd. 29 |
| [19CD]-isoGlu-RC()NTATC()ATQRLAEFLHH-DSer-SNNF-Gly(Me)-Ile(Me)-LSSTNVGSNTP-$NH_2$ | Compd. 30 |
| [19CD]-isoGlu-RC()NTATC()ATQRLAEFLHHSSNNF-Gly(Me)-A-Ile(Me)-LSSNVGSNTP-$NH_2$ | Compd. 37 |
| [19CD]-isoGlu-RC()NTATC()ATQRLAEFLHHSSNF-Gly(Me)-Ile(Me)-LSSTNVGSNTP-$NH_2$ | Compd. 38 |
| [19CD]-isoGlu-RC()NTATC()ATQRLAEFLHSSNNF-Gly(Me)-A-Ile(Me)-LSSTNVGSNTP-$NH_2$ | Compd. 39 |
| [19CD]-isoGlu-RC()NTATC()ATQRLAEFLHSSNNF-Gly(Me)-Ile(Me)-LSSTNVGSNTP-$NH_2$ | Compd. 40 |
| [19CD]-isoGlu-RC()NTATC()ATQRLAEFLSSNNF-Gly(Me)-A-Ile(Me)-LSSTNVGSNTP-$NH_2$ | Compd. 41 |
| [19CD]-isoGlu-RC()NTATC()ATQRLAEFLSSNNF-Gly(Me)-Ile(Me)-LSSTNVGSNTP-$NH_2$ | Compd. 42 |
| [19CD]-isoGlu-RC()NTATC()ATQRLAEFLHHSSNF-Gly(Me)-A-Ile(Me)-LSSTNVGSNTP-$NH_2$ | Compd. 44 |
| [19CD]-isoGlu-RC()NTATC()ATQRLAEFLHHSNNF-Gly(Me)-A-Ile(Me)-LSSTNVGSNTP-$NH_2$ | Compd. 45 |
| [19CD]-isoGlu-RC()NTATC()ATQRLAEFLHHSNNF-Gly(Me)-Ile(Me)-LSSTNVGSNTP-$NH_2$ | Compd. 46 |
| [19CD]-isoGlu-RC()NTATC()ATQRLAEFLHHNNF-Gly(Me)-A-Ile(Me)-LSSTNVGSNTP-$NH_2$ | Compd. 47 |
| [19CD]-isoGlu-RC()NTATC()ATQRLAEFLHHNNF-Gly(Me)-Ile(Me)-LSSTNVGSNTP-$NH_2$ | Compd. 48 |
| [19CD]-isoGlu-RC()NTATC()ATQRLAEFLHHSSNNFA-Ile(Me)-LSSTNVGSNTP-$NH_2$ | Compd. 49 |
| [19CD]-isoGlu-RC()NTATC()ATQRLAEFLHHSSF-Gly(Me)-A-Ile(Me)-LSSTNVGSNTP-$NH_2$ | Compd. 52 |
| [19CD]-isoGlu-RC()NTATC()ATQRLAEFLHHSSF-Gly(Me)-Ile(Me)-LSSTNVGSNTP-$NH_2$ | Compd. 53 |
| [19CD]-isoGlu-RC()NTATC()ATQRLAEFLHHSSNNF-Gly(Me)-A-Ile(Me)-SSTNVGSNTP-$NH_2$ | Compd. 54 |
| [19CD]-isoGlu-RC()NTATC()ATQRLAEFLHHSSNNF-Gly(Me)-Ile(Me)-SSTNVGSNTP-$NH_2$ | Compd. 55 |
| [19CD]-isoGlu-KC()NTATC()ATQRLAEFLHRSSNNF-Gly(Me)-A-Ile(Me)-LSSTNVGSNTP-$NH_2$ | Compd. 57 |
| [19CD]-isoGlu-KC()NTATC()ATQRLAEFLHRSSNNF-Gly(Me)-Ile(Me)-LSSTNVGSNTP-$NH_2$ | Compd. 58 |
| [19CD]-isoGlu-RC()NTATC()ATQRLAEFLHHSSNN-DPhe-Gly(Me)-A-Ile(Me)-L-DSer-STNVGSNTP-$NH_2$ | Compd. 59 |
| [19CD]-isoGlu-RC()NTATC()ATQRLAEFLHHSSNN-DPhe-Gly(Me)-Ile(Me)-L-DSer-STNVGSNTP-$NH_2$ | Compd. 60 |
| [19CD]-isoGlu-RC()NTATG()ATQRLAEFLHHSSNNF-Gly(Me)-A-Ile(Me)-LSSTNVGSNT-Tyr (Me)-$NH_2$ | Compd. 61 |
| [19CD]-isoGlu-RC()NTATCQATQRLAEFLHHSSNNF-Gly(Me)-Ile(Me)-LSSTNVGSNT-Tyr (Me)-$NH_2$ | Compd. 62 |
| [19CD]-isoGlu-KC()NTATC()ATQRLAEFLHRSSNNF-Gly(Me)-A-Ile(Me)-L-DSer-STNVGSNTP-$NH_2$ | Compd. 68 |
| [19CD]-isoGlu-KC()NTATC()ATQRLAEFLHRSSNNF-Gly(Me)-Ile(Me)-L-DSer-STNVGSNTP-$NH_2$ | Compd. 70 |
| [19CD]-isoGlu-KC()NTATC()ATQRLAEFLHRSSNN-DPhe-Gly(Me)-A-Ile(Me)-LSSTNVGSNTP-$NH_2$ | Compd. 71 |
| [19CD]-isoGlu-KC()NTATC()ATQRLAEFLHRSSNN-DPhe-Gly(Me)-A-Ile(Me)-L-DSer-STNVGSNTP-$NH_2$ | Compd. 72 |
| [19CD]-isoGlu-RC()NTATC()ATQRLAEFLHRSSSSF-Gly(Me)-A-Ile(Me)-LSSTDVGSDTP-$NH_2$ | Compd. 73 |
| [19CD]-isoGlu-RC()NTATC()ATQRLAEFLHRSSSSF-Gly(Me)-A-Ile(Me)-LSSTDVGSETP-$NH_2$ | Compd. 74 |
| [19CD]-isoGlu-RC()NTATG()ATQRLAEFLHRSSSSF-Gly(Me)-Ile(Me)-LSSTDVGSETP-$NH_2$ | Compd. 75 |
| [19CD]-isoGlu-RC()NTATC()ATQRLAEFLHRSSSSF-Gly(Me)-A-Ile(Me)-LSSTEVGSETP-$NH_2$ | Compd. 76 |
| [19CD]-isoGlu-RC()NTATC()ATQRLAEFLHRSSSSF-Gly(Me)-Ile(Me)-LSSTEVGSETP-$NH_2$ | Compd. 77 |
| [19CD]-isoGlu-RC()NTATC()ATQRLAEFLHRSSF-Gly(Me)-A-Ile(Me)-LSSTEVGSDTP-$NH_2$ | Compd. 78 |
| [19CD]-isoGlu-RC()NTATC()ATQRLAEFLHRSSF-Gly(Me)-Ile(Me)-LSSTEVGSDTP-$NH_2$ | Compd. 79 |
| [19CD]-isoGlu-RC()NTATC()ATQRLAEFLHRSSF-Gly(Me)-A-Ile(Me)-LSSTDVGSETP-$NH_2$ | Compd. 80 |
| [19CD]-isoGlu-RC()NTATC()ATQRLAEFLHRSSF-Gly(Me)-A-Ile(Me)-LSSTDVGSDTP-$NH_2$ | Compd. 81 |
| [19CD]-isoGlu-RC()NTATC()ATQRLAEFLHRSSF-Gly(Me)-A-Ile(Me)-LSSTEVGSETP-$NH_2$ | Compd. 82 |
| [17CD]-isoGlu-RC()NTATC()ATQRLAEFLHRSSNNF-Gly(Me)-A-Ile(Me)-LSSTNVGSNTP-$NH_2$ | Compd. 89 |
| [15CD]-isoGlu-RC()NTATC()ATQRLAEFLHRSSNNF-Gly(Me)-A-Ile(Me)-LSSTNVGSNTP-$NH_2$ | Compd. 90 |
| [Tetradecanoyl]-isoGlu-RC()NTATC()ATQRLAEFLHRSSNNF-Gly(Me)-A-Ile(Me)-LSSTNVGSNTP-$NH_2$ | Compd. 91 |
| [Hexadecanoyl]-isoGlu-RC()NTATC()ATQRLAEFLHRSSNNF-Gly(Me)-A-Ile(Me)-LSSTNVGSNTP-$NH_2$ | Compd. 92 |

(suite)

| | |
|---|---|
| [Octadecanoyl]-isoGlu-RC()NTATC()ATQRLAEFLHRSSNNF-Gly(Me)-A-Ile(Me)-LSSTNVGSNTP-$NH_2$ | Compd. 93 |
| [Eicosanoyl]-isoGlu-RC()NTATC()ATQRLAEFLHRSSNNF-Gly(Me)-A-Ile(Me)-LSSTNVGSNTP-$NH_2$ | Compd. 94 |
| [19CD]-isoGlu-RC()NTATC()ATQRLAEFLHRSSF-Gly(Me)-Ile(Me)-LSSTEVGSETP-$NH_2$ | Compd. 95 |
| [19CD]-isoGlu-RC()NTATC()ATQRLAEFLHRSSNNF-Gly(Me)-A-Ile(Me)-LSSTNVGSNT-Hyp-$NH_2$ | Compd. 105 |
| [19CD]-isoGlu-RC()NTATC()ATQRLAEFLHRSSNNF-Gly(Me)-Ile(Me)-LSSTNVGSNT-Hyp-$NH_2$ | Compd. 106 |
| [19CD]-isoGlu-RC()NTATC()ATQRLAEFLHRSSNNF-Gly(Me)-A-Ile(Me)-LSSTNVGSNT-Aze-$NH_2$ | Compd. 107 |
| [19CD]-isoGlu-RC()NTATC()ATQRLAEFLHRSSNNF-Gly(Me)-Ile(Me)-LSSTNVGSNT-Aze-$NH_2$ | Compd. 108 |
| [19CD]-isoGlu-RC()NTATC()ATQRLAEFLHRSSNNF-Gly(Me)-A-Ile(Me)-LSSTNVGSNT-Pip-$NH_2$ | Compd. 109 |
| [19CD]-isoGlu-RC()NTATC()ATQRLAEFLHRSSNNF-Gly(Me)-A-Ile(Me)-LSSTNVGSNT-Apr-$NH_2$ | Compd. 110 |
| [19CD]-isoGlu-RC()NTATC()ATQRLAEFLHRSSNNF-Gly(Me)-Ile(Me)-LSSTNVGSNT-Apr-$NH_2$ | Compd. 111 |
| [19CD]-isoGlu-RC()NTATC()ATQRLAEFLHRSSNNF-Gly(Me)-A-Ile(Me)-LSSTNVGSNT-Php-$NH_2$ | Compd. 112 |
| [19CD]-isoGlu-RC()NTATC()ATQRLAEFLHRSSNNF-Gly(Me)-Ile(Me)-LSSTNVGSNT-Php-$NH_2$ | Compd. 113 |
| [19CD]-isoGlu-RC()NTATC()ATQRLAEFLHRSSNNF-Gly(Me)-A-Ile(Me)-LSSTNVGSNT-Bep-$NH_2$ | Compd. 114 |
| [19CD]-isoGlu-RC()NTATC()ATERLAEFLHRSSSSF-Gly(Me)-A-Ile(Me)-LSSTEVGSETP-$NH_2$ | Compd. 119 |
| [19CD]-isoGlu-RC()NTATC()ATDRLAEFLHRSSSSF-Gly(Me)-A-Ile(Me)-LSSTEVGSETP-$NH_2$ | Compd. 120 |
| [19CD]-isoGlu-WC()NTATC()ATQRLAEFLHRSSNNF-Gly(Me)-A-Ile(Me)-LSSTNVGSNTP-$NH_2$ | Compd. 125 |
| [19CD]-isoGlu-RC()ETATC()ATQRLAEFLHRSSSSF-Gly(Me)-A-Ile(Me)-LSSTEVGSETP-$NH_2$ | Compd. 128 |
| [19CD]-isoGlu-RC()PTATC()ATQRLAEFLHRSSSSF-Gly(Me)-A-Ile(Me)-LSSTEVGSETP-$NH_2$ | Compd. 129 |
| [19CD]-isoGlu-RC()GTATC()ATQRLAEFLHRSSSSF-Gly(Me)-A-Ile(Me)-LSSTEVGSETP-$NH_2$ | Compd. 130 |
| [19CD]-isoGlu-RC()ETATC()ATERLAEFLHRSSSSF-Gly(Me)-A-Ile(Me)-LSSTEVGSETP-$NH_2$ | Compd. 134 |
| [19CD]-isoGlu-RC()PTATC()ATERL-AEFLHRSSSSF-Gly(Me)-A-Ile(Me)-LSSTEVGSETP-$NH_2$ | Compd. 136 |
| [19CD]-isoGlu-RC()ETATC()ATDRLAEFLHRSSSSF-Gly(Me)-A-Ile(Me)-LSSTEVGSETP-$NH_2$ | Compd. 150 |
| [19CD]-isoGlu-RC()PTATC()ATDRLAEFLHRSSSSF-Gly(Me)-A-Ile(Me)-LSSTEVGSETP-$NH_2$ | Compd. 151 |
| [19CD]-isoGlu-RC()NTATC()ATQRLAEFLHRSSNNF-Gly(Me)-A-Ile(Me)-LSSTNVGSNTP-$NHM_2$ | Compd. 152 |
| [19CD]-isoGlu-RC()NTATC()ATQRLAEFLHRSSNNF-Gly(Me)-Ile(Me)-LSSTNVGSNTP-$NHM_2$ | Compd. 153 |
| [19CD]-isoGlu-RC()NTATC()ATQRLAEFLHRSSNNF-Gly(Me)-A-Ile(Me)-LSSTNVGSNTY-$NHM_2$ | Compd. 154 |
| [19CD]-isoGlu-RC()NTATC()ATQRLAEFLHRSSNNF-Gly(Me)-Ile(Me)-LSSTNVGSNTY-$NHM_2$ | Compd. 155 |
| [19CD]-isoGlu-RC()NTATC()ATQRLAEFLHRSSNNF-Gly(Me)-A-Ile(Me)-LSSTNVGSNTY-$NH_2$ | Compd. 156 |
| [19CD]-isoGlu-WC()GTATC()ATDRLAEFLHRSSF-Gly(Me)-A-Ile(Me)-LSSTDVGSDTP-$NH_2$ | Compd. 157 |
| [19CD]-isoGlu-RC()GTATC()ATDRLAEFLHRSSF-Gly(Me)-A-Ile(Me)-LSSTDVGSDTP-$NH_2$ | Compd. 158 |
| [19CD]-isoGlu-RC()NTATC()ATERLAEFLHRSSSSF-Gly(Me)-A-Ile(Me)-LSSTDVGSDTP-$NH_2$ | Compd. 160 |
| [19CD]-isoGlu-WC()GTATC()ATERLAEFLHRSSF-Gly(Me)-A-Ile(Me)-LSSTEVGSETP-$NH_2$ | Compd. 161 |
| [19CD]-isoGlu-RC()GTATC()ATERLAEFLHRSSP-Gly(Me)-A-Ile(Me)-LSSTEVGSETP-$NH_2$ | Compd. 162 |
| [19CD]-isoGlu-WC()GTATC()ATERLAEFLHRSSF-Gly(Me)-A-Ile(Me)-LSSTEVGSET-Hyp-$NH_2$ | Compd. 164 |
| [19CD]-isoGlu-WC()GTATC()ATERLAEFLHRSSF-Gly(Me)-Ile(Me)-LSSTEVGSET-Hyp-$NH_2$ | Compd. 165 |
| [19CD]-isoGlu-RC()GTATC()ATERLAEFLHRSSF-Gly(Me)-A-Ile(Me)-LSSTEVGSET-Hyp-$NH_2$ | Compd. 166 |
| [19CD]-isoGlu-RC()GTATC()ATERLAEFLHRSSF-Gly(Me)-Ile(Me)-LSSTEVGSET-Hyp-$NH_2$ | Compd. 167 |
| [19CD]-isoGlu-RC()ETATC()ATERLAEFLHRSSSSF-Gly(Me)-A-Ile(Me)-LSSTEVGSET-Hyp-$NH_2$ | Compd. 170 |
| [19CD]-isoGlu-RC()ETATC()ATERLAEFLHRSSSSF-Gly(Me)-Ile(Me)-LSSTEVGSET-Hyp-$NH_2$ | Compd. 171 |
| [19CD]-isoGlu-RC()PTATC()ATDRLAEFLHRSSF-Gly(Me)-A-Ile(Me)-LSSTDVGSDTP-$NH_2$ | Compd. 172 |
| [19CD]-isoGlu-RC()PTATC()ATERLAEFLHRSSSSF-Gly(Me)-A-Ile(Me)-LSSTDVGSDTP-$NH_2$ | Compd. 173 |
| et | |
| [19CD]-isoGlu-RC()NTATC()ATQRLAEFLHHSSNNF-Gly(Me)-Ile(Me)-LPPTNVGSNTP-$NH_2$ | Compd. 176 |

**13.** Procédé pour la synthèse d'un composé ou d'un sel ou solvate pharmaceutiquement acceptable selon l'une quelconque des revendications précédentes, comprenant la synthèse du peptide par une méthodologie en phase solide ou en phase liquide, éventuellement l'isolation et la purification du produit final, et en outre comprenant éventuellement l'étape de formation d'une liaison disulfure entre les groupes thiol des chaînes latérales de cystéine aux positions 2 et 7.

**14.** Composé ou sel ou solvate pharmaceutiquement acceptable de celui-ci selon l'une quelconque des revendications 1 à 12, pour utilisation dans une méthode de traitement médical d'une maladie, d'un état ou d'un trouble choisi parmi la maladie d'Alzheimer, le diabète, le diabète de type 1, le diabète de type 2, le pré-diabète, le syndrome de résistance à l'insuline, la dégradation de la tolérance au glucose (IGT), le coeur diabétique (y compris la cardiomyopathie et l'insuffisance cardiaque diabétiques), le syndrome métabolique, la stéatose hépatique non alcoolique, la stéatose hépatique, l'obésité, l'obésité morbide, l'obésité morbide avant opération chirurgicale, une inflammation liée à l'obésité, une maladie de la vésicule biliaire liée à l'obésité, l'apnée du sommeil et les problèmes respiratoires induits par l'obésité, la dégénérescence des cartilages et l'arthrose, et les complications médicales reproductrices telles que l'infertilité, lequel composé ou sel ou solvate pharmaceutiquement acceptable est éventuellement administré en combinaison avec un agent antidiabétique, un agent anti-obésité, un agent anti-hypertension, un agent anti-dyslipidémie, un inhibiteur de la pompe à protons ou un agent anti-inflammatoire, par exemple dans lequel ledit agent antidiabétique est la metformine, une sulfonylurée, un glinide, un inhibiteur de DPP-IV, une glitazone, le GLP-1 ou un analogue de GLP-1, l'exendine-4 ou un analogue d'exendine-4, un agoniste double de glucagon-GLP-1 ou n'importe quel autre agoniste de récepteur de GLP-1, un inhibiteur de SGLT2, un agoniste de GPR40, l'insuline ou un analogue de l'insuline, éventuellement dans lequel le sujet devant être traité est un humain.

**15.** Composition pharmaceutique comprenant un composé ou un sel ou solvate pharmaceutiquement acceptable de celui-ci selon l'une quelconque des revendications 1 à 12, et un support, excipient ou véhicule pharmaceutiquement acceptable.

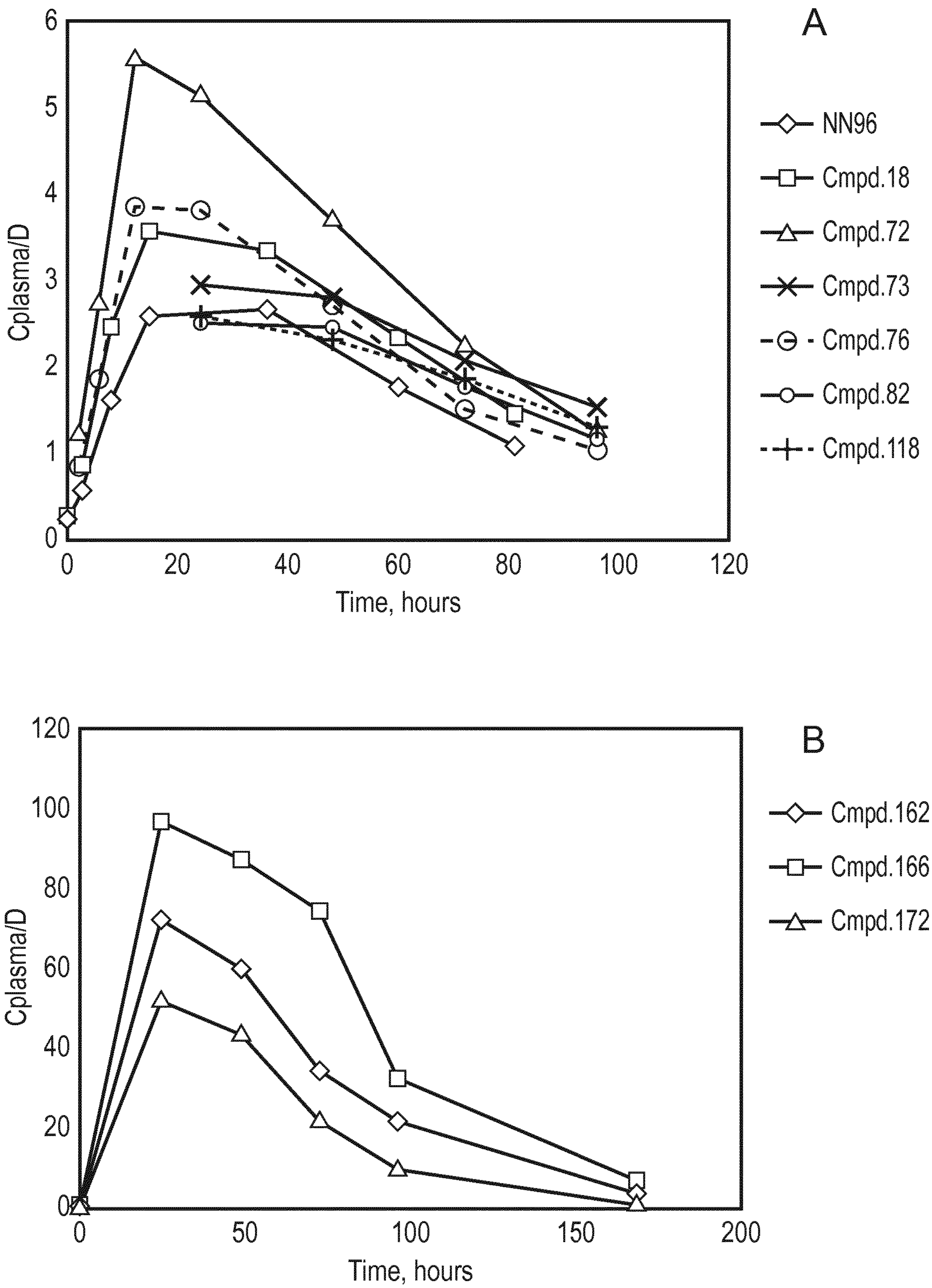
A
6
5
4
3
2
1
0
Cplasma/D
0
20
40
60
80
100
120
Time, hours
NN96
Cmpd.18
Cmpd.72
Cmpd.73
Cmpd.76
Cmpd.82
Cmpd.118
B
120
100
80
60
40
20
0
Cplasma/D
0
50
100
150
200
Time, hours
Cmpd.162
Cmpd.166
Cmpd.172

FIG. 1

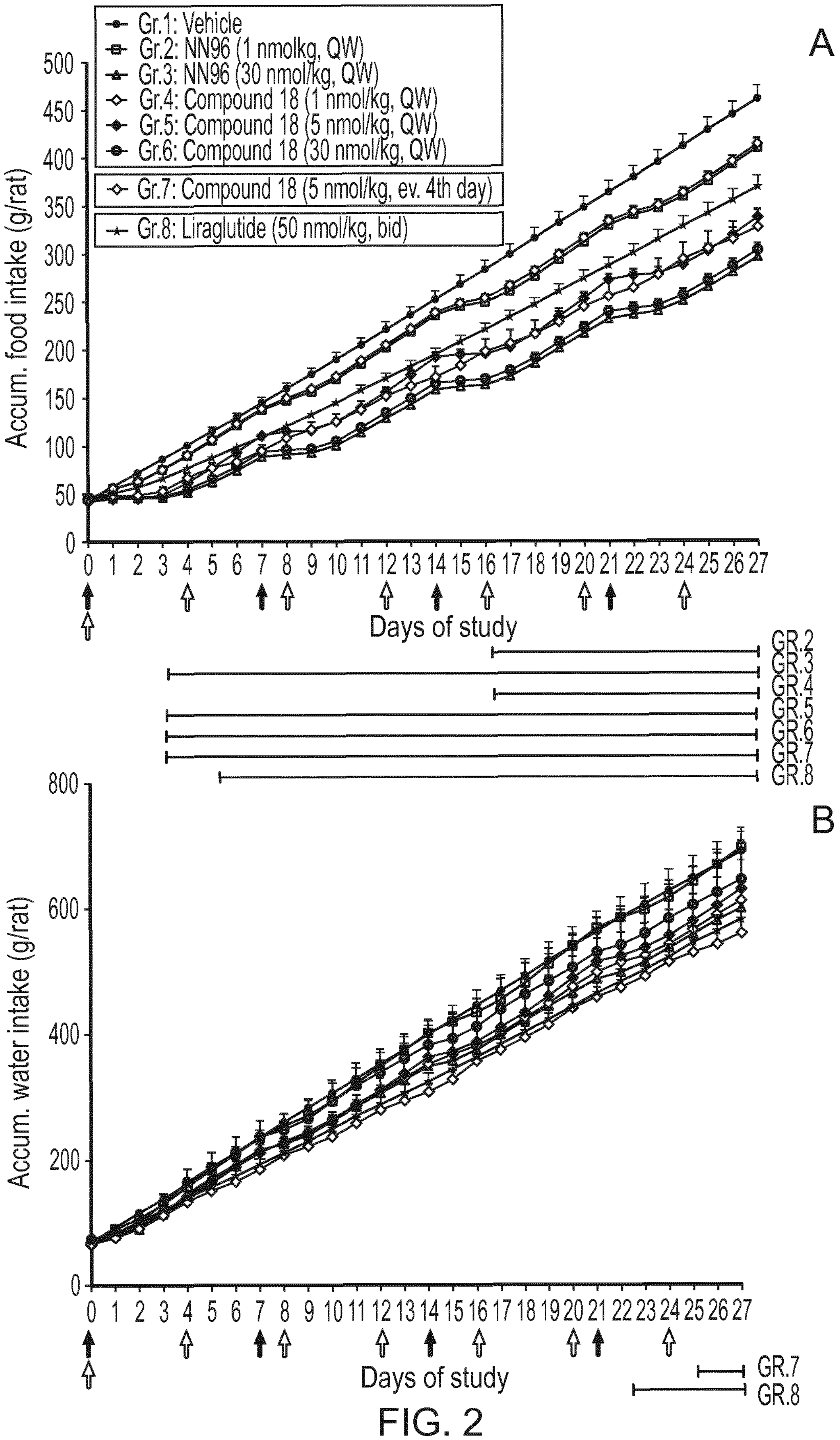
Gr.1: Vehicle
Gr.2: NN96 (1 nmolkg, QW)
Gr.3: NN96 (30 nmol/kg, QW)
Gr.4: Compound 18 (1 nmol/kg, QW)
Gr.5: Compound 18 (5 nmol/kg, QW)
Gr.6: Compound 18 (30 nmol/kg, QW)
Gr.7: Compound 18 (5 nmol/kg, ev. 4th day)
Gr.8: Liraglutide (50 nmol/kg, bid)
A
Accum. food intake (g/rat)
Days of study
GR.2
GR.3
GR.4
GR.5
GR.6
GR.7
GR.8
B
Accum. water intake (g/rat)
Days of study
GR.7
GR.8

FIG. 2

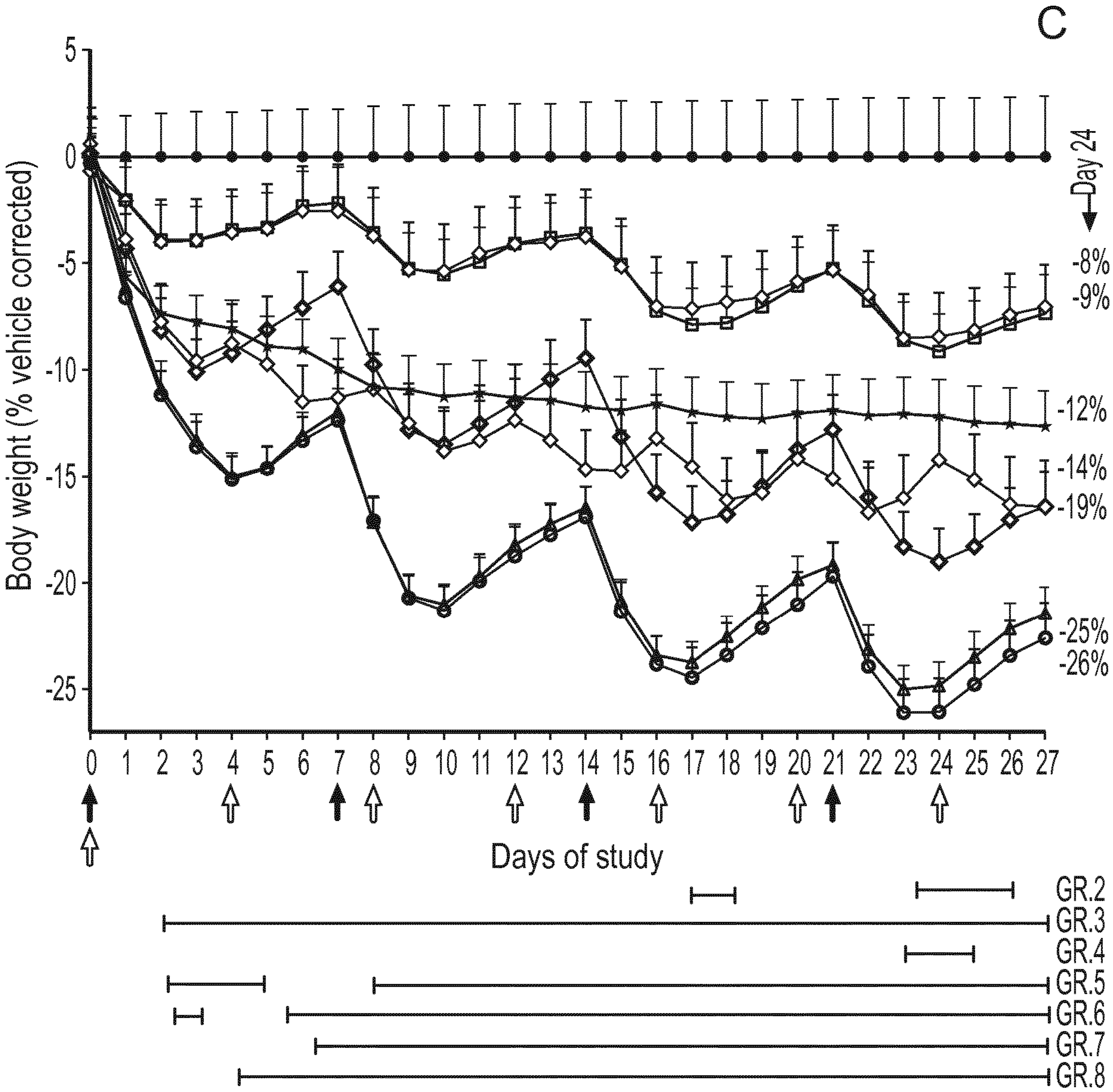
C
Body weight (% vehicle corrected)
5
0
-5
-10
-15
-20
-25
Day 24
-8%
-9%
-12%
-14%
-19%
-25%
-26%
0 1 2 3 4 5 6 7 8 9 10 11 12 13 14 15 16 17 18 19 20 21 22 23 24 25 26 27
Days of study
GR.2
GR.3
GR.4
GR.5
GR.6
GR.7
GR.8

FIG. 2

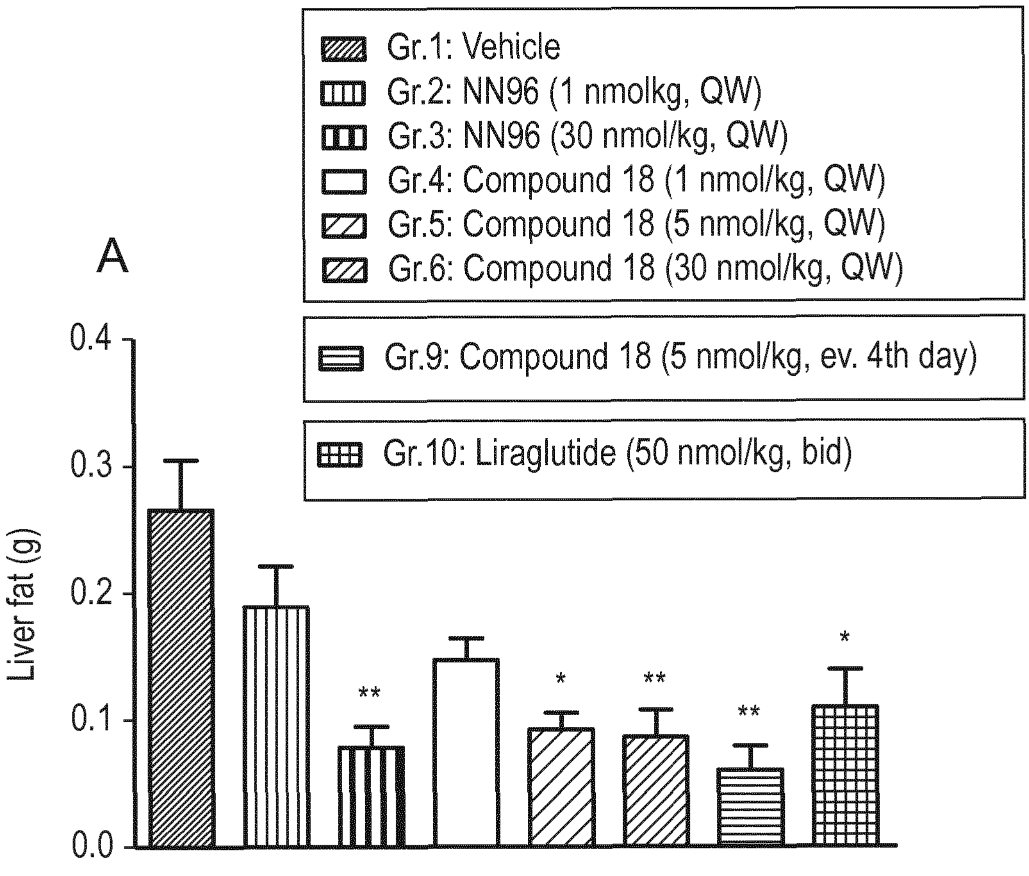
A
Gr.1: Vehicle
Gr.2: NN96 (1 nmolkg, QW)
Gr.3: NN96 (30 nmol/kg, QW)
Gr.4: Compound 18 (1 nmol/kg, QW)
Gr.5: Compound 18 (5 nmol/kg, QW)
Gr.6: Compound 18 (30 nmol/kg, QW)
Gr.9: Compound 18 (5 nmol/kg, ev. 4th day)
Gr.10: Liraglutide (50 nmol/kg, bid)
Liver fat (g)
0.4
0.3
0.2
0.1
0.0
**
*
**
**
*

FIG. 3

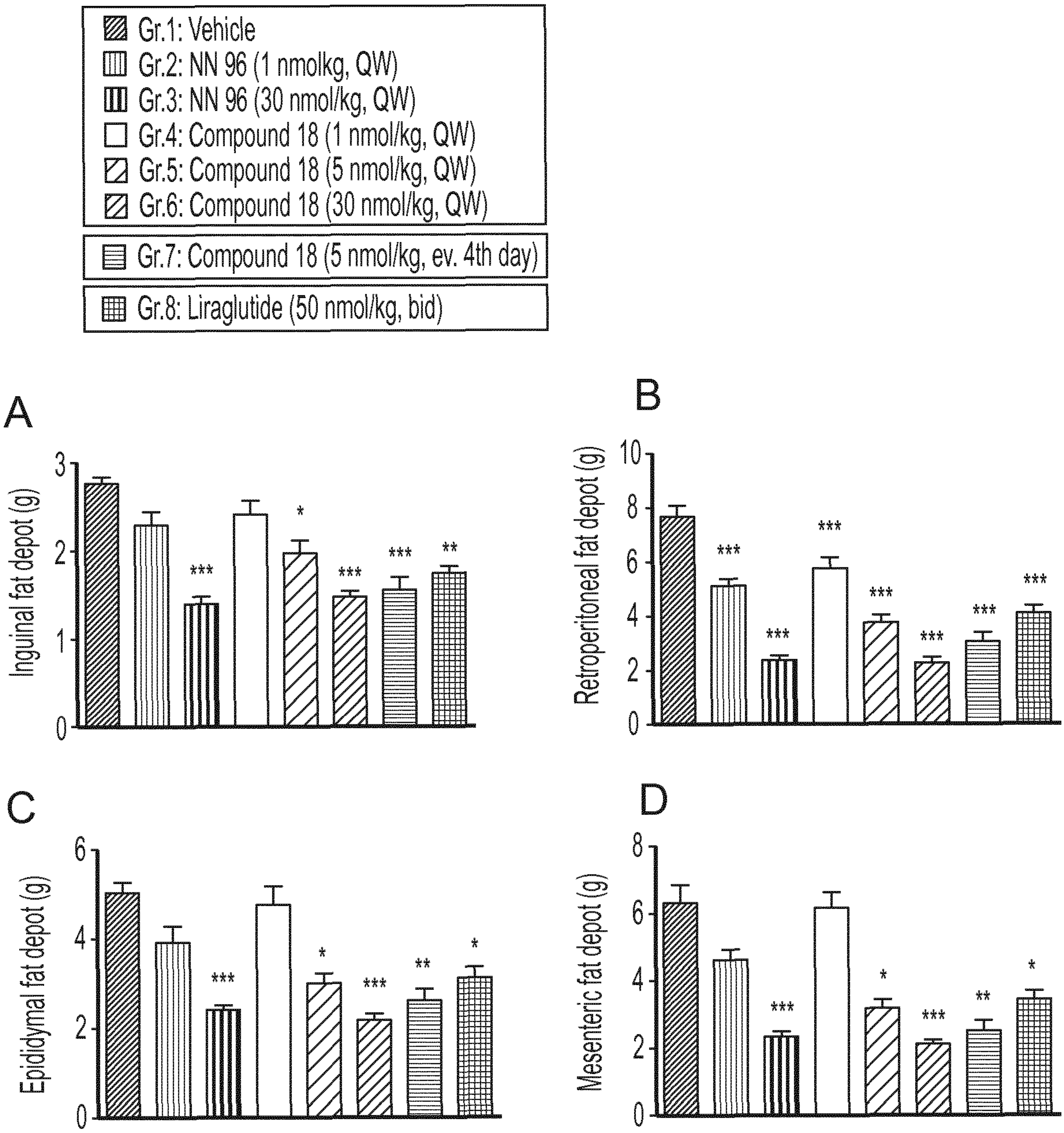
Gr.1: Vehicle
Gr.2: NN 96 (1 nmolkg, QW)
Gr.3: NN 96 (30 nmol/kg, QW)
Gr.4: Compound 18 (1 nmol/kg, QW)
Gr.5: Compound 18 (5 nmol/kg, QW)
Gr.6: Compound 18 (30 nmol/kg, QW)
Gr.7: Compound 18 (5 nmol/kg, ev. 4th day)
Gr.8: Liraglutide (50 nmol/kg, bid)
A
Inguinal fat depot (g)
B
Retroperitoneal fat depot (g)
C
Epididymal fat depot (g)
D
Mesenteric fat depot (g)

FIG. 4

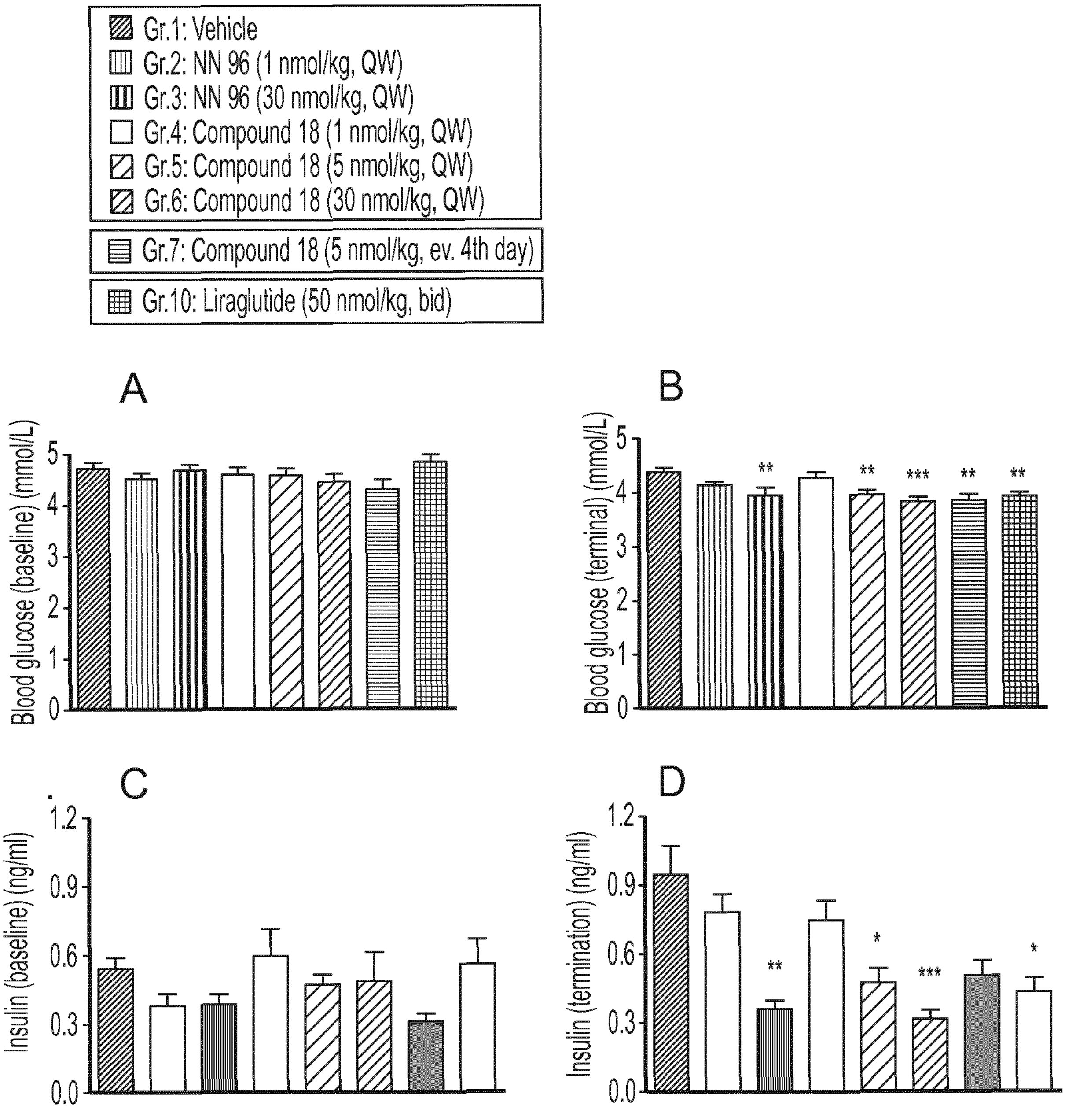
Gr.1: Vehicle
Gr.2: NN 96 (1 nmol/kg, QW)
Gr.3: NN 96 (30 nmol/kg, QW)
Gr.4: Compound 18 (1 nmol/kg, QW)
Gr.5: Compound 18 (5 nmol/kg, QW)
Gr.6: Compound 18 (30 nmol/kg, QW)
Gr.7: Compound 18 (5 nmol/kg, ev. 4th day)
Gr.10: Liraglutide (50 nmol/kg, bid)
A
Blood glucose (baseline) (mmol/L)
B
Blood glucose (terminal) (mmol/L)
C
Insulin (baseline) (ng/ml)
D
Insulin (termination) (ng/ml)

FIG. 5

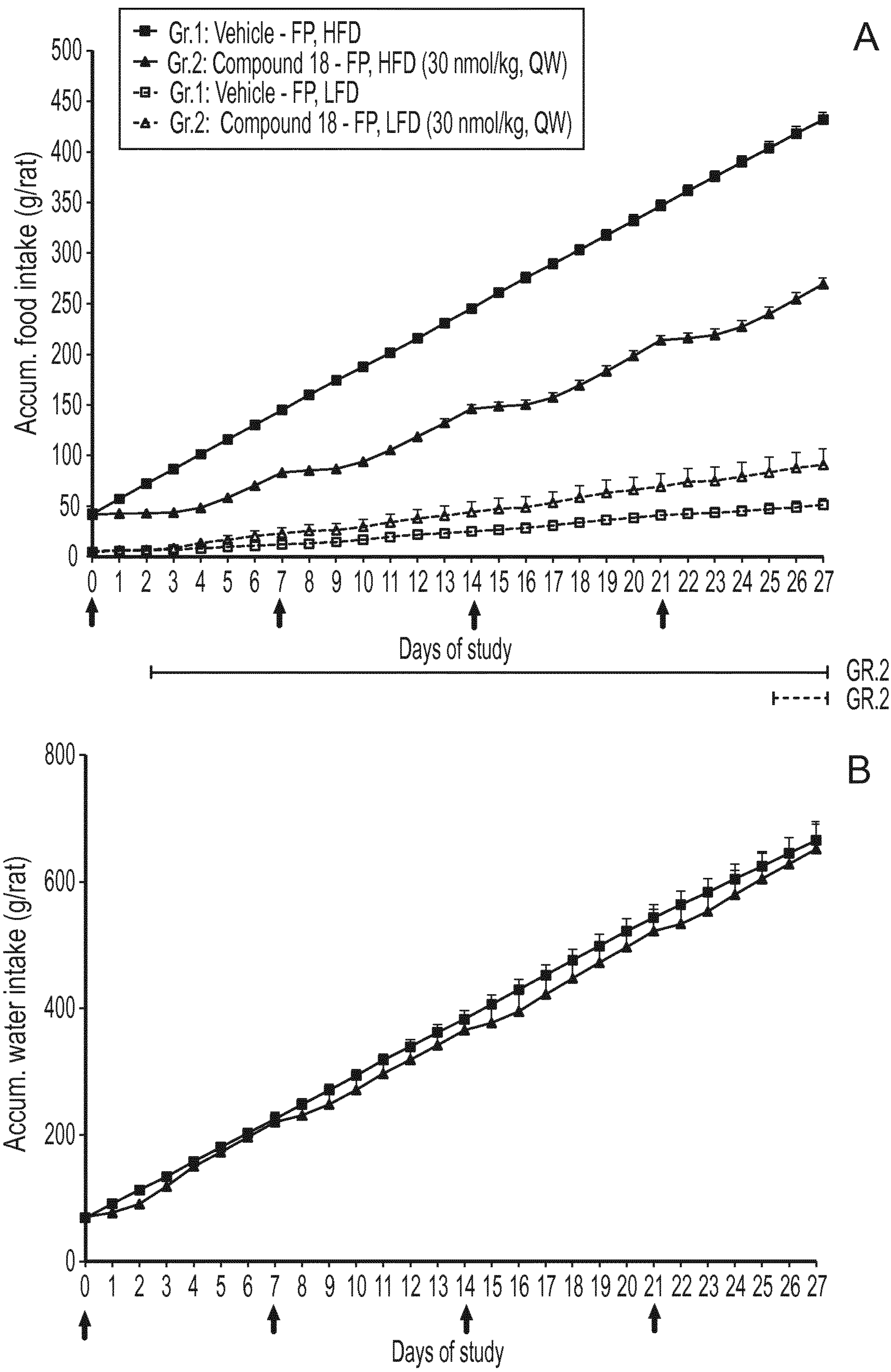
A
Gr.1: Vehicle - FP, HFD
Gr.2: Compound 18 - FP, HFD (30 nmol/kg, QW)
Gr.1: Vehicle - FP, LFD
Gr.2: Compound 18 - FP, LFD (30 nmol/kg, QW)
Accum. food intake (g/rat)
0
50
100
150
200
250
300
350
400
450
500
0 1 2 3 4 5 6 7 8 9 10 11 12 13 14 15 16 17 18 19 20 21 22 23 24 25 26 27
Days of study
GR.2
GR.2
B
Accum. water intake (g/rat)
0
200
400
600
800
0 1 2 3 4 5 6 7 8 9 10 11 12 13 14 15 16 17 18 19 20 21 22 23 24 25 26 27
Days of study

FIG. 6

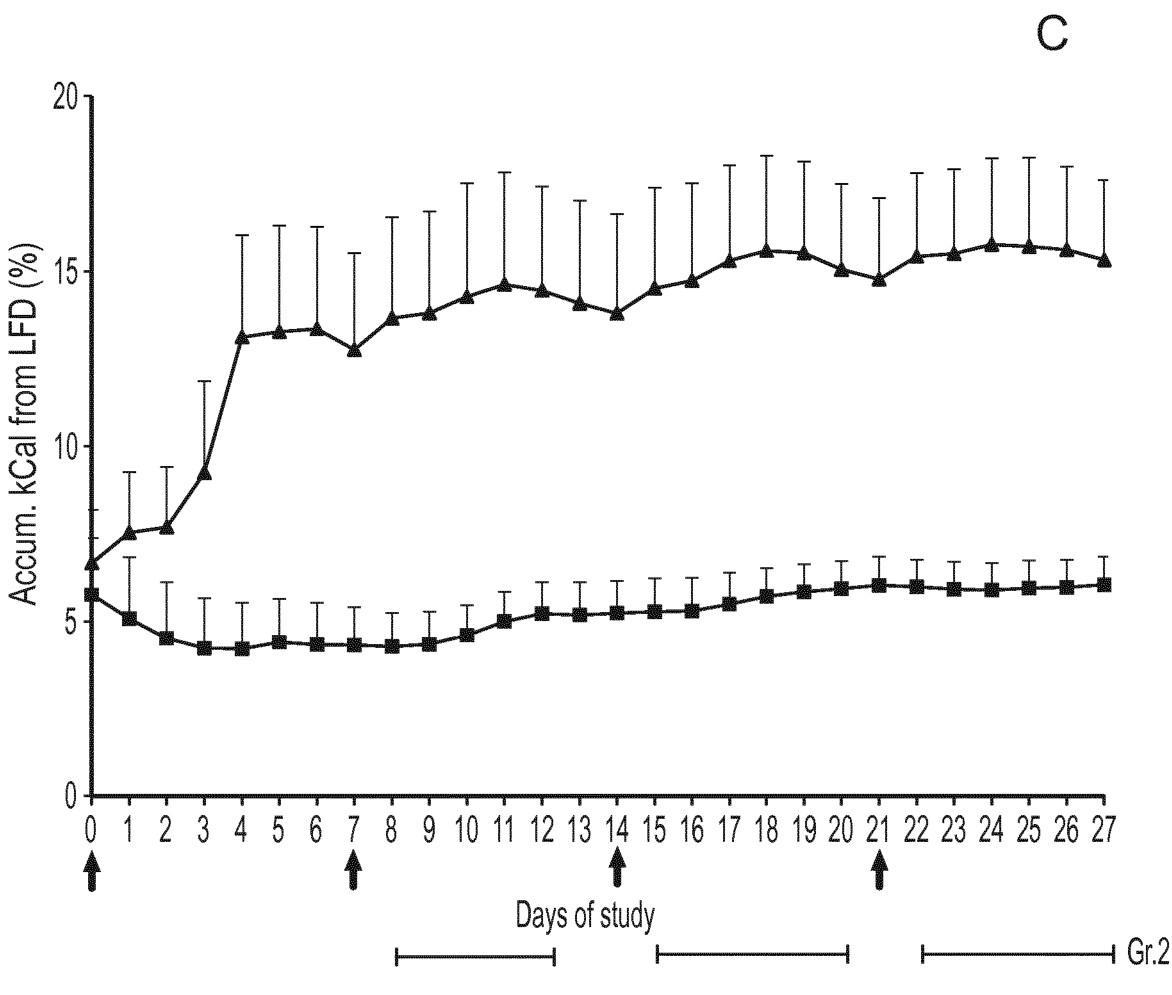
C
20
15
10
5
0
Accum. kCal from LFD (%)
0 1 2 3 4 5 6 7 8 9 10 11 12 13 14 15 16 17 18 19 20 21 22 23 24 25 26 27
Days of study
Gr.2

FIG. 6

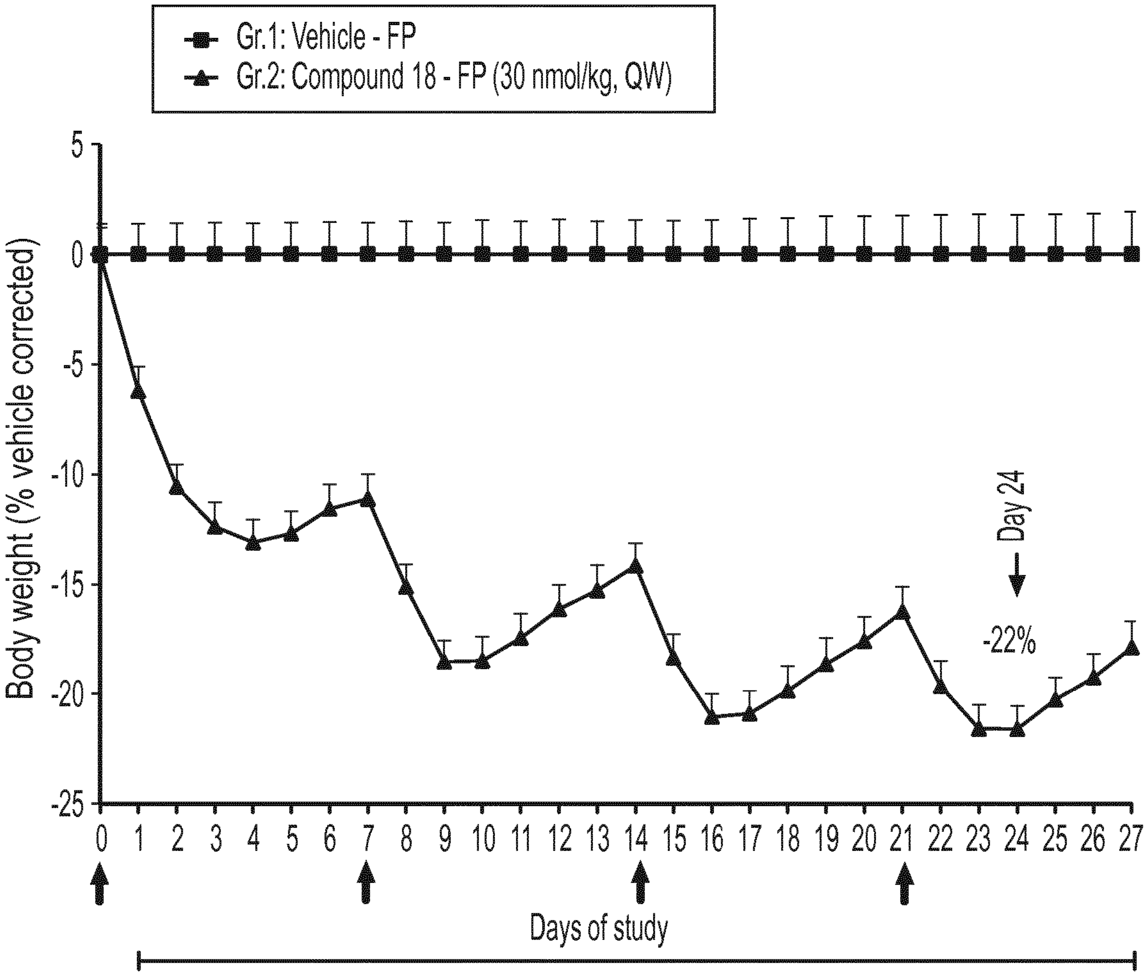
Gr.1: Vehicle - FP
Gr.2: Compound 18 - FP (30 nmol/kg, QW)
Body weight (% vehicle corrected)
5
0
-5
-10
-15
-20
-25
Day 24
-22%
0 1 2 3 4 5 6 7 8 9 10 11 12 13 14 15 16 17 18 19 20 21 22 23 24 25 26 27
Days of study

FIG. 7

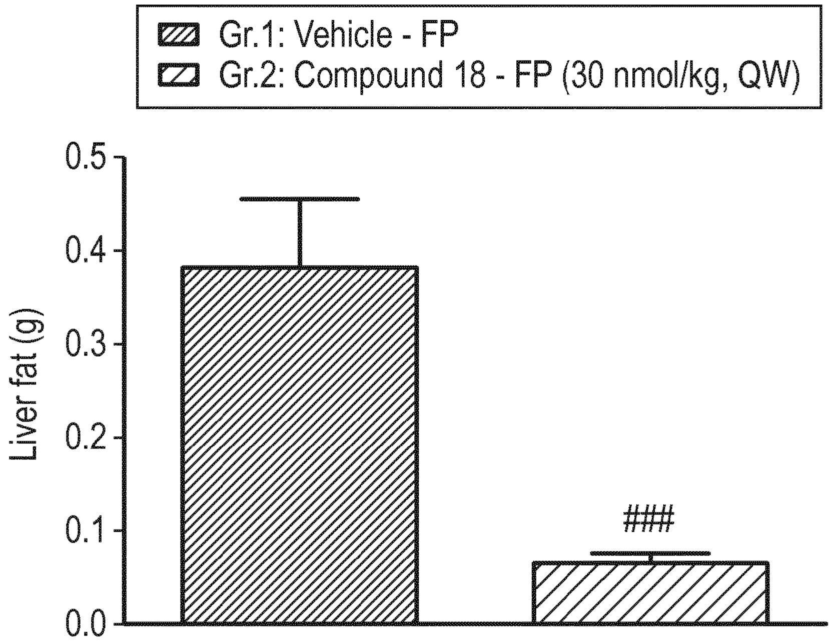
Gr.1: Vehicle - FP
Gr.2: Compound 18 - FP (30 nmol/kg, QW)
Liver fat (g)
0.5
0.4
0.3
0.2
0.1
0.0
###

FIG. 8

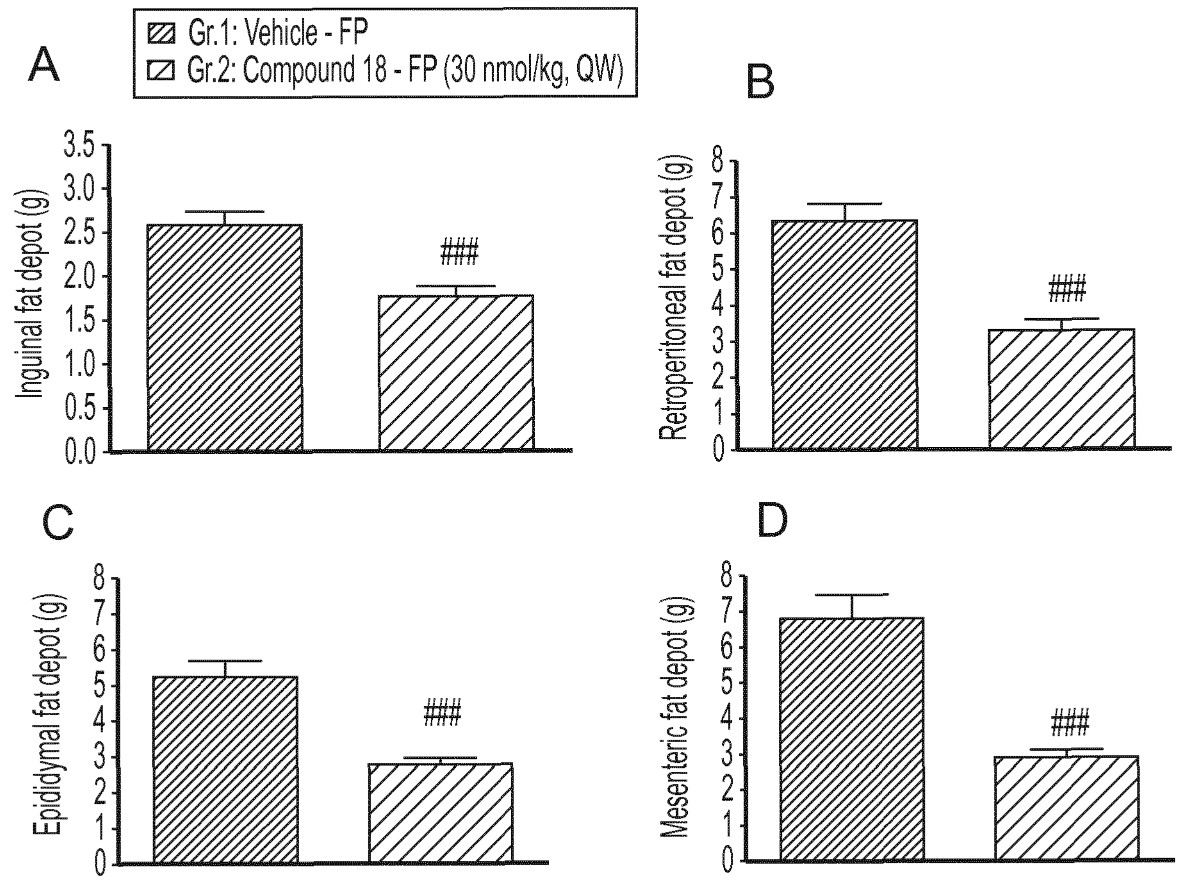
Gr.1: Vehicle - FP
Gr.2: Compound 18 - FP (30 nmol/kg, QW)
A
B
C
D
Inguinal fat depot (g)
Retroperitoneal fat depot (g)
Epididymal fat depot (g)
Mesenteric fat depot (g)
3.5
3.0
2.5
2.0
1.5
1.0
0.5
0.0
8
7
6
5
4
3
2
1
0
###

FIG. 9

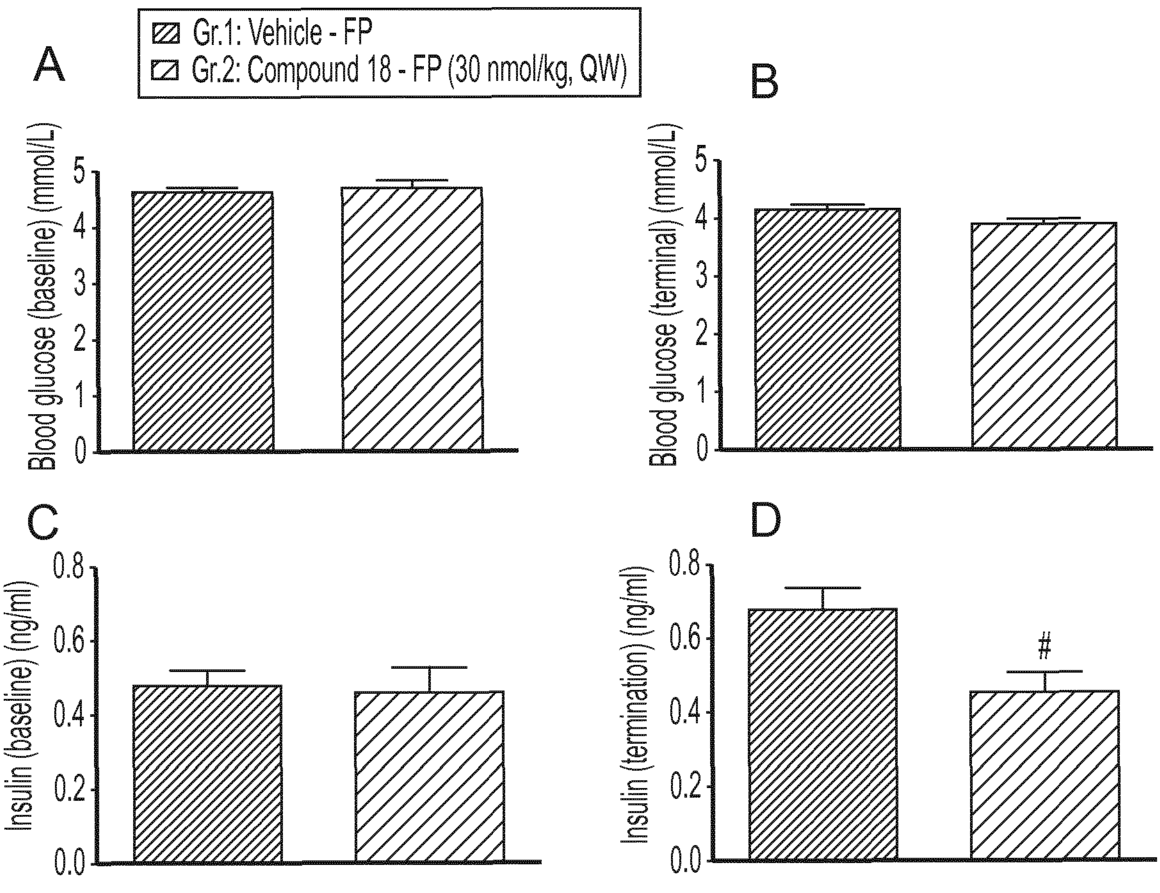
Gr.1: Vehicle - FP
Gr.2: Compound 18 - FP (30 nmol/kg, QW)
A
B
C
D
Blood glucose (baseline) (mmol/L)
Blood glucose (terminal) (mmol/L)
Insulin (baseline) (ng/ml)
Insulin (termination) (ng/ml)
0
1
2
3
4
5
0.0
0.2
0.4
0.6
0.8
#

FIG. 10

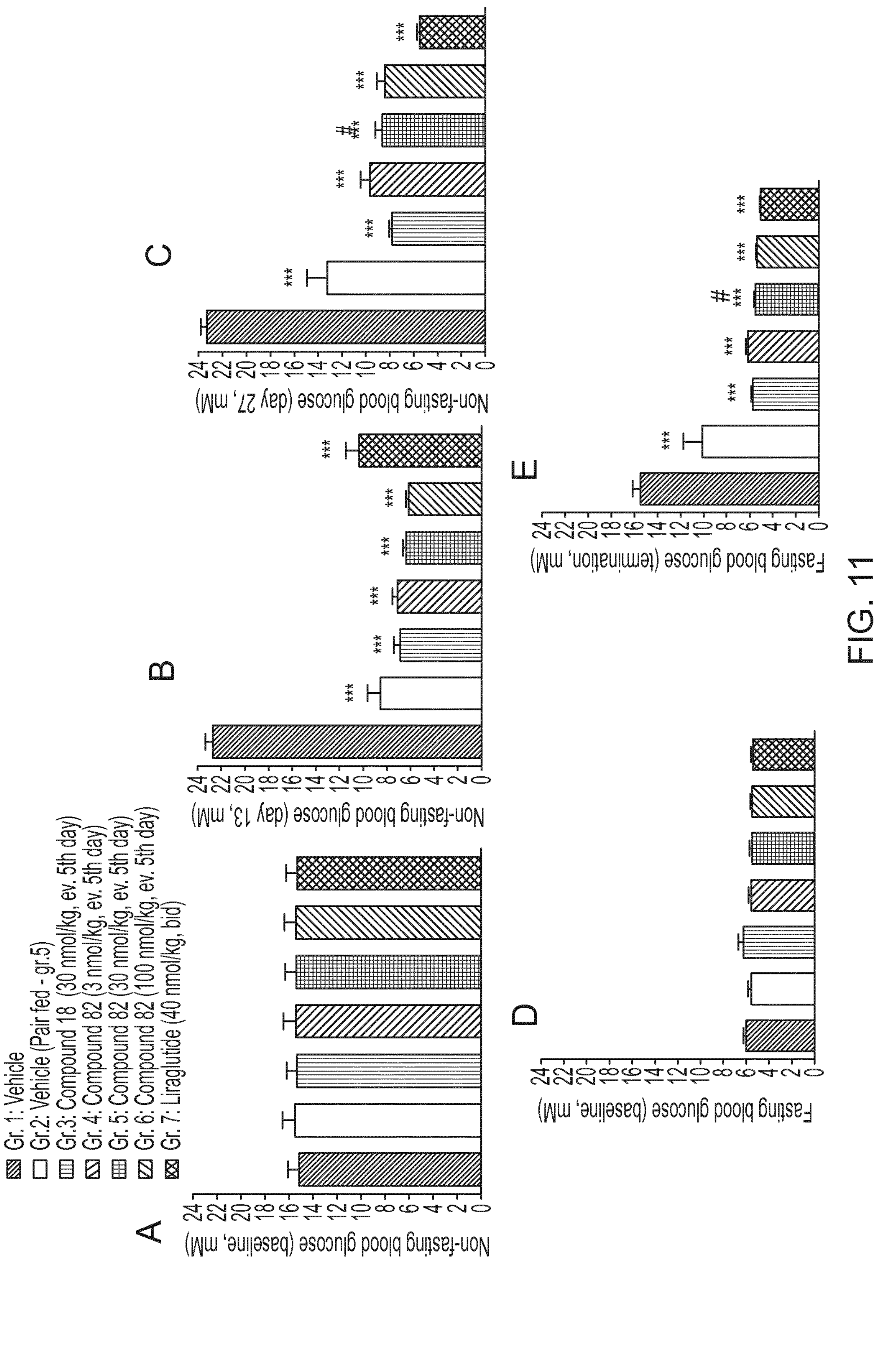
A
B
C
D
E
Gr. 1: Vehicle
Gr.2: Vehicle (Pair fed - gr.5)
Gr.3: Compound 18 (30 nmol/kg, ev. 5th day)
Gr. 4: Compound 82 (3 nmol/kg, ev. 5th day)
Gr. 5: Compound 82 (30 nmol/kg, ev. 5th day)
Gr. 6: Compound 82 (100 nmol/kg, ev. 5th day)
Gr. 7: Liraglutide (40 nmol/kg, bid)
Non-fasting blood glucose (baseline, mM)
Non-fasting blood glucose (day 13, mM)
Non-fasting blood glucose (day 27, mM)
Fasting blood glucose (baseline, mM)
Fasting blood glucose (termination, mM)
24 22 20 18 16 14 12 10 8 6 4 2 0
***
#

FIG. 11

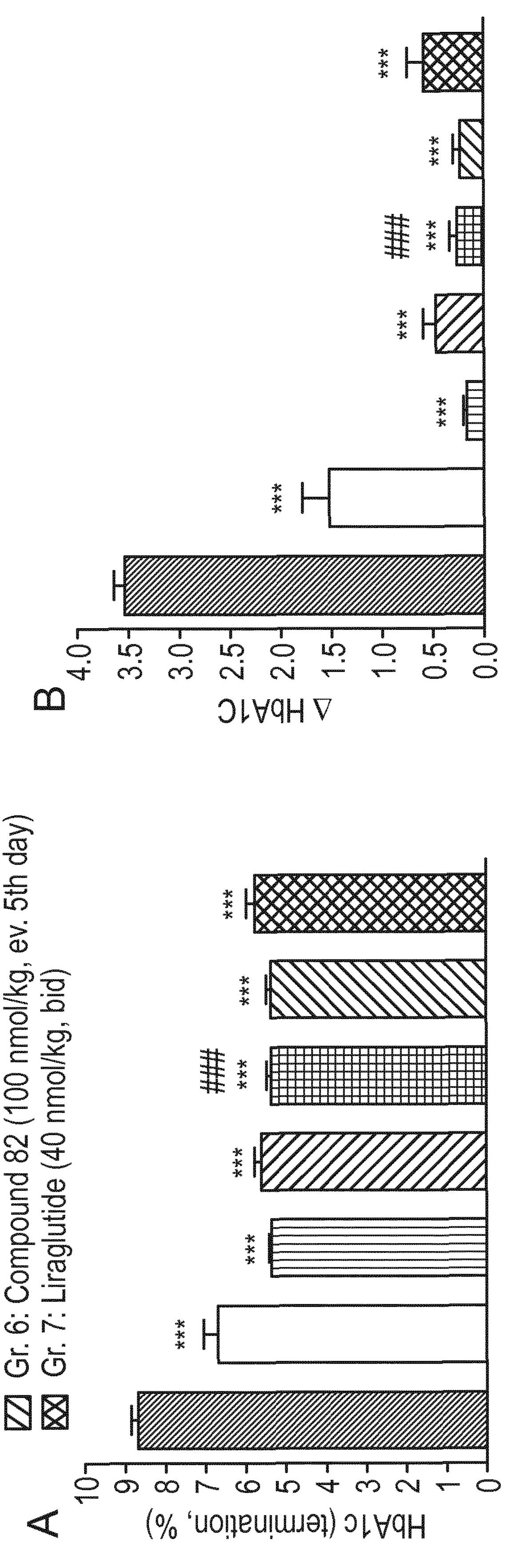
A
HbA1c (termination, %)
0
1
2
3
4
5
6
7
8
9
10
Gr. 1: Vehicle
Gr.2: Vehicle (Pair fed - gr. 5)
Gr.3: Compound 18 (30 nmol/kg, ev. 5th day)
Gr. 4: Compound 82 (3 nmol/kg, ev. 5th day)
Gr. 5: Compound 82 (30 nmol/kg, ev. 5th day)
Gr. 6: Compound 82 (100 nmol/kg, ev. 5th day)
Gr. 7: Liraglutide (40 nmol/kg, bid)
***
####
B
Δ HbA1C
0.0
0.5
1.0
1.5
2.0
2.5
3.0
3.5
4.0

FIG. 12

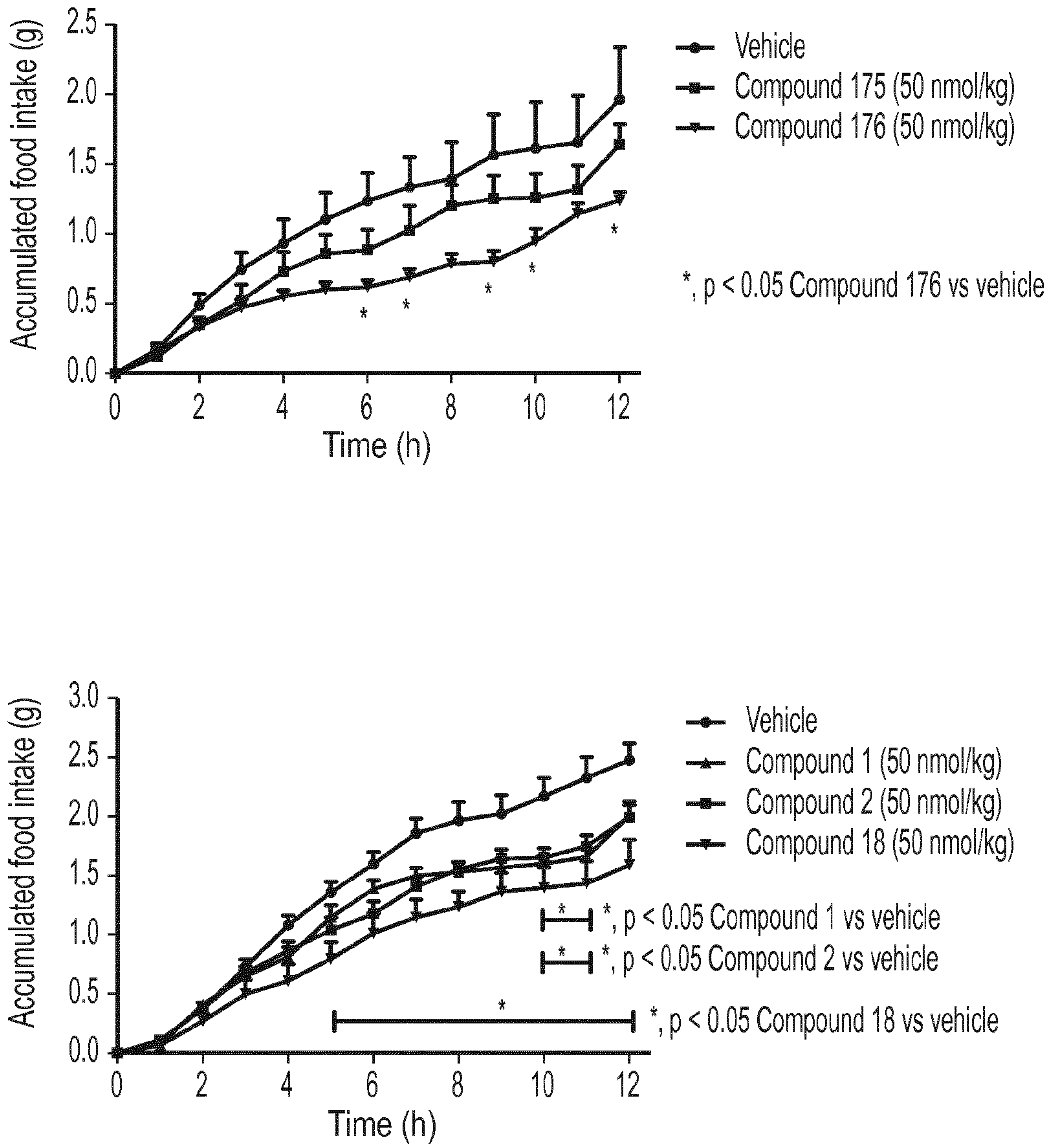

Accumulated food intake (g)
Time (h)
Vehicle
Compound 175 (50 nmol/kg)
Compound 176 (50 nmol/kg)
*, p < 0.05 Compound 176 vs vehicle
Accumulated food intake (g)
Time (h)
Vehicle
Compound 1 (50 nmol/kg)
Compound 2 (50 nmol/kg)
Compound 18 (50 nmol/kg)
*, p < 0.05 Compound 1 vs vehicle
*, p < 0.05 Compound 2 vs vehicle
*, p < 0.05 Compound 18 vs vehicle


FIG. 13

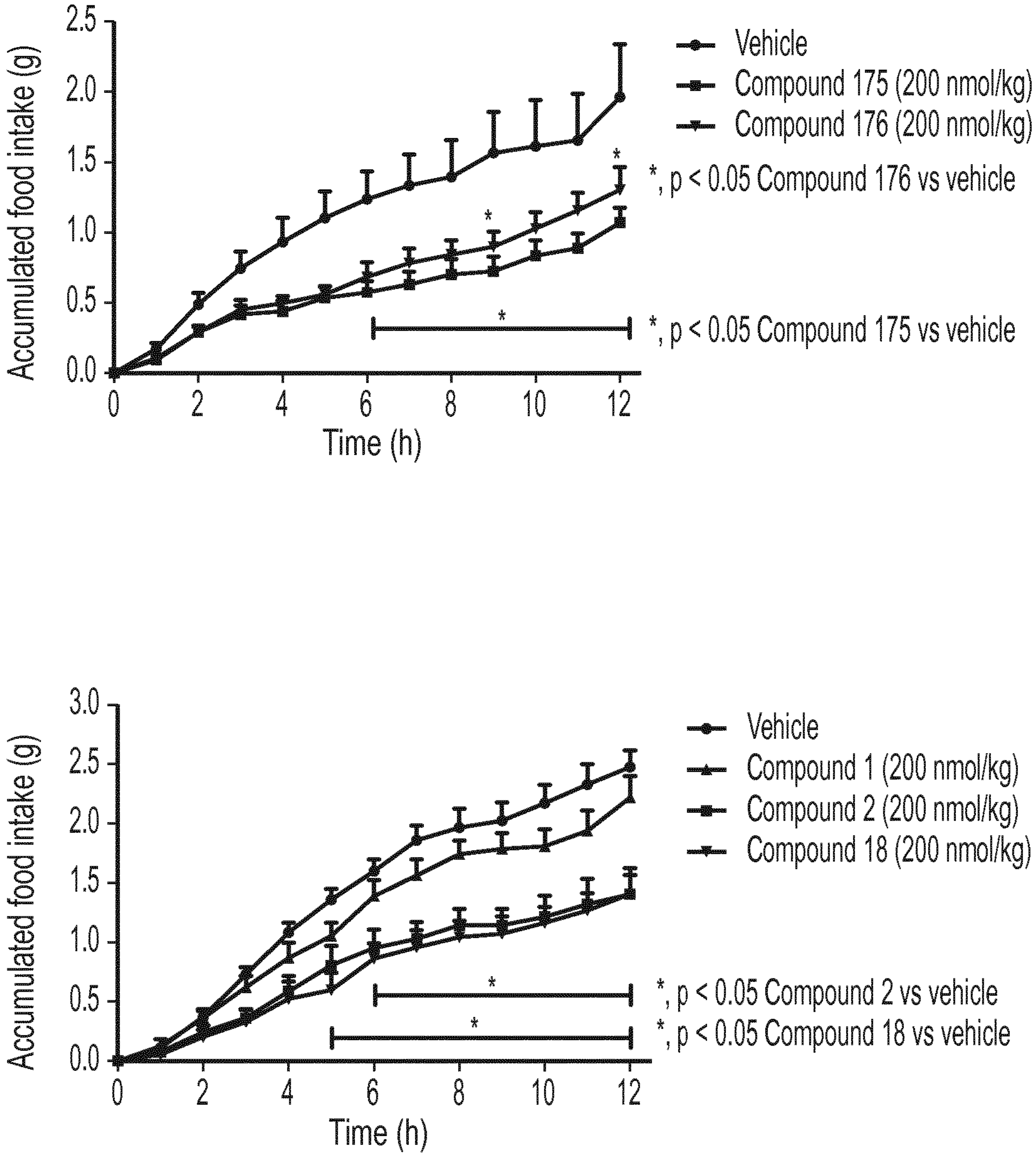

Accumulated food intake (g)
2.5
2.0
1.5
1.0
0.5
0.0
0
2
4
6
8
10
12
Time (h)
Vehicle
Compound 175 (200 nmol/kg)
Compound 176 (200 nmol/kg)
*, p < 0.05 Compound 176 vs vehicle
*, p < 0.05 Compound 175 vs vehicle
Accumulated food intake (g)
3.0
2.5
2.0
1.5
1.0
0.5
0.0
0
2
4
6
8
10
12
Time (h)
Vehicle
Compound 1 (200 nmol/kg)
Compound 2 (200 nmol/kg)
Compound 18 (200 nmol/kg)
*, p < 0.05 Compound 2 vs vehicle
*, p < 0.05 Compound 18 vs vehicle


FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9310146 A
- WO 2012168430 A
- WO 2012168431 A
- WO 2012168432 A
- WO 2006042745 A
- WO 9811125 A
- WO 9808871 A
- WO 0055184 A
- WO 0055119 A
- WO 2008101017 A
- WO 2008152403 A
- WO 2010070252 A
- WO 2010070253 A
- WO 2010070255 A
- WO 2010070251 A
- WO 2011006497 A
- WO 2011160630 A
- WO 2011160633 A
- WO 2013092703 A
- WO 2014041195 A
- WO 2012168430 A2


### Non-patent literature cited in the description


- **YAN et al.** *PNAS,* 2006, vol. 103 (7), 2046-2051
- Remington's Pharmaceutical Sciences. Mark Publishing Company, 1985
- Encyclopaedia of Pharmaceutical Technology. Informa Healthcare USA (Inc.), 2007
- *J. Pharm. Sci.,* 1977, vol. 66, 2
- Principles and Practice of Solid-Phase Peptide Synthesis. **FIELDS, G.B. et al.** Synthetic Peptides. Oxford University Press, 2002
- **MADSEN et al.** *J. Med. Chem.,* 2007, vol. 50, 6126-32
- **KNUDSEN et al.** *J. Med Chem.,* 2000, vol. 43, 1664-1669
- **HAFFNER et al.** *Diabetes,* 2005, vol. 54, 1566-1572
- *CHEMICAL ABSTRACTS,* 84793-07
- **GROENNING, M.** *J. Chem. Biol.,* 2010, vol. 3 (1), 1-18
- **GROENNING et al.** *J. Struct. Biol.,* 2007, vol. 158, 358-369
- **LEVINE, H., III.** *Protein Sci.,* 1993, vol. 2, 404-410